(19) Europäisches Patentamt
European Patent Office
Office européen des brevets

(11) EP 3 287 457 B1

(12) EUROPEAN PATENT SPECIFICATION

(45) Date of publication and mention of the grant of the patent:
**18.09.2019 Bulletin 2019/38**

(21) Application number: **16783074.4**

(22) Date of filing: **13.04.2016**

(51) Int Cl.:
***C07D 471/04*** *(2006.01)* ***A01G 7/06*** *(2006.01)*
***A01M 99/00*** *(2006.01)* ***A01N 25/00*** *(2006.01)*
***A01N 43/90*** *(2006.01)* ***A01N 47/02*** *(2006.01)*
***A01P 7/04*** *(2006.01)*

(86) International application number:
**PCT/JP2016/061894**

(87) International publication number:
**WO 2016/171053 (27.10.2016 Gazette 2016/43)**

# (54) MESOIONIC COMPOUND

MESOIONISCHE VERBINDUNG

COMPOSÉ MÉSOIONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.04.2015 JP 2015086533**

(43) Date of publication of application:
**28.02.2018 Bulletin 2018/09**

(73) Proprietor: **Nippon Kayaku Kabushiki Kaisha**
**Tokyo 100-0005 (JP)**

(72) Inventors:
- **HASEGAWA, Shinji**
  **Kamisu-shi**
  **Ibaraki 314-0255 (JP)**
- **KAMO, Tomohiro**
  **Kamisu-shi**
  **Ibaraki 314-0255 (JP)**
- **KAGOHARA, Yuma**
  **Kamisu-shi**
  **Ibaraki 314-0255 (JP)**
- **MIYAKE, Takaaki**
  **Kamisu-shi**
  **Ibaraki 314-0255 (JP)**
- **KOBAYASHI, Takeru**
  **Kamisu-shi**
  **Ibaraki 314-0255 (JP)**
- **MATSUDA, Ryusei**
  **Kamisu-shi**
  **Ibaraki 314-0255 (JP)**
- **ASANO, Shu**
  **Kamisu-shi**
  **Ibaraki 314-0255 (JP)**
- **KUDAMATSU, Akio**
  **Kawasaki-shi**
  **Kanagawa 211-0022 (JP)**

(74) Representative: **Gille Hrabal**
**Brucknerstrasse 20**
**40593 Düsseldorf (DE)**

(56) References cited:
**WO-A1-2009/099929**
**WO-A1-2012/136724**
**WO-A2-2011/017334**
**WO-A2-2011/017342**
**JP-A- 2014 024 829**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 287 457 B1

## Description

[Technical Field]

The present invention relates to a mesoionic compound, a manufacturing method thereof, and a harmful organism control agent containing the same as active ingredient.

[Background Art]

In Patent Document 1 and Patent Document 2, specific mesoionic pyrimidinium compounds are disclosed as insecticides.

[Prior Art Documents]

[Patent Document]

Patent Document 1: International Publication No. WO2011/017342 pamphlet
Patent Document 2: International Publication No. WO2012/092115 pamphlet

[Summary of Invention]

[Technical Problem]

The compounds described in Patent Document 1 and Patent Document 2 do not necessarily exhibit sufficient controlling effect against various harmful organisms. Furthermore, many of the harmful organisms easily acquire resistance to insecticides to considerably lessen the control effects of existing chemical agents. Accordingly, the demand for novel active ingredients of insecticides having a chemical structure, an action mechanism, or a site of action different from those of conventional insecticidal active compounds is always present.
An object of the present invention is to provide a novel mesoionic compound or a salt thereof as active ingredient of insecticides having a more excellent controlling activity against various harmful organisms.

[Solution to Problem]

Through extensive study of the technical problem, the present inventors found that the modification of the chemical structure of conventional mesoionic compounds considerably improves the controlling activity against various harmful organisms. Through further investigation, the present invention has been accomplished.
The present invention relates to the following:

"(1) A compound represented by a formula (1) or a salt thereof:

[Formula 1]

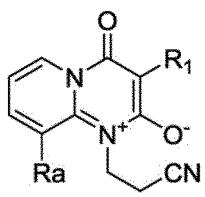

(1)

wherein

Ra represents a hydrogen atom, a halogen atom or a $C_1$ alkyl group, wherein the C1 alkyl group is optionally substituted with one or more of the same or different halogen atoms;

$R_1$ represents a phenyl group or a pyridyl group, which is optionally substituted with up to three substituents $R_2$; wherein
each $R_2$ represents a halogen atom;
a cyano group;
a C1-C4 alkyl group,
wherein the C1-C4 alkyl group is optionally substituted with
one or more of the same or different halogen atoms, and/or
a C1-C3 alkyloxy group which is optionally substituted with one or more of the same or different halogen atoms, and/or
a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms;
a C2-C3 alkenyl group,
wherein the C2-C3 alkenyl group is optionally substituted with one or more of the same or different halogen atoms;
a C2-C3 alkynyl group,
wherein the C2-C3 alkynyl group is optionally substituted with one or more of the same or different halogen atoms;
a C1-C5 alkyloxy group,
wherein the C1-C5 alkyloxy group is optionally substituted with
one or more of the same or different halogen atoms, and/or
a C1-C3 alkyloxy group which is optionally substituted with one or more of the same or different halogen atoms, and/or
a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms;
an amino group having two substituents selected from a hydrogen atom, a C1-C4 alkyl group, and a C2 acyl group,
wherein the C1-C4 alkyl group is optionally substituted with
one or more of the same or different halogen atoms, and/or
a C1-C3 alkyloxy group which is optionally substituted with one or more of the same or different halogen atoms, and/or
a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms; and
wherein the C2 acyl group is optionally substituted with one or more of the same or different halogen atoms; or
a C1-C4 alkylthio group, a C1-C4 alkylsulfoxy group, or a C1-C4 alkylsulfonyl group,
wherein the C1-C4 alkylthio group, the C1-C4 alkylsulfoxy group, or the C1-C4 alkylsulfonyl group may be substituted with a substituent selected from the group consisting of:
one or more of the same or different halogen atoms, and/or
a C1-C3 alkyloxy group which is optionally substituted with one or more of the same or different halogen atoms, and/or
a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms;

(2) The compound or the salt thereof described in item (1), wherein,
Ra is a hydrogen atom or a methyl group; and
$R_1$ is a phenyl group which is optionally substituted with up to two substituents $R_2$; wherein

each $R_2$ is independently selected from the group consisting of:

a halogen atom,
a C1-C4 alkyl group which is optionally substituted with one or more of the same or different halogen atoms,
a C2-C3 alkenyl group which is optionally substituted with one or more of the same or different halogen atoms,
a C2-C3 alkynyl group which is optionally substituted with one or more of the same or different halogen atoms,
a C1-C5 alkyloxy group which is optionally substituted with one or more of the same or different halogen atoms,
an N-alkylacetamide group, wherein the alkyl group is C1-C4 and is optionally substituted with one or more of the same or different halogen atoms,
an N-alkyltrifluoroacetamide group, wherein the alkyl group is C1-C4 and is optionally substituted with one or more of the same or different halogen atoms,
an acetamide group,
a trifluoroacetamide group,
a C1-C4 alkylthio group which is optionally substituted with one or more of the same or different halogen atoms,
a C1-C4 alkylsulfoxy group which is optionally substituted with one or more of the same or different halogen atoms, and
a C1-C4 alkylsulfonyl group which is optionally substituted with one or more of the same or different halogen atoms;

(3) The compound or the salt thereof described in item (1), wherein,
Ra is a hydrogen atom or a methyl group; and
$R_1$ is a phenyl group which is optionally substituted with up to two substituents $R_2$; wherein
each $R_2$ is independently selected from the group consisting of:
a fluorine atom, a chlorine atom, a bromine atom, a methyl group, an ethyl group, an n-propyl group, an i-propyl group, a c-propyl group, an n-butyl group, an i-butyl group, an s-butyl group, a t-butyl group, a fluoromethyl group, a difluoromethyl group, a trifluoromethyl group, a methyloxy group, an ethyloxy group, an n-propyloxy group, an i-propyloxy group, a c-propyloxy group, an n-butyloxy group, an i-butyloxy group, an s-butyloxy group, a t-butyloxy group, an n-pentyloxy group, a trifluoromethyl group, a 2,2,2-trifluoroethyloxy group, a methylthio group, a methylsulfoxy group, a methylsulfonyl group, a vinyl group, and an ethynyl group;
(4) The compound and the salt thereof described in item (1), wherein the compound represented by the formula (1) is one selected from the group consisting of:

1-(2-cyanoethyl)-2-hydroxy-4-oxo-3-phenyl-4H-pyrido[1,2-a]pyrimidinium inner salt (i.e., Compound 1 in Table 1);
1-(2-cyanoethyl)-2-hydroxy-4-oxo-3-(3-toluyl)-4H-pyrido[1,2-a]pyrimidinium inner salt (i.e., Compound 3 in Table 1);
1-(2-cyanoethyl)-3-(3-ethylphenyl)-2-hydroxy-4-oxo-4H-pyrido[1,2-a]pyrimidinium inner salt (i.e., Compound 6 in Table 1);
1-(2-cyanoethyl)-2-hydroxy-3-(3-isopropylphenyl)-4-oxo-4H-pyrido[1,2-a]pyrimidinium inner salt (i.e., Compound 12 in Table 1;
1-(2-cyanoethyl)-2-hydroxy-3-(3-tert-butylphenyl)-4-oxo-4H-pyrido[1,2-a]pyrimidinium inner salt (i.e., Compound 15 in Table 1);
1-(2-cyanoethyl)-2-hydroxy-3-(3-methoxyphenyl)-4-oxo-4H-pyrido[1,2-a]pyrimidinium inner salt (i.e., Compound 18 in Table 1);
1-(2-cyanoethyl)-3-(3-ethoxyphenyl)-2-hydroxy-4-oxo-4H-pyrido[1,2-a]pyrimidinium inner salt (i.e., Compound 21 in Table 1);
1-(2-cyanoethyl)-2-hydroxy-(3-n-propoxyphenyl)-4-oxo-3-4H-pyrido[1,2-a]pyrimidinium inner salt (i.e., Compound 24 in Table 1);
1-(2-cyanoethyl)-2-hydroxy-3-(3-isopropoxyphenyl)-4-oxo-4H-pyrido[1,2-a]pyrimidinium inner salt (i.e., Compound 27 in Table 1);
1-(2-cyanoethyl)-2-hydroxy-3-(3-n-butoxyphenyl)-4-oxo-4H-pyrido[1,2-a]pyrimidinium inner salt (i.e., Compound 30 in Table 1);
1-(2-cyanoethyl)-2-hydroxy-3-(3-n-pentyloxyphenyl)-4-oxo-4H-pyrido[1,2-a]pyrimidinium inner salt (i.e., Compound 36 in Table 1);
1-(2-cyanoethyl)-3-(2-fluorophenyl)-2-hydroxy-4-oxo-4H-pyrido[1,2-a]pyrimidinium inner salt (i.e., Compound 38 in Table 1);
1-(2-cyanoethyl)-3-(3-fluorophenyl)-2-hydroxy-4-oxo-4H-pyrido[1,2-a]pyrimidinium inner salt (i.e., Compound 39 in Table 1);

3-(3-chlorophenyl)-1-(2-cyanoethyl)-2-hydroxy-4-oxo-4H-pyrido[1,2-a]pyrimidinium inner salt (i.e., Compound 42 in Table 1);

3-(3-bromophenyl)-1-(2-cyanoethyl)-2-hydroxy-4-oxo-4H-pyrido[1,2-a]pyrimidinium inner salt (i.e., Compound 45 in Table 1);

1-(2-cyanoethyl)-3-(3-difluoromethylphenyl)-2-hydroxy-4-oxo-4H-pyrido[1,2-a]pyrimidinium inner salt (i.e., Compound 48 in Table 1);

1-(2-cyanoethyl)-2-hydroxy-4-oxo-3-(3-trifluoromethylphenyl)-4H-pyrido[1,2-a]pyrimidinium inner salt (i.e., Compound 51 in Table 1);

1-(2-cyanoethyl)-3-(3-difluoromethoxyphenyl)-2-hydroxy-4-oxo-4H-pyrido[1,2-a]pyrimidinium inner salt (i.e., Compound 54 in Table 1);

1-(2-cyanoethyl)-2-hydroxy-4-oxo-3-(3-trifluoromethoxyphenyl)-4H-pyrido[1,2-a]pyrimidinium inner salt (i.e., Compound 57 in Table 1);

1-(2-cyanoethyl)-2-hydroxy-4-oxo-3-(4-trifluoromethoxyphenyl)-4H-pyrido[1,2-a]pyrimidinium inner salt (i.e., Compound 58 in Table 1);

1-(2-cyanoethyl)-2-hydroxy-4-oxo-3-(3-(2,2,2-trifluoroethoxy)phenyl)-4H-pyrido[1,2-a]pyrimidinium inner salt (i.e., Compound 60 in Table 1);

1-(2-cyanoethyl)-2-hydroxy-3-(3-methylthiophenyl)-4-oxo-4H-pyrido[1,2-a]pyrimidinium inner salt (i.e., Compound 63 in Table 1);

1-(2-cyanoethyl)-2-hydroxy-3-(3-ethylthiophenyl)-4-oxo-4H-pyrido[1,2-a]pyrimidinium inner salt (i.e., Compound 66 in Table 1);

3-(3-acetamidophenyl)-1-(2-cyanoethyl)-2-hydroxy-4-oxo-4H-pyrido[1,2-a]pyrimidinium inner salt (i.e., Compound 78 in Table 1);

1-(2-cyanoethyl)-2-hydroxy-3-(3-(N-methylacetamide)phenyl)-4-oxo-4H-pyrido[1,2-a]pyrimidinium inner salt (i.e., Compound 84 in Table 1);

1-(2-cyanoethyl)-2-hydroxy-4-oxo-3-(3-vinylphenyl)-4H-pyrido[1,2-a]pyrimidinium inner salt (i.e., Compound 90 in Table 1);

1-(2-cyanoethyl)-2-hydroxy-3-(3,5-dimethylphenyl)-4-oxo-4H-pyrido[1,2-a]pyrimidinium inner salt (i.e., Compound 100 in Table 1);

1-(2-cyanoethyl)-2-hydroxy-3-(2,5-dimethoxyphenyl)-4-oxo-4H-pyrido[1,2-a]pyrimidinium inner salt (i.e., Compound 103 in Table 1);

3-(3,5-dichlorophenyl)-1-(2-cyanoethyl)-2-hydroxy-4-oxo-4H-pyrido[1,2-a]pyrimidinium inner salt (i.e., Compound 112 in Table 1);

1-(2-cyanoethyl)-3-(4-fluoro-3-methylphenyl)-2-hydroxy-4-oxo-4H-pyrido[1,2-a]pyrimidinium inner salt (i.e., Compound 140 in Table 1);

3-(3-chloro-6-methoxyphenyl)-1-(2-cyanoethyl)-2-hydroxy-4-oxo-4H-pyrido[1,2-a]pyrimidinium inner salt (i.e., Compound 168 in Table 1);

3-(2-chloro-5-trifluoromethylphenyl)-1-(2-cyanoethyl)-2-hydroxy-4-oxo-4H-pyrido[1,2-a]pyrimidinium inner salt (i.e., Compound 183 in Table 1);

1-(2-cyanoethyl)-2-hydroxy-3-(4-methyl-3-trifluoromethylphenyl)-4-oxo-4H-pyrido[1,2-a]pyrimidinium inner salt (i.e., Compound 200 in Table 1);

1-(2-cyanoethyl)-2-hydroxy-8-methyl-4-oxo-3-(3-toluyl)-4H-pyrido[1,2-a]pyrimidinium inner salt (i.e., Compound 223 in Table 1);

1-(2-cyanoethyl)-3-(3-ethoxyphenyl)-2-hydroxy-8-methyl-4-oxo-4H-pyrido[1,2-a]pyrimidinium inner salt (i.e., Compound 241 in Table 1);

1-(2-cyanoethyl)-2-hydroxy-8-methyl-4-oxo-3-(3-trifluoromethylphenyl)-4H-pyrido[1,2-a]pyrimidinium inner salt (i.e., Compound 271 in Table 1); and

1-(2-cyanoethyl)-3-(3,5-dichlorophenyl)-2-hydroxy-8-methyl-4-oxo-4H-pyrido[1,2-a]pyrimidinium inner salt (i.e., Compound 333 in Table 1);

(5) The compound or the salt thereof described in item (1), wherein the compound represented by the formula (1) is one selected from the group consisting of:

1-(2-cyanoethyl)-2-hydroxy-4-oxo-3-(3-toluyl)-4H-pyrido[1,2-a]pyrimidinium inner salt (i.e., Compound 3 in Table 1);

1-(2-cyanoethyl)-2-hydroxy-3-(3-methoxyphenyl)-4-oxo-4H-pyrido[1,2-a]pyrimidinium inner salt (i.e., Compound 18 in Table 1);

1-(2-cyanoethyl)-3-(3-ethoxyphenyl)-2-hydroxy-4-oxo-4H-pyrido[1,2-a]pyrimidinium inner salt (i.e., Compound 21 in Table 1); and

1-(2-cyanoethyl)-2-hydroxy-4-oxo-3-(3-trifluoromethylphenyl)-4H-pyrido[1,2-a]pyrimidinium inner salt (i.e., Compound 51 in Table 1);

(6) An insecticidal composition comprising the compound or the salt described in items (1) to (5), and at least one component selected from the group consisting of a surfactant, a solid diluent and a liquid diluent;
(7) The insecticidal composition described in item (6) further comprising at least one biologically active compound or chemical agent;
(8) The composition described in item (7), wherein the at least one biologically active compound or chemical agent is selected from the group consisting of:
alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, metolcarb, fenothiocarb, fenoxycarb, acephate, azamethiphos, azinphos-ethyl, azinphos-methyl, ethylthiometon, chloroethoxyfos, cadusafos, chlorethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, EPN, ethion, ethoprophos, famphur, fenamiphos, fenitrothion, fenthion, fosthiazate, heptenophos, imicyafos, isofenphos, isopropyl=O-(methoxy aminothio phosphoryl), salicylates, isoxathion, malathion, mecarbam, methamidophos, methidathion, mevinphos, monocrotophos, naled, omethoate, oxydemeton ethyl, parathion, parathion-methyl, PAP, phorate, phosalone, phosmet, phosphamidon, phoxim, pirimiphos-methyl, profenofos-propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, chlorpyrifos-ethyl, disulfoton, sulprofos, flupyrazofos, phenthoate, fonofos, tribufos, endosulfan, alpha-endosulfan, gamma-HCH, dicofol, chlordane, dieldrin, methoxychlor, acetoprole, fipronil, ethiprole, pyrafluprole, pyriprole, flufiprole, broflanilide, afoxolaner, fluralaner, sarolaner, acrinathrin, allethrin, d-cis-trans allethrin, d-trans allethrin, bifenthrin, kappa-bifenthrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, ethofenprox, fenpropathrin, fenvalerate, flucythrinate, flumethrin, tau-fluvalinate, halfenprox, imiprothrin, kadethrin, permethrin, phenothrin, prallethrin, pyrethrin, resmethrin, silafluofen, tefluthrin, kappa-tefluthrin, phthalthrin, tetramethrin, tralomethrin, transfluthrin, methoxadiazone, metofluthrin, profluthrin, pyrethrum, terallethrin, momfluorothrin, heptafluthrin, meperfluthrin, tetramethylfluthrin, dimefluthrin, protrifenbute, acetamiprid, clothianidin, dinotefuran, imidacloprid, nitenpyram, thiacloprid, thiamethoxam, sulfoxaflor, flupyradifurone, triflumezopyrim, dicloromezotiaz, spinosad, spinetoram, abamectin, ivermectin, emamectin benzoate, milbemectin, lepimectin, hydroprene, kinoprene, diofenolan, methoprene, pyriproxyfen, pymetrozine, flonicamid, etoxazole, diafenthiuron, azocyclotin, cyhexatin, fenbutatin oxide, propargite, tetradifon, chlorfenapyr, tralopyril, DNOC, bensultap, cartap, thiocyclam, thiosultap, thiosultap-sodium, bistrifluron, chlorfluazuron, diflubenzuron, flucycloxuron, flufenoxuron, hexaflumuron, lufenuron, novaluron, noviflumuron, teflubenzuron, triflumuron, bistrifluron, buprofezin, cyromazine, chromafenozide, halofenozide, methoxyfenozide, tebufenozide, amitraz, hydramethylnon, acequinocyl, fluacrypyrim, pyriminostrobin, flufenoxystrobin, fenazaquin, fenpyroximate, pyrimidifen, pyridaben, tebufenpyrad, tolfenpyrad, pyflubumide, spirodiclofen, spirotetramat, spiromesifen, cyflumetofen, cyenopyrafen, flubendiamide, chlorantraniliprole, cyantraniliprole, cyclaniliprole, tetraniliprole, quinomethionate, hexythiazox, bifenazate, flufenerim, pyrifluquinazon, flometoquin, fluopyram, 8-chloro-N-((2-chloro-5-methoxyphenyl)sulfonyl)-6-(trifluoromethyl)imidazo[1,2-a]pyridine-2-carboxamide], amidoflumet, nicotine, chloropicrin, sulfuryl fluoride, chlorothie, clofentezine, diflovidazin, rotenone, indoxacarb, piperonyl butoxide, chlordimeform, pyridalyl, azadirachtin, benzoxymate, afidopiropen, fluhexafon, tioxazafen, fluensulfone, benclothiaz, carzole, an insecticidal soap, dimehypo, nithiazine, a borate, metaldehyde, ryanodine, sulfluramid, (E)-N-(1-((6-chloropyridin-3-yl)methyl)pyridine-2(1H)-ylidene)-2,2,2-trifluoroacetamide, 5-(trifluoromethyl)-2-(4-(4-(trifluoromethyl)phenoxy)piperidin-1-yl)pyridine, metalaxyl, metalaxyl-M, oxadixyl, ofurace, benalaxyl, benalaxyl-M, kiralaxyl, ofurace, furalaxyl, cyprofuram, bupirimate, dimethirimol, ethirimol, hymexazol, hydroxyisoxazole, oxathiapiprolin, octhilinone, oxolinic acid, benomyl, thiophanate-methyl, carbendazim, fuberidazole, thiabendazole, debacarb, diethofencarb, zoxamide, ethaboxam, pencycuron, fluopicolide, diflumetorim, bupirimate, benodanil, flutolanil, mepronil, isofetamid, fenfuram, oxycarboxin, carboxin, thifluzamide, fluxapyroxad, furametpyr, penflufen, penthiopyrad, benzovindiflupyr, bixafen, isopyrazam, sedaxane, boscalid, azoxystrobin, coumetoxystrobin, kresoxym-methyl, trifloxystrobin, picoxystrobin, pyraclostrobin, dimoxystrobin, metominostrobin, orysastrobin, fluoxastrobin, pyraoxystrobin, pyrametostrobin, flufenoxystrobin, fenaminstrobin, enoxastrobin, coumoxystrobin, mandestrobin, triclopyricarb, famoxadon, fenamidone, triclopyricarb, pyribencarb, cyazofamid, amisulbrom, binapacryl, meptyldinocarb, dinocap, fluazinam, ferimzone, fentin-acetate, fentin chloride, fentin hydroxide, triphenyltin hydroxide, triphenyltin acetate, oxine-copper, silthiofam, ametoctradin, mepanipyrim, nitrapyrin, pyrimethanil, cyprodinil, blasticidin S, kasugamycin, kasugamycin hydrochloride hydrate, streptomycin, oxytetracyclin, quinoxyfen, proquinazid, fludioxonil, fenpiclonil, fluoroimide, procymidone, iprodione, vinclozolin, edifenphos, iprobenfos, pyrazophos, isoprothiolane, propamocarb, propamocarb hydrochloride, tea tree oil extract, triforine, pyrifenox, pyrisoxazole, fenarimol, nuarimol, azaconazole, bromuconazole, diniconazole, diniconazole-M, epoxiconazole, fluquinconazole, oxpoconazole, pefurazoate, difenoconazole, fenbuconazole, imibenconazole, ipconazole, metconazole, tetraconazole, triadimefon, triadimenol, triticonazole, uniconazole, imazalil, bitertanol, triflumizole, etaconazole, propiconazole, penconazole, flusilazole, flutriafol, myclobutanil, paclobutrazol, prothioconazole, cyproconazole, tebuconazole, hexaconazole, prochloraz, simeconazole, aldimorph, dodemorph, dodemorph acetate, tridemorph, fenpropimorph, dimethomorph, flumorph, pyrimorph, piperalin, fenpropidin, spiroxamine, fenhexamid, fenpyrazamine, ferbam, metam, methasulfocarb, metiram, thiram, manzeb, maneb, zineb, ziram, polycarbamate, propineb, thiuram, pyributicarb, validamycin, mildiomycin, polyoxin, benthiavalicarb, benthiavalicarb-isopropyl, valifenalate, iprovalicarb, mandipropamid, fthalide, pyroquilon, tricyclazole, carpropamid, diclocymet, fenoxanil, acibenzolar-S-methyl, probenazole, tiadinil, isotianil, cymoxanil, fosetyl, tecloftalam, triazoxide, flusulfamide, diclomezine, cyflufenamid, metrafenone, pyriofenon, flutianil, tebufloquin, fosetyl aluminum, tolclofos-methyl, echlomezol, tolprocarb, Bordeaux mixture, copper acetate, basic copper sulfate, copper oxychloride, cupric hydroxide, oxyquinoline copper, copper, sulfur, captan, captafol, folpet, anilazine, chlorothalonil, dichlorophene, pentachlorophenol and salts thereof, hexachlorobenzene, quintozene, iminoctadine acetate, iminoctadine albesilate, guanidine, dodine, dodine free base, guazatine, guazatine acetate, albesilate, dithianon, quinomethionate, fluoroimide, tolylfluanid, dichlofluanid, dinobuton, dazomet, dipymetitrone, pyraziflumid, picarbutrazox, tecnazene, nitrothal-isopropyl, dicyclomet, acibenzolar, prohexadione-calcium, bronopol, diphenylamine, flumetover, bentoxazin, biphenyl, chloroneb, CNA, iodocarb, prothiocarb, N-(1-(2,4-dichlorophenyl)-1-methoxypropan-2-yl)-2-iodobenzamide, 3-(5-fluoro-3,3,4,4-tetramethyl-3,4-dihydroisoquinolin-1-yl)quinoline, 3-(4,4-difluoro-3,3-dimethyl-3,4-dihydroisoquinolin-1-yl)quinoline, 3-(4,4,5-trifluoro-3,3-dimethyl-3,4-dihydroisoquinolin-1-yl)quinoline, Bacillus species and insecticidal proteins and bactericidal proteins produced therefrom, and insecticidal proteins, bactericidal proteins, entomopathogenic bacteria, entomopathogenic viruses and entomopathogenic fungi produced from Bt crops;

(9) A composition for protecting animals from harmful parasitic invertebrate organisms, comprising the compound or the salts thereof described in item (1) in a parasiticidally effective amount and at least one carrier;

(10) A method for controlling harmful invertebrate organisms, comprising bringing the harmful invertebrate organisms or the environment thereof into contact with the compound or the salt thereof described in item (1) in a biologically effective amount, wherein the environment thereof does not encompass the human or animal body;

(11) A method for enhancing the vitality of crops, comprising bringing the crops, seeds from which crops will grow, or the base of crops into contact with the compound or the salt thereof described in item (1) in a biologically effective amount; and

(12) A treated seed comprising the compound or the salt thereof described in item (1) in an amount of about 0.0001 to 1 mass% relative to the seed after treatment."

The present invention provides a composition comprising the compound of the formula (1) and at least one component selected from the group consisting of a surfactant, a solid diluent and a liquid diluent. In an embodiment, the present invention provides a composition for controlling harmful invertebrate organisms comprising the compound of the formula (1) and at least one component selected from the group consisting of a surfactant, a solid diluent and a liquid diluent, wherein the compound may further comprise at least one biologically active compound or chemical agent.

[Effects of Invention]

A mesoionic compound represented by the formula (1) according to the present invention having a cyanoethyl group bonded to the 1-position exhibits excellent controlling effects against harmful organisms, being useful as a harmful organism-controlling agent.

[Description of Embodiments]

A formula (1) provides a general definition of the compounds according to the present invention. Preferred substituents and/or the scope of the substituents in the compounds represented by the formula (1) are specifically described below.

In the present specification, a halogen atom means a fluorine atom, a chlorine atom, a bromine atom or an iodine atom; "n-" means normal, "i-" means iso, "s-" means secondary, "c-" means cyclo, and "t-" means tertiary; "Me" means a methyl group, "Et" means an ethyl group, "Pr" means a propyl group, "Bu" means a butyl group, "Pen" means a pentyl group, and "Ac" means an acetyl group. Furthermore, a numerical range shown by using "-" indicates a range including the respective numerical values prepositioned and post-positioned relative to "-" as the minimum and the maximum.

Ra in the compound of the formula (1) is a hydrogen atom, a halogen atom, or a $C_1$ alkyl group which is optionally

substituted with one or more of the same or different halogen atoms.

Specific examples thereof include a hydrogen atom, a fluorine atom, a chlorine atom, a bromine atom, an iodine atom, a methyl group, a fluoromethyl group, a difluoromethyl group, a trifluoromethyl group, and a trichloromethyl group.

$R_1$ in the compound of the formula (1) is a phenyl group or a pyridyl group which is optionally substituted with up to three substituents $R_2$; wherein

each $R_2$ is independently selected from the group consisting of: a halogen atom, a cyano group, any straight or branched chain C1-C4 alkyl group, any straight or branched chain C2-C3 alkenyl group, any straight or branched chain C1-C5 alkyloxy group, an amino group having two substituents selected from a hydrogen atom, any straight or branched chain C1-C4 alkyl group and an acetyl group, any straight or branched chain C1-C4 alkylthio group, a C1-C4 alkylsulfoxy group, and a C1-C4 alkylsulfonyl group.

The C1-C4 alkyl group represented by each $R_2$ may have a substituent, and examples of the substituent include one or more of the same or different halogen atoms, a straight or branched chain C1-C3 alkyloxy group which is optionally substituted with one or more of the same or different halogen atoms, a straight or branched chain C1-C3 alkylthio group which is optionally substituted with one or more of the same or different halogen atoms, a straight or branched chain C1-C3 sulfoxy group which is optionally substituted with one or more of the same or different halogen atoms, and a straight or branched chain C1-C3 sulfonyl group which is optionally substituted with one or more of the same or different halogen atoms.

Specific examples thereof include a methyl group, an ethyl group, an n-propyl group, an i-propyl group, a c-propyl group, an n-butyl group, an i-butyl group, an s-butyl group, a t-butyl group, a chloromethyl group, a dichloromethyl group, a trichloromethyl group, a fluoromethyl group, a difluoromethyl group, a trifluoromethyl group, a 2,2,2-trifluoroethyl group, a 1,1,2,2-tetrafluoroethyl group, a pentafluoroethyl group, a heptafluoro-n-propyl group, a 1,1,1,3,3,3-hexafluoro-2-propyl group, a heptafluoro-i-propyl group, a 1,1,1,3,3,3-hexafluoro-2-methyloxy-2-propyl group, a methyloxymethyl group, an ethyloxymethyl group, an n-propyloxymethyl group, a 2,2,2-trifluoroethyloxymethyl group, a 3,3,3-trifluoro-n-propyloxymethyl group, and a 2,2,3,3,3-pentafluoro-n-propyloxymethyl group.

The C2-C3 alkenyl group represented by each $R_2$ may have a substituent, and examples of the substituent include one or more of the same or different halogen atoms.

Specific examples thereof include a vinyl group, a 1-propenyl group, a 2-propenyl group, and a 1-propen-2-yl group.

The C2-C3 alkynyl group represented by each $R_2$ may have a substituent, and examples of the substituent include one or more of the same or different halogen atoms.

Specific examples thereof include an ethynyl group, a 1-propyn-1-yl group, and a 2-propyn-1-yl group.

The C1-C4 alkyloxy group represented by each $R_2$ may have a substituent, and examples of the substituent include one or more of the same or different halogen atoms, a straight or branched chain C1-C3 alkyloxy group which is optionally substituted with one or more of the same or different halogen atoms, a straight or branched chain C1-C3 alkylthio group which is optionally substituted with one or more of the same or different halogen atoms, a straight or branched chain C1-C3 sulfoxy group which is optionally substituted with one or more of the same or different halogen atoms, and a straight or branched chain C1-C3 sulfonyl group which is optionally substituted with one or more of the same or different halogen atoms.

Specific examples thereof include a methyloxy group, an ethyloxy group, an n-propyloxy group, an i-propyloxy group, a c-propyloxy group, an n-butyloxy group, an i-butyloxy group, an s-butyloxy group, a t-butyloxy group, n-pentyloxy, a methyloxymethyloxy group, an ethyloxymethyloxy group, a methylthiomethyloxy group, a 2-methyloxyethyloxy group, a 2-ethyloxyethyloxy group, a 2-thiomethylethyloxy group, a difluoromethyloxy group, a trifluoromethyloxy group, a chlorodifluoromethyloxy group, a 2,2,2-trifluoroethyloxy group, a 1,1,2,2-tetrafluoroethyloxy group, and a 1,1,1,3,3,3-hexafluoro-2-propyloxy group.

In the amino group represented by each $R_2$ having two substituents selected from a hydrogen atom, a C1-C4 alkyl group and an acetyl group, the C1-C4 alkyl group may have a substituent including, for example, one or more of the same or different halogen atoms, a straight or branched chain C1-C3 alkyloxy group which is optionally substituted with one or more of the same or different halogen atoms, a straight or branched chain C1-C3 alkylthio group which is optionally substituted with one or more of the same or different halogen atoms, a straight or branched chain C1-C3 sulfoxy group which is optionally substituted with one or more of the same or different halogen atoms, and a straight or branched chain C1-C3 sulfonyl group which is optionally substituted with one or more of the same or different halogen atoms, and the acetyl group may be substituted with one or more of the same or different halogen atoms.

Specific examples of the C1-C4 alkyl group include a methyl group, an ethyl group, an n-propyl group, an i-propyl group, a c-propyl group, an n-butyl group, an i-butyl group, an s-butyl group, a t-butyl group, a trichloromethyl group, a fluoromethyl group, a difluoromethyl group, a trifluoromethyl group, and a 2,2,2-trifluoroethyl group.

Specific examples of the acetyl group include an acetyl group and a trifluoroacetyl group.

In the C1-C4 alkylthio group, the C1-C4 alkylsulfoxy group and the C1-C4 alkylsulfonyl group, the C1-C4 alkyl group may have a substituent, and examples of the substituent include one or more of the same or different halogen

atoms, a straight or branched chain C1-C3 alkyloxy group which is optionally substituted with one or more of the same or different halogen atoms, a straight or branched chain C1-C3 alkylthio group which is optionally substituted with one or more of the same or different halogen atoms, a straight or branched chain C1-C3 sulfoxy group which is optionally substituted with one or more of the same or different halogen atoms, and a straight or branched chain C1-C3 sulfonyl group which is optionally substituted with one or more of the same or different halogen atoms.

Specific examples thereof include a methylthio group, an ethylthio group, an n-propylthio group, an i-propylthio group, a c-propylthio group, an n-butylthio group, a methyloxymethylthio group, an ethyloxymethylthio group, a methylthiomethylthio group, a difluoromethylthio group, a trifluoromethylthio group, a chlorodifluoromethylthio group, a 2,2,2-trifluoroethylthio group, a methylsulfoxy group, an ethylsulfoxy group, an n-propylsulfoxy group, an i-propylsulfoxy group, a c-propylsulfoxy group, an n-butylsulfoxy group, a methyloxymethylsulfoxy group, an ethyloxymethylsulfoxy group, a difluoromethylsulfoxy group, a trifluoromethylsulfoxy group, a chlorodifluoromethylsulfoxy group, a 2,2,2-trifluoroethylsulfoxy group, a methylsulfonyl group, an ethylsulfonyl group, an n-propylsulfonyl group, an i-propylsulfonyl group, a c-propylsulfonyl group, an n-butylsulfonyl group, a methyloxymethylsulfonyl group, an ethyloxymethylsulfonyl group, a difluoromethylsulfonyl group, a trifluoromethylsulfonyl group, a chlorodifluoromethylsulfonyl group, and a 2,2,2-trifluoroethylsulfonyl group.

According to a preferred embodiment of the present invention, Ra is a hydrogen atom, a halogen atom, a methyl group, or a trifluoromethyl group, $R_1$ is a phenyl group or a pyridyl group which is optionally substituted with up to three substituents $R_2$, and each $R_2$ is independently selected from the group consisting of a halogen atom, a C1-C4 alkyl group which is optionally substituted with one or more of the same or different halogen atoms, a C2-C3 alkenyl group which is optionally substituted with one or more of the same or different halogen atoms, a C2-C3 alkynyl group which is optionally substituted with one or more of the same or different halogen atoms, a C1-C5 alkyloxy group which is optionally substituted with one or more of the same or different halogen atoms, an N-substituted C1-C4 alkylacetamide group, a substituted C1-C4 alkyltrifluoroacetamide group having an alkyl group which is optionally substituted with one or more of the same or different halogen atoms, a C1-C4 alkylthio group which is optionally substituted with one or more of the same or different halogen atoms, a C1-C4 sulfoxy group which is optionally substituted with one or more of the same or different halogen atoms, and a C1-C4 sulfonyl group which is optionally substituted with one or more of the same or different halogen atoms. More preferably, Ra is a hydrogen atom or methyl.

More preferably, Ra is a hydrogen atom or a methyl group, and each $R_2$ is independently selected from the group consisting of: a halogen atom, methyl, ethyl, an n-propyl group, an i-propyl group, a c-propyl group, an n-butyl group, an i-butyl group, an s-butyl group, a t-butyl group, an n-pentyl group, a fluoromethyl group, a difluoromethyl group, a trifluoromethyl group, a methyloxy group, an ethyloxy group, an n-propyloxy group, an i-propyloxy group, a c-propyloxy group, an n-butyloxy group, an i-butyloxy group, an s-butyloxy group, a t-butyloxy group, an n-pentyloxy group, a trifluoromethyloxy group, a 2,2,2-trifluoroethoxy group, an N-methylacetamide group, a 2,2,2-trifluoroethylacetamide group, an N-methyltrifluoroacetamide group, a 2,2,2-trifluoroethyltrifluoroacetamide group, a methylthio group, a methylsulfoxy group, a methylsulfonyl group, a vinyl group, and an ethynyl group.

Still more preferably, Ra is a hydrogen atom or a methyl group and $R_1$ is a phenyl which is optionally substituted with up to two substituents $R_2$, wherein each $R_2$ is independently selected from the group consisting of: a halogen atom, methyl, ethyl, an n-propyl group, an i-propyl group, a c-propyl group, an n-butyl group, an i-butyl group, an s-butyl group, a t-butyl group, an n-pentyl group, a trifluoromethyl group, a methyloxy group, an ethyloxy group, an n-propyloxy group, an i-propyloxy group, a c-propyloxy group, an n-butyloxy group, an i-butyloxy group, an s-butyloxy group, a t-butyloxy group, an n-pentyloxy group, a trifluoromethyloxy group, an N-methylacetamide group, and a methylthio group.

In the compounds or salts thereof of the present invention, the most preferred Ra is a hydrogen atom.

In the compounds or the salts thereof of the present invention having a phenyl as $R_1$, preferably $R_1$ is a substituted phenyl having a substituent at the 3-position, more preferably $R_1$ is a substituted phenyl having a substituent at the 3-position only. Among the compounds or the salts thereof of the present invention, those having a hydrogen atom as Ra and a substituted phenyl with a substituent at the 3-position as $R_1$ are preferred, and those having a hydrogen atom as Ra and a substituted phenyl with a substituent at the 3-position only as $R_1$ are more preferred. In these cases, the preferred substituent of the phenyl group for constituting $R_1$, i.e., $R_2$, is as follows:

a C1-C4 alkyl group which is optionally substituted with one or more of the same or different halogen atoms;
a C2-C3 alkenyl group which is optionally substituted with one or more of the same or different halogen atoms;
a C2-C3 alkynyl group which is optionally substituted with one or more of the same or different halogen atoms;
a C1-C5 alkyloxy group which is optionally substituted with one or more of the same or different halogen atoms;
an N-substituted C1-C4 alkylacetamide group;
a substituted C1-C4 alkyltrifluoroacetamide group having an alkyl group which is optionally substituted with one or more of the same or different halogen atoms;
a C1-C4 alkylthio group which is optionally substituted with one or more of the same or different halogen atoms;
a C1-C4 sulfoxy group which is optionally substituted with one or more of the same or different halogen atoms;

a C1-C4 sulfonyl group which is optionally substituted with one or more of the same or different halogen atoms; or an amino group.

In the case where $R_1$ is substituted with two substituents $R_2$, the two substituents $R_2$ are selected independently from each other.

Examples of more preferred $R_2$ include a halogen atom, methyl, ethyl, an n-propyl group, an i-propyl group, a c-propyl group, an n-butyl group, an i-butyl group, an s-butyl group, a t-butyl group, an n-pentyl group, a trifluoromethyl group, a methyloxy group, an ethyloxy group, an n-propyloxy group, an i-propyloxy group, a c-propyloxy group, an n-butyloxy group, an i-butyloxy group, an s-butyloxy group, a t-butyloxy group, an n-pentyloxy group, a trifluoromethyloxy group, an N-methylacetamide group, and a methylthio group.

Other examples of more preferred $R_2$ include a C1-C4 alkyl group and a C1-C5 alkyloxy group which are optionally substituted with one or more of the same or different halogen atoms, and a $C_1$ alky group and a $C_{1-2}$ alkyloxy group which are optionally substituted with one or more of the same or different halogen atoms are even more preferred, respectively. In the case where $R_1$ is phenyl, the compounds or the salts thereof of the present invention having a substituted phenyl with these groups at the 3-position as $R_1$ are preferred.

The mesoionic compounds with a cyanoethyl group bonded to the 1-position of the present invention may be a free base in a free form, or may be in a form of acid addition salt with a suitable acid. Examples of the specific acid addition salts include inorganic acids such as hydrochlorides, sulfates, nitrates, and phosphates, carboxylic acid salts such as formate, acetate, oxalate, citrate, succinate, maleate, fumarate, tartrate, lactate, benzoate, and phthalate, and sulfonic acid salts such as mesylate, tosylate, and benzenesulfonate.

Further, examples of specific combinations of the substituents Ra, $R_{11}$, $R_{12}$, $R_{13}$, $R_{14}$ and $R_{15}$ in the following formula (i) representing the substituent $R_1$ of the compound of the formula (1) are shown in Table 1; and examples of specific combinations of the substituents Ra, $A_1$, $A_2$, $A_3$, $A_4$ and $A_5$ in the following formula (ii) representing the substituent $R_1$ of the compound of the formula (1) are shown in Table 2; though the present invention is not limited thereto.

[Formula 2]

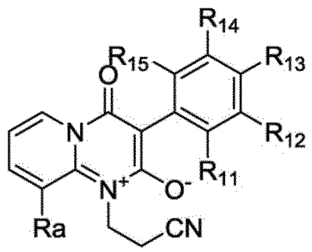

(i)

[Table 1-1]

| Compound Number | Ra | $R_{11}$ | $R_{12}$ | $R_{13}$ | $R_{14}$ | $R_{15}$ |
|---|---|---|---|---|---|---|
| 1 | H | H | H | H | H | H |
| 2 | H | Me | H | H | H | H |
| 3 | H | H | Me | H | H | H |
| 4 | H | H | H | Me | H | H |
| 5 | H | Et | H | H | H | H |
| 6 | H | H | Et | H | H | H |
| 7 | H | H | H | Et | H | H |
| 8 | H | *n*Pr | H | H | H | H |
| 9 | H | H | *n*Pr | H | H | H |
| 10 | H | H | H | *n*Pr | H | H |

(continued)

| Compound Number | Ra | $R_{11}$ | $R_{12}$ | $R_{13}$ | $R_{14}$ | $R_{15}$ |
|---|---|---|---|---|---|---|
| 11 | H | *i*Pr | H | H | H | H |
| 12 | H | H | *i*Pr | H | H | H |
| 13 | H | H | H | *i*Pr | H | H |
| 14 | H | *t*Bu | H | H | H | H |
| 15 | H | H | *t*Bu | H | H | H |
| 16 | H | H | H | *t*Bu | H | H |
| 17 | H | OMe | H | H | H | H |
| 18 | H | H | OMe | H | H | H |
| 19 | H | H | H | OMe | H | H |
| 20 | H | OEt | H | H | H | H |
| 21 | H | H | OEt | H | H | H |
| 22 | H | H | H | OEt | H | H |
| 23 | H | O*n*Pr | H | H | H | H |
| 24 | H | H | O*n*Pr | H | H | H |
| 25 | H | H | H | O*n*Pr | H | H |
| 26 | H | O*i*Pr | H | H | H | H |
| 27 | H | H | O*i*Pr | H | H | H |
| 28 | H | H | H | O*i*Pr | H | H |
| 29 | H | O*n*Bu | H | H | H | H |
| 30 | H | H | O*n*Bu | H | H | H |
| 31 | H | H | H | O*n*Bu | H | H |
| 32 | H | O*i*Bu | H | H | H | H |
| 33 | H | H | O*i*Bu | H | H | H |
| 34 | H | H | H | O*i*Bu | H | H |
| 35 | H | O*n*Pen | H | H | H | H |
| 36 | H | H | O*n*Pen | H | H | H |
| 37 | H | H | H | O*n*Pen | H | H |

[Table 1-2]

| | | | | | | |
|---|---|---|---|---|---|---|
| 38 | H | F | H | H | H | H |
| 39 | H | H | F | H | H | H |
| 40 | H | H | H | F | H | H |
| 41 | H | Cl | H | H | H | H |
| 42 | H | H | Cl | H | H | H |
| 43 | H | H | H | Cl | H | H |
| 44 | H | Br | H | H | H | H |
| 45 | H | H | Br | H | H | H |

(continued)

| | | | | | | |
|---|---|---|---|---|---|---|
| 46 | H | H | H | Br | H | H |
| 47 | H | $CHF_2$ | H | H | H | H |
| 48 | H | H | $CHF_2$ | H | H | H |
| 49 | H | H | H | $CHF_2$ | H | H |
| 50 | H | $CF_3$ | H | H | H | H |
| 51 | H | H | $CF_3$ | H | H | H |
| 52 | H | H | H | $CF_3$ | H | H |
| 53 | H | $OCHF_2$ | H | H | H | H |
| 54 | H | H | $OCHF_2$ | H | H | H |
| 55 | H | H | H | $OCHF_2$ | H | H |
| 56 | H | $OCF_3$ | H | H | H | H |
| 57 | H | H | $OCF_3$ | H | H | H |
| 58 | H | H | H | $OCF_3$ | H | H |
| 59 | H | $OCH_2CF_3$ | H | H | H | H |
| 60 | H | H | $OCH_2CF_3$ | H | H | H |
| 61 | H | H | H | $OCH_2CF_3$ | H | H |
| 62 | H | SMe | H | H | H | H |
| 63 | H | H | SMe | H | H | H |
| 64 | H | H | H | SMe | H | H |
| 65 | H | SEt | H | H | H | H |
| 66 | H | H | SEt | H | H | H |
| 67 | H | H | H | SEt | H | H |
| 68 | H | SOMe | H | H | H | H |
| 69 | H | H | SOMe | H | H | H |
| 70 | H | H | H | SOMe | H | H |
| 71 | H | $SO_2Me$ | H | H | H | H |
| 72 | H | H | $SO_2Me$ | H | H | H |
| 73 | H | H | H | $SO_2Me$ | H | H |
| 74 | H | $NH_2$ | H | H | H | H |
| 75 | H | H | $NH_2$ | H | H | H |

[Table 1-3]

| | | | | | | |
|---|---|---|---|---|---|---|
| 76 | H | H | H | $NH_2$ | H | H |
| 77 | H | NHAc | H | H | H | H |
| 78 | H | H | NHAc | H | H | H |
| 79 | H | H | H | NHAc | H | H |
| 80 | H | $NHCOCF_3$ | H | H | H | H |
| 81 | H | H | $NHCOCF_3$ | H | H | H |

(continued)

| | | | | | | |
|---|---|---|---|---|---|---|
| 82 | H | H | H | $NHCOCF_3$ | H | H |
| 83 | H | NMeAc | H | H | H | H |
| 84 | H | H | NMeAc | H | H | H |
| 85 | H | H | H | NMeAc | H | H |
| 86 | H | $NMeCOCF_3$ | H | H | H | H |
| 87 | H | H | $NMeCOCF_3$ | H | H | H |
| 88 | H | H | H | $NMeCOCF_3$ | H | H |
| 89 | H | $CH=CH_2$ | H | H | H | H |
| 90 | H | H | $CH=CH_2$ | H | H | H |
| 91 | H | H | H | $CH=CH_2$ | H | H |
| 92 | H | C≡CH | H | H | H | H |
| 93 | H | H | C≡CH | H | H | H |
| 94 | H | H | H | C≡CH | H | H |
| 95 | H | Me | Me | H | H | H |
| 96 | H | Me | H | Me | H | H |
| 97 | H | Me | H | H | Me | H |
| 98 | H | Me | H | H | H | Me |
| 99 | H | H | Me | Me | H | H |
| 100 | H | H | Me | H | Me | H |
| 101 | H | OMe | OMe | H | H | H |
| 102 | H | OMe | H | OMe | H | H |
| 103 | H | OMe | H | H | OMe | H |
| 104 | H | OMe | H | H | H | OMe |
| 105 | H | H | OMe | OMe | H | H |
| 106 | H | H | OMe | H | OMe | H |
| 107 | H | Cl | Cl | H | H | H |
| 108 | H | Cl | H | Cl | H | H |
| 109 | H | Cl | H | H | Cl | H |
| 110 | H | Cl | H | H | H | Cl |
| 111 | H | H | Cl | Cl | H | H |
| 112 | H | H | Cl | H | Cl | H |
| 113 | H | F | F | H | H | H |

[Table 1-4]

| | | | | | | |
|---|---|---|---|---|---|---|
| 114 | H | F | H | F | H | H |
| 115 | H | F | H | H | F | H |
| 116 | H | F | H | H | H | F |
| 117 | H | H | F | F | H | H |

(continued)

| | | | | | | |
|---|---|---|---|---|---|---|
| 118 | H | H | F | H | F | H |
| 119 | H | $CF_3$ | $CF_3$ | H | H | H |
| 120 | H | $CF_3$ | H | $CF_3$ | H | H |
| 121 | H | $CF_3$ | H | H | $CF_3$ | H |
| 122 | H | $CF_3$ | H | H | H | $CF_3$ |
| 123 | H | H | $CF_3$ | $CF_3$ | H | H |
| 124 | H | H | $CF_3$ | H | $CF_3$ | H |
| 125 | H | $OCF_3$ | $OCF_3$ | H | H | H |
| 126 | H | $OCF_3$ | H | $OCF_3$ | H | H |
| 127 | H | $OCF_3$ | H | H | $OCF_3$ | H |
| 128 | H | $OCF_3$ | H | H | H | $OCF_3$ |
| 129 | H | H | $OCF_3$ | $OCF_3$ | H | H |
| 130 | H | H | $OCF_3$ | H | $OCF_3$ | H |
| 131 | H | F | Me | H | H | H |
| 132 | H | F | H | Me | H | H |
| 133 | H | F | H | H | Me | H |
| 134 | H | F | H | H | H | Me |
| 135 | H | Me | F | H | H | H |
| 136 | H | H | F | Me | H | H |
| 137 | H | H | F | H | Me | H |
| 138 | H | H | F | H | H | Me |
| 139 | H | Me | H | F | H | H |
| 140 | H | H | Me | F | H | H |
| 141 | H | Cl | Me | H | H | H |
| 142 | H | Cl | H | Me | H | H |
| 143 | H | Cl | H | H | Me | H |
| 144 | H | Cl | H | H | H | Me |
| 145 | H | Me | Cl | H | H | H |
| 146 | H | H | Cl | Me | H | H |
| 147 | H | H | Cl | H | Me | H |
| 148 | H | H | Cl | H | H | Me |
| 149 | H | Me | H | Cl | H | H |
| 150 | H | H | Me | Cl | H | H |
| 151 | H | F | OMe | H | H | H |

[Table 1-5]

| | | | | | | |
|---|---|---|---|---|---|---|
| 152 | H | F | H | OMe | H | H |
| 153 | H | F | H | H | OMe | H |

(continued)

| | | | | | | |
|---|---|---|---|---|---|---|
| 154 | H | F | H | H | H | OMe |
| 155 | H | OMe | F | H | H | H |
| 156 | H | H | F | OMe | H | H |
| 157 | H | H | F | H | OMe | H |
| 158 | H | H | F | H | H | OMe |
| 159 | H | OMe | H | F | H | H |
| 160 | H | H | OMe | F | H | H |
| 161 | H | Cl | OMe | H | H | H |
| 162 | H | Cl | H | OMe | H | H |
| 163 | H | Cl | H | H | OMe | H |
| 164 | H | Cl | H | H | H | OMe |
| 165 | H | OMe | Cl | H | H | H |
| 166 | H | H | Cl | OMe | H | H |
| 167 | H | H | Cl | H | OMe | H |
| 168 | H | H | Cl | H | H | OMe |
| 169 | H | OMe | H | Cl | H | H |
| 170 | H | H | OMe | Cl | H | H |
| 171 | H | F | $CF_3$ | H | H | H |
| 172 | H | F | H | $CF_3$ | H | H |
| 173 | H | F | H | H | $CF_3$ | H |
| 174 | H | F | H | H | H | $CF_3$ |
| 175 | H | $CF_3$ | F | H | H | H |
| 176 | H | H | F | $CF_3$ | H | H |
| 177 | H | H | F | H | $CF_3$ | H |
| 178 | H | H | F | H | H | $CF_3$ |
| 179 | H | $CF_3$ | H | F | H | H |
| 180 | H | H | $CF_3$ | F | H | H |
| 181 | H | Cl | $CF_3$ | H | H | H |
| 182 | H | Cl | H | $CF_3$ | H | H |
| 183 | H | Cl | H | H | $CF_3$ | H |
| 184 | H | Cl | H | H | H | $CF_3$ |
| 185 | H | $CF_3$ | Cl | H | H | H |
| 186 | H | H | Cl | $CF_3$ | H | H |
| 187 | H | H | Cl | H | $CF_3$ | H |
| 188 | H | H | Cl | H | H | $CF_3$ |
| 189 | H | $CF_3$ | H | Cl | H | H |

[Table 1-6]

| | | | | | | |
|---|---|---|---|---|---|---|
| 190 | H | H | $CF_3$ | Cl | H | H |
| 191 | H | Me | $CF_3$ | H | H | H |
| 192 | H | Me | H | $CF_3$ | H | H |
| 193 | H | Me | H | H | $CF_3$ | H |
| 194 | H | Me | H | H | H | $CF_3$ |
| 195 | H | $CF_3$ | Me | H | H | H |
| 196 | H | H | Me | $CF_3$ | H | H |
| 197 | H | H | Me | H | $CF_3$ | H |
| 198 | H | H | Me | H | H | $CF_3$ |
| 199 | H | $CF_3$ | H | Me | H | H |
| 200 | H | H | $CF_3$ | Me | H | H |
| 201 | H | F | $OCF_3$ | H | H | H |
| 202 | H | F | H | $OCF_3$ | H | H |
| 203 | H | F | H | H | $OCF_3$ | H |
| 204 | H | F | H | H | H | $OCF_3$ |
| 205 | H | $OCF_3$ | F | H | H | H |
| 206 | H | H | F | $OCF_3$ | H | H |
| 207 | H | H | F | H | $OCF_3$ | H |
| 208 | H | H | F | H | H | $OCF_3$ |
| 209 | H | $OCF_3$ | H | F | H | H |
| 210 | H | H | $OCF_3$ | F | H | H |
| 211 | H | Cl | $OCF_3$ | H | H | H |
| 212 | H | Cl | H | $OCF_3$ | H | H |
| 213 | H | Cl | H | H | $OCF_3$ | H |
| 214 | H | Cl | H | H | H | $OCF_3$ |
| 215 | H | $OCF_3$ | Cl | H | H | H |
| 216 | H | H | Cl | $OCF_3$ | H | H |
| 217 | H | H | Cl | H | $OCF_3$ | H |
| 218 | H | H | Cl | H | H | $OCF_3$ |
| 219 | H | $OCF_3$ | H | Cl | H | H |
| 220 | H | H | $OCF_3$ | Cl | H | H |
| 221 | Me | H | H | H | H | H |
| 222 | Me | Me | H | H | H | H |
| 223 | Me | H | Me | H | H | H |
| 224 | Me | H | H | Me | H | H |
| 225 | Me | Et | H | H | H | H |
| 226 | Me | H | Et | H | H | H |
| 227 | Me | H | H | Et | H | H |

[Table 1-7]

| | | | | | | |
|---|---|---|---|---|---|---|
| 228 | Me | *n*Pr | H | H | H | H |
| 229 | Me | H | *n*Pr | H | H | H |
| 230 | Me | H | H | *n*Pr | H | H |
| 231 | Me | *i*Pr | H | H | H | H |
| 232 | Me | H | *i*Pr | H | H | H |
| 233 | Me | H | H | *i*Pr | H | H |
| 234 | Me | *t*Bu | H | H | H | H |
| 235 | Me | H | *t*Bu | H | H | H |
| 236 | Me | H | H | tBu | H | H |
| 237 | Me | OMe | H | H | H | H |
| 238 | Me | H | OMe | H | H | H |
| 239 | Me | H | H | OMe | H | H |
| 240 | Me | OEt | H | H | H | H |
| 241 | Me | H | OEt | H | H | H |
| 242 | Me | H | H | OEt | H | H |
| 243 | Me | O*n*Pr | H | H | H | H |
| 244 | Me | H | O*n*Pr | H | H | H |
| 245 | Me | H | H | O*n*Pr | H | H |
| 246 | Me | O*i*Pr | H | H | H | H |
| 247 | Me | H | O*i*Pr | H | H | H |
| 248 | Me | H | H | O*i*Pr | H | H |
| 249 | Me | O*n*Bu | H | H | H | H |
| 250 | Me | H | O*n*Bu | H | H | H |
| 251 | Me | H | H | O*n*Bu | H | H |
| 252 | Me | O*i*Bu | H | H | H | H |
| 253 | Me | H | O*i*Bu | H | H | H |
| 254 | Me | H | H | O*i*Bu | H | H |
| 255 | Me | O*n*Pen | H | H | H | H |
| 256 | Me | H | O*n*Pen | H | H | H |
| 257 | Me | H | H | O*n*Pen | H | H |
| 258 | Me | F | H | H | H | H |
| 259 | Me | H | F | H | H | H |
| 260 | Me | H | H | F | H | H |
| 261 | Me | Cl | H | H | H | H |
| 262 | Me | H | Cl | H | H | H |
| 263 | Me | H | H | Cl | H | H |
| 264 | Me | Br | H | H | H | H |
| 265 | Me | H | Br | H | H | H |

[Table 1-8]

| | | | | | | |
|---|---|---|---|---|---|---|
| 266 | Me | H | H | Br | H | H |
| 267 | Me | $CHF_2$ | H | H | H | H |
| 268 | Me | H | $CHF_2$ | H | H | H |
| 269 | Me | H | H | $CHF_2$ | H | H |
| 270 | Me | $CF_3$ | H | H | H | H |
| 271 | Me | H | $CF_3$ | H | H | H |
| 272 | Me | H | H | $CF_3$ | H | H |
| 273 | Me | $OCHF_2$ | H | H | H | H |
| 274 | Me | H | $OCHF_2$ | H | H | H |
| 275 | Me | H | H | $OCHF_2$ | H | H |
| 276 | Me | $OCF_3$ | H | H | H | H |
| 277 | Me | H | $OCF_3$ | H | H | H |
| 278 | Me | H | H | $OCF_3$ | H | H |
| 279 | Me | $OCH_2CF_3$ | H | H | H | H |
| 280 | Me | H | $OCH_2CF_3$ | H | H | H |
| 281 | Me | H | H | $OCH_2CF_3$ | H | H |
| 282 | Me | H | H | $OCF_3$ | H | H |
| 283 | Me | SMe | H | H | H | H |
| 284 | Me | H | SMe | H | H | H |
| 285 | Me | H | H | SMe | H | H |
| 286 | Me | SEt | H | H | H | H |
| 287 | Me | H | SEt | H | H | H |
| 288 | Me | H | H | SEt | H | H |
| 289 | Me | SOMe | H | H | H | H |
| 290 | Me | H | SOMe | H | H | H |
| 291 | Me | H | H | SOMe | H | H |
| 292 | Me | $SO_2Me$ | H | H | H | H |
| 293 | Me | H | $SO_2Me$ | H | H | H |
| 294 | Me | H | H | $SO_2Me$ | H | H |
| 295 | Me | $NH_2$ | H | H | H | H |
| 296 | Me | H | $NH_2$ | H | H | H |
| 297 | Me | H | H | $NH_2$ | H | H |
| 298 | Me | NHAc | H | H | H | H |
| 299 | Me | H | NHAc | H | H | H |
| 300 | Me | H | H | NHAc | H | H |
| 301 | Me | $NHCOCF_3$ | H | H | H | H |
| 302 | Me | H | $NHCOCF_3$ | H | H | H |
| 303 | Me | H | H | $NHCOCF_3$ | H | H |

[Table 1-9]

| | | | | | | |
|---|---|---|---|---|---|---|
| 304 | Me | NMeAc | H | H | H | H |
| 305 | Me | H | NMeAc | H | H | H |
| 306 | Me | H | H | NMeAc | H | H |
| 307 | Me | $NMeCOCF_3$ | H | H | H | H |
| 308 | Me | H | $NMeCOCF_3$ | H | H | H |
| 309 | Me | H | H | $NMeCOCF_3$ | H | H |
| 310 | Me | $CH=CH_2$ | H | H | H | H |
| 311 | Me | H | $CH=CH_2$ | H | H | H |
| 312 | Me | H | H | $CH=CH_2$ | H | H |
| 313 | Me | C≡CH | H | H | H | H |
| 314 | Me | H | C≡CH | H | H | H |
| 315 | Me | H | H | C≡CH | H | H |
| 316 | Me | Me | Me | H | H | H |
| 317 | Me | Me | H | Me | H | H |
| 318 | Me | Me | H | H | Me | H |
| 319 | Me | Me | H | H | H | Me |
| 320 | Me | H | Me | Me | H | H |
| 321 | Me | H | Me | H | Me | H |
| 322 | Me | OMe | OMe | H | H | H |
| 323 | Me | OMe | H | OMe | H | H |
| 324 | Me | OMe | H | H | OMe | H |
| 325 | Me | OMe | H | H | H | OMe |
| 326 | Me | H | OMe | OMe | H | H |
| 327 | Me | H | OMe | H | OMe | H |
| 328 | Me | Cl | Cl | H | H | H |
| 329 | Me | Cl | H | Cl | H | H |
| 330 | Me | Cl | H | H | Cl | H |
| 331 | Me | Cl | H | H | H | Cl |
| 332 | Me | H | Cl | Cl | H | H |
| 333 | Me | H | Cl | H | Cl | H |
| 334 | Me | F | F | H | H | H |
| 335 | Me | F | H | F | H | H |
| 336 | Me | F | H | H | F | H |
| 337 | Me | F | H | H | H | F |
| 338 | Me | H | F | F | H | H |
| 339 | Me | H | F | H | F | H |
| 340 | Me | $CF_3$ | $CF_3$ | H | H | H |
| 341 | Me | $CF_3$ | H | $CF_3$ | H | H |

[Table 1-10]

| | | | | | | |
|---|---|---|---|---|---|---|
| 342 | Me | $CF_3$ | H | H | $CF_3$ | H |
| 343 | Me | $CF_3$ | H | H | H | $CF_3$ |
| 344 | Me | H | $CF_3$ | $CF_3$ | H | H |
| 345 | Me | H | $CF_3$ | H | $CF_3$ | H |
| 346 | Me | $OCF_3$ | $OCF_3$ | H | H | H |
| 347 | Me | $OCF_3$ | H | $OCF_3$ | H | H |
| 348 | Me | $OCF_3$ | H | H | $OCF_3$ | H |
| 349 | Me | $OCF_3$ | H | H | H | $OCF_3$ |
| 350 | Me | H | $OCF_3$ | $OCF_3$ | H | H |
| 351 | Me | H | $OCF_3$ | H | $OCF_3$ | H |
| 352 | Me | F | Me | H | H | H |
| 353 | Me | F | H | Me | H | H |
| 354 | Me | F | H | H | Me | H |
| 355 | Me | F | H | H | H | Me |
| 356 | Me | Me | F | H | H | H |
| 357 | Me | H | F | Me | H | H |
| 358 | Me | H | F | H | Me | H |
| 359 | Me | H | F | H | H | Me |
| 360 | Me | Me | H | F | H | H |
| 361 | Me | H | Me | F | H | H |
| 362 | Me | Cl | Me | H | H | H |
| 363 | Me | Cl | H | Me | H | H |
| 364 | Me | Cl | H | H | Me | H |
| 365 | Me | Cl | H | H | H | Me |
| 366 | Me | Me | Cl | H | H | H |
| 367 | Me | H | Cl | Me | H | H |
| 368 | Me | H | Cl | H | Me | H |
| 369 | Me | H | Cl | H | H | Me |
| 370 | Me | Me | H | Cl | H | H |
| 371 | Me | F | OMe | H | H | H |
| 372 | Me | F | H | OMe | H | H |
| 373 | Me | F | H | H | OMe | H |
| 374 | Me | F | H | H | H | OMe |
| 375 | Me | OMe | F | H | H | H |
| 376 | Me | H | F | OMe | H | H |
| 377 | Me | H | F | H | OMe | H |
| 378 | Me | H | F | H | H | OMe |
| 379 | Me | OMe | H | F | H | H |

[Table 1-11]

| | | | | | | |
|---|---|---|---|---|---|---|
| 380 | Me | H | OMe | F | H | H |
| 381 | Me | Cl | OMe | H | H | H |
| 382 | Me | Cl | H | OMe | H | H |
| 383 | Me | Cl | H | H | OMe | H |
| 384 | Me | Cl | H | H | H | OMe |
| 385 | Me | OMe | Cl | H | H | H |
| 386 | Me | H | Cl | OMe | H | H |
| 387 | Me | H | Cl | H | OMe | H |
| 388 | Me | H | Cl | H | H | OMe |
| 389 | Me | OMe | H | Cl | H | H |
| 390 | Me | H | OMe | Cl | H | H |
| 391 | Me | F | $CF_3$ | H | H | H |
| 392 | Me | F | H | $CF_3$ | H | H |
| 393 | Me | F | H | H | $CF_3$ | H |
| 394 | Me | F | H | H | H | $CF_3$ |
| 395 | Me | $CF_3$ | F | H | H | H |
| 396 | Me | H | F | $CF_3$ | H | H |
| 397 | Me | H | F | H | $CF_3$ | H |
| 398 | Me | H | F | H | H | $CF_3$ |
| 399 | Me | $CF_3$ | H | F | H | H |
| 400 | Me | H | $CF_3$ | F | H | H |
| 401 | Me | Cl | $CF_3$ | H | H | H |
| 402 | Me | Cl | H | $CF_3$ | H | H |
| 403 | Me | Cl | H | H | $CF_3$ | H |
| 404 | Me | Cl | H | H | H | $CF_3$ |
| 405 | Me | $CF_3$ | Cl | H | H | H |
| 406 | Me | H | Cl | $CF_3$ | H | H |
| 407 | Me | H | Cl | H | $CF_3$ | H |
| 408 | Me | H | Cl | H | H | $CF_3$ |
| 409 | Me | $CF_3$ | H | Cl | H | H |
| 410 | Me | H | $CF_3$ | Cl | H | H |
| 411 | Me | Me | $CF_3$ | H | H | H |
| 412 | Me | Me | H | $CF_3$ | H | H |
| 413 | Me | Me | H | H | $CF_3$ | H |
| 414 | Me | Me | H | H | H | $CF_3$ |
| 415 | Me | $CF_3$ | Me | H | H | H |
| 416 | Me | H | Me | $CF_3$ | H | H |
| 417 | Me | H | Me | H | $CF_3$ | H |

[Table 1-12]

| | | | | | | |
|---|---|---|---|---|---|---|
| 418 | Me | H | Me | H | H | $CF_3$ |
| 419 | Me | $CF_3$ | H | Me | H | H |
| 420 | Me | H | $CF_3$ | Me | H | H |
| 421 | Me | F | $OCF_3$ | H | H | H |
| 422 | Me | F | H | $OCF_3$ | H | H |
| 423 | Me | F | H | H | $OCF_3$ | H |
| 424 | Me | F | H | H | H | $OCF_3$ |
| 425 | Me | $OCF_3$ | F | H | H | H |
| 426 | Me | H | F | $OCF_3$ | H | H |
| 427 | Me | H | F | H | $OCF_3$ | H |
| 428 | Me | H | F | H | H | $OCF_3$ |
| 429 | Me | $OCF_3$ | H | F | H | H |
| 430 | Me | H | $OCF_3$ | F | H | H |
| 431 | Me | Cl | $OCF_3$ | H | H | H |
| 432 | Me | Cl | H | $OCF_3$ | H | H |
| 433 | Me | Cl | H | H | $OCF_3$ | H |
| 434 | Me | Cl | H | H | H | $OCF_3$ |
| 435 | Me | $OCF_3$ | Cl | H | H | H |
| 436 | Me | H | Cl | $OCF_3$ | H | H |
| 437 | Me | H | Cl | H | $OCF_3$ | H |
| 438 | Me | H | Cl | H | H | $OCF_3$ |
| 439 | Me | $OCF_3$ | H | Cl | H | H |
| 440 | Me | H | $OCF_3$ | Cl | H | H |

[Formula 3]

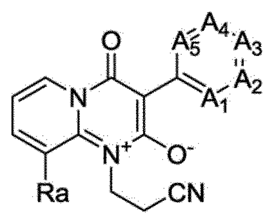

(i i)

[Table 2]

| Compound Number | Ra | $A_1$ | $A_2$ | $A_3$ | $A_4$ | $A_5$ |
|---|---|---|---|---|---|---|
| 441 | H | N | C-H | C-H | C-H | C-H |
| 442 | H | H | N | C-H | C-H | C-H |
| 443 | H | H | C-H | N | C-H | C-H |
| 444 | H | N | $C-CH_3$ | C-H | C-H | C-H |

(continued)

| Compound Number | Ra | $A_1$ | $A_2$ | $A_3$ | $A_4$ | $A_5$ |
|---|---|---|---|---|---|---|
| 445 | H | N | C-H | C-H | $C-CH_3$ | C-H |
| 446 | H | H | N | C-H | $C-CH_3$ | C-H |
| 447 | H | H | $C-CH_3$ | N | C-H | C-H |
| 448 | $CH_3$ | N | C-H | C-H | C-H | C-H |
| 449 | $CH_3$ | H | N | C-H | C-H | C-H |
| 450 | $CH_3$ | H | C-H | N | C-H | C-H |
| 451 | $CH_3$ | N | $C-CH_3$ | C-H | C-H | C-H |
| 452 | $CH_3$ | N | C-H | C-H | $C-CH_3$ | C-H |
| 453 | $CH_3$ | H | N | C-H | $C-CH_3$ | C-H |
| 454 | $CH_3$ | H | $C-CH_3$ | N | C-H | C-H |
| 455 | H | N | $C-CF_3$ | C-H | C-H | C-H |
| 456 | H | N | C-H | C-H | $C-CF_3$ | C-H |
| 457 | H | H | N | C-H | $C-CF_3$ | C-H |
| 458 | H | H | $C-CF_3$ | N | C-H | C-H |
| 459 | $CH_3$ | N | $C-CF_3$ | C-H | C-H | C-H |
| 460 | $CH_3$ | N | C-H | C-H | $C-CF_3$ | C-H |
| 461 | $CH_3$ | H | N | C-H | $C-CF_3$ | C-H |
| 462 | $CH_3$ | H | $C-CF_3$ | N | C-H | C-H |

The compound of formula (1) is a mesoionic inner salt. The "inner salt", i.e. also known as "zwitterion" in the technical field, is an electrically neutral molecule, having positive and negative formal charges on different atoms in each valence bond structure according to the valence bond theory. The molecular structure of the compound of formula (1) may be represented, for example, by the six valence bond structures shown below, though not limited thereto. Each has positive and negative formal charges on different atoms. Due to this resonance, the compounds of formula (1) are described to be "mesoionic". Although the molecular structure of formula (1) is shown herein as a single valence bond structure for simplicity, this particular valence bond structure should be understood as a representative of, for example, all the six valence bond structures shown below, relating to the bonds in the molecule of the compound of formula (1). Any reference to the formula (1) herein, therefore, relates to all the six applicable valence bond structures and other structures, for example, in the molecular orbital theory, if not specified otherwise.

<Six valence bond structures>

[Formula 4]

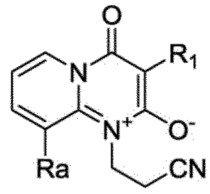
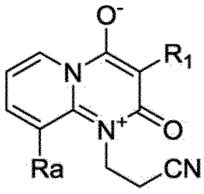
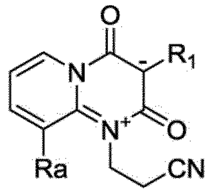
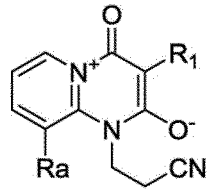
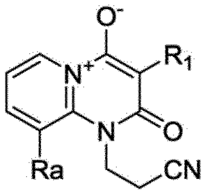
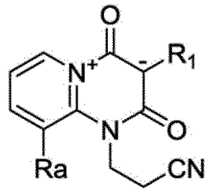

The compounds of the present invention can exist as one or more conformational isomers due to restricted bond rotation caused by steric hindrance. For example, the compounds of formula (1) having phenyl substituted with a bulky alkyl group such as isopropyl at the ortho position as $R_1$ can exist as two rotational isomers due to the restricted rotation relative to an $R_1$-pyrimidinium ring bond. The present invention includes a mixture of conformational isomers.

The compounds of formula (1) may be prepared, for example, by reacting a compound of formula (2) with a formula (3a) having an activated leaving group LvO as shown in a scheme 1, though the manufacturing method of the compound of the present invention is not limited to the preparation method. Examples of the Lv having easiness in synthesis or preferred reactivity include phenyl, 4-nitrophenyl, or halogen-substituted phenyl such as 2,4,6-trichlorophenyl, pentachlorophenyl and pentafluorophenyl. The typical reaction temperature in manufacturing the compounds of the present invention is in the range of 50 to 200°C. These reactions may be performed in a microwave reactor or a reaction vessel, with or without a solvent such as toluene, in the same temperature range. The typical reaction time of the reaction is in the range of 5 minutes to 2 hours.

[Formula 5]

Scheme 1

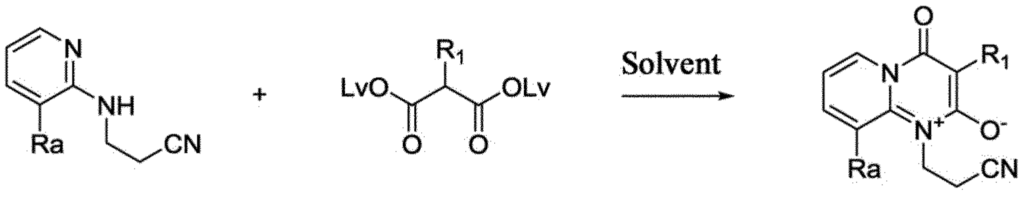


(2) (3a) (1)

The compounds of formula (1) may be also prepared by condensing a compound of formula (2) and a compound of formula (3b) as shown in a scheme 2. These reactions may be typically performed in an inert solvent such as dichloromethane, optionally in the presence of 2 equivalents of acid acceptor. Examples of the typical acid acceptor include triethylamine, N,N-diisopropyl-ethylamine, pyridine and substituted pyridine, and metal hydroxides, carbonates and bicarbonates, though not limited thereto.

[Formula 6]

Scheme 2

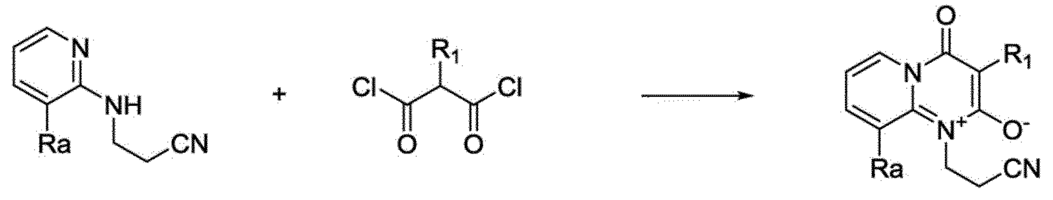

(2) (3b) (1)

Furthermore, the compounds of formula (1) may be prepared from compounds of formula (1a), i.e., the compounds of formula (1) having a hydrogen atom at the site where $R_1$ is introduced, and compounds of formula (4) having Cl, Br or I as $X_1$, preferably Br or I as $X_1$, as shown in a scheme 3.

[Formula 7]

Scheme3

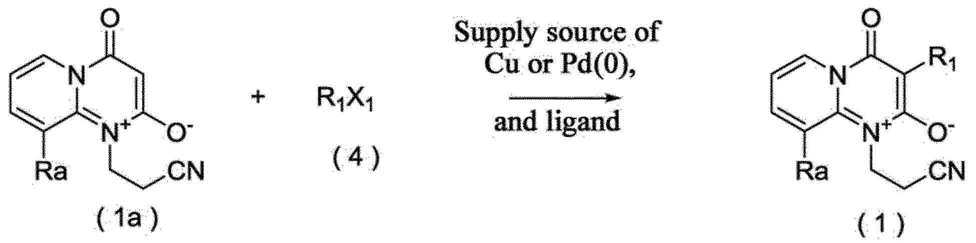


(1a) (4) (1)

These reactions may be performed typically in the presence of a copper or palladium catalyst, preferably under an inert gas atmosphere. The copper catalyst used in the present method may typically include copper in a metallic form such as powder, or monovalent copper, i.e., Cu(I). Examples of the useful copper-containing compound as catalyst in the process of scheme 3 include Cu, CuI, CuBr, and CuCl. Examples of the useful palladium-containing compound as catalyst in the process of scheme 3 include $Pd(OAc)_2$. Examples of the useful solvent for the process of scheme 3 include an ether solvent such as 1,4-dioxane, and a polar solvent such as N,N-dimethylacetamide and dimethylsulfoxide.

The process of scheme 3 can be performed over a wide temperature range of 25 to 200°C. The reaction temperature in the process of scheme 3 is preferably 40 to 150°C, though not limited thereto. The process of scheme 3 can be performed in the presence of a ligand. Various copper-binding compounds are useful as the ligand of the present method. Examples of the useful ligands include 1,10-phenanthroline, N,N-dimethylethylenediamine, L-proline and 2-picolinic acid, though not limited thereto.

The compounds of formula (1) can be also prepared from a compound of formula (1b), and a compound of formula (5) wherein M forms boronic acid, a boronic acid ester or a trifluoroborate, or wherein M is trialkylstannyl or zinc, as shown in Scheme 4.

[Formula 8]

Scheme 4

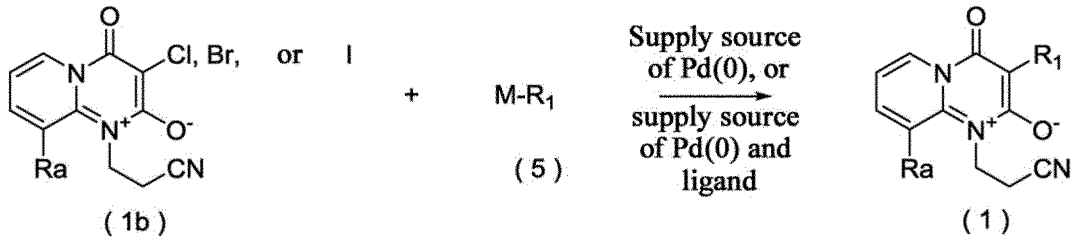


( 1b ) ( 5 ) ( 1 )

The compounds of formula (1b) can be prepared from compounds of formula (1a), for example, by halogenation using liquid bromine or N-halosuccinimide of formula (6), as shown in a scheme 5. The reaction system for performing this reaction is not limited, and the reaction is performed preferably in an inert solvent, more preferably in a halogen solvent such as methylene chloride or 1,2-dichloroethane. The temperature at which the reaction is performed is preferably 0 to 80°C, though not limited thereto.

[Formula 9]

Scheme 5

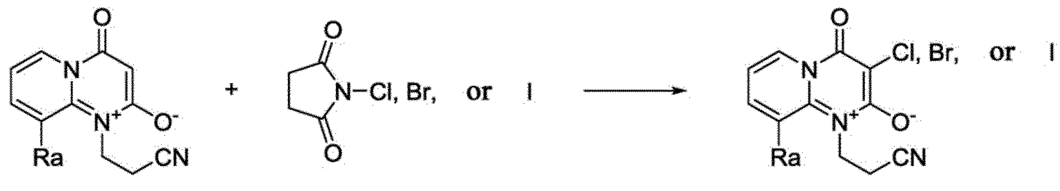

( 1a ) ( 6 ) ( 1b )

The compounds of formula (1) can be also prepared by alkylation of the compounds of formula (7), using the compound of formula (8), wherein X is a leaving group such as halogen group, and a base such as potassium carbonate, as shown in a scheme 6. Examples of the specific compound of formula (8) include 3-chloropropionitrile, 3-bromopropionitrile, and 3-iodopropionitrile.

[Formula 10]

Scheme 6

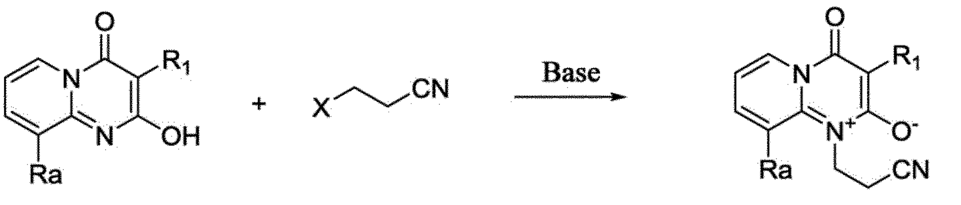


( 7 ) ( 8 ) ( 1 )

The compounds of formula (7) can be prepared by the same processes as shown in the schemes 1 to 4.

In the coupling processes in the schemes 1 to 2, the acid addition salts of the compounds of formula (2) such as a hydrochloride and an acetate can be also used as the compound.

A particularly useful preparation process of the compounds of formula (2) is shown in a scheme 7. In the process of scheme 7, 2-aminopyridine of formula (2a) is protected by a suitable protecting group such as t-butoxycarbonyl, acetyl or formyl, so that an intermediate of formula (2b) having PG as protecting group is formed, though not limited to these protecting groups. Subsequently, the compound of formula (2b) is alkylated by the compound of formula (8), wherein X

is a leaving group such as a halogen atom, so that an intermediate of formula (2c) is produced. The protecting group is removed to produce the compound of formula (2).

[Formula 11]

Scheme 7

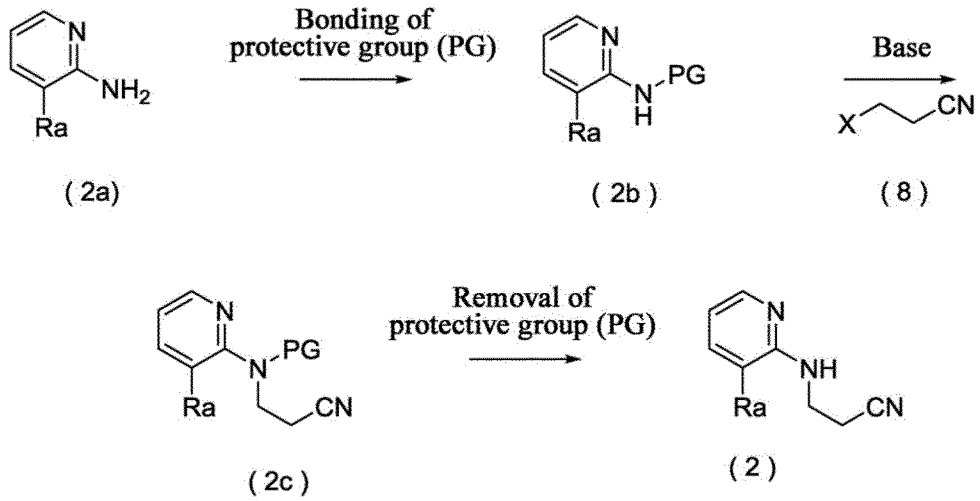


(2a) (2b) (8)

(2c) (2)

Another useful process for preparing compounds of formula (2) is shown in a scheme 8. In the process of scheme 8, pyridine oxide of formula (2d) can be prepared by reacting 3-aminopropanenitrile of formula (9) with a condensing agent such as bromotris(pyrrolidino)phosphonium hexafluorophosphate in the presence of a base such as diisopropylethylamine, though not limited the condensing agent. A positional isomer of formula (2') is also produced when an asymmetric pyridine oxide is used.

[Formula 12]

Scheme 8

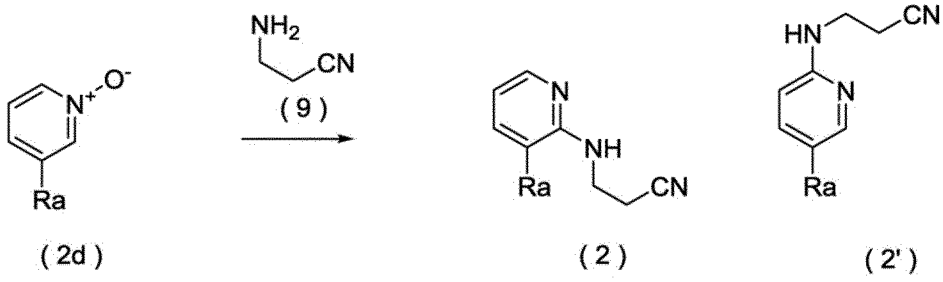

(9)

(2d) (2) (2')

Yet another useful process of the compound of formula (2) is shown in a scheme 9. According to the process of scheme 9, 2-halogenated pyridine of a formula (2e) reacts with 3-aminopropanenitrile of a formula (9). The process of scheme 9 can be performed over a wide temperature range of 25 to 200°C. These reactions may be performed with or without a solvent such as N,N-dimethylacetamide.

[Formula 13]

Scheme 9

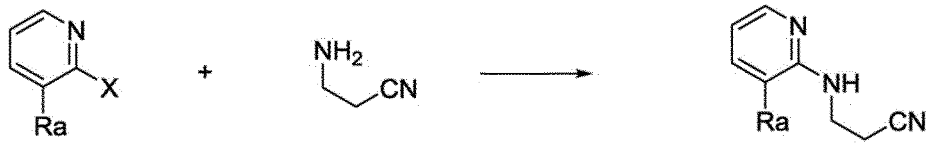

(2e) (9) (2)

Yet another useful process of the compound of formula (2) is shown in a scheme 10. According to the process of scheme 10, 2-halogenated pyridine N-oxide of formula (2f) reacts with 3-aminopropanenitrile of the formula (9) to produce a formula (2g), and N-oxide of the formula (2g) is then reduced. The reaction between the formula (2f) and the formula (9) in the scheme 10 can be performed over a wide temperature range of 0 to 200°C. The reduction reaction of formula (2g) can be performed over a wide temperature range of 0 to 200°C.

[Formula 14]

Scheme 10

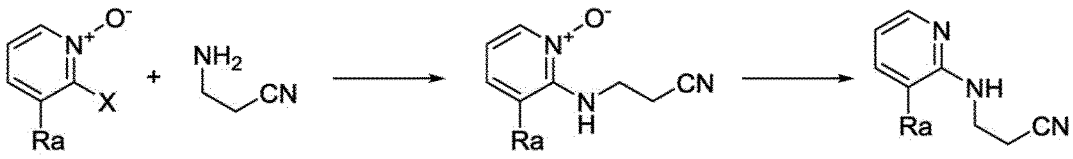

(2f) (9) (2g) (2)

The compound represented by the formula (1) has an excellent controlling activity against agricultural and horticultural harmful insects as shown in the examples below. According to the present invention, therefore, an agricultural and horticultural insecticide comprising a compound represented by the formula (1) or a salt thereof as an active ingredient is provided. Further, the agricultural and horticultural insecticide according to the present invention may contain agriculturally and horticulturally acceptable acid addition salts of these compounds as active ingredients.

Examples of the specific acid addition salt include inorganic acids such as hydrochloride, sulfate, nitrate, and phosphate, carboxylic acid salt such as formate, acetate, oxalate, citrate, succinate, maleate, fumarate, tartrate, lactate, benzoate and phthalate, and sulfonic acid salts such as mesylate, tosylate, and benzenesulfonate.

Insect species to be controlled in the present invention, i.e., insect species on which the compound represented by formula (1) has control effect, is not limited in particular, and a wide range of agricultural and horticultural harmful insects can be controlled thereby. Examples of preferred insect species to be controlled include lepidopteran pests (e.g. Noctuids such as common cutworm, beet armyworm, rice armyworm, cabbage armyworm, black cutworm, Trichoplusia spp, Heliothis spp, and Helicoverpa spp; Pyralids such as rice stem borer, rice leafroller, European corn borer, cabbage webworm, bluegrass webworm, cotton leaf-roller, and Indian meal moth; Pierids such as small cabbage white butterfly; Tortricids such as Adoxophyes spp, oriental fruit moth, and codling moth; snoutworms such as peach fruit moth; leafminers such as Lyonetia spp; tussock moths such as Lymantria spp and Euproctis spp; Yponomeutids such as diamondback moth; gelechiid moths such as pink bollworm; garden tiger moths such as fall webworm; and tineid moths such as clothes moth and webbing clothes moth); hemipteran pests (e.g. aphids such as green peach aphid and cotton aphid; planthoppers such as Laodel phax sriatellus, brown planthopper, and white backed planthoppers; Cicadellids such as Bothrogonia japonica; stink bugs such as Trigonotylus caelestialium, brown winged green stink bug, Nezara viridula, and Riptortus pedestris; whiteflies such as greenhouse whiteflies and silver leaf whiteflies; Coccoids such as comstock mealybug; lace bugs; and Psyllids); coleopteran pests (e.g. weevils such as maize weevil, rice water weevil, and azuki bean weevil; Neatus picipes such as Tenebrio molitor Linnaeus; scarabs such as cupreous chafer and soybeen beetle; leaf beetles such as striped flea beetle, melon beetle, Colorado potato beetle, western corn root worm, and southern corn root worm; Epilachnas such as Inedorooimushi, Paederus fuscipes, and Epilachna vigintioctopunctata; Western bean cutworms and Melanotus okinawensis); Acarian pests (e.g. spider mites such as two-spotted spider mite, Kanzawa spider mite, mandarin orange spider mite, apple spider mite, and Oligonicus spp; Eriophyids such as tomato rust mite, mandarin orange rust mite and tea rust mite; Tarsonemus such as tea plant Tarsonemus; animal parasitic mite such as mold mite,

Chelacaropsis moorei and tick); hymenopteran pests (e.g. sawflies such as Athalia); orthopteran pests (e.g. grasshoppers); dipteran pests (e.g. muscids; culexes; anopheles; midges; blowflies; flesh flies; Fannia canicularis; anthomyiid flies; leafminer flies such as bean leafminer flies, tomato leafminer flies, and eggplant leafminer flies; fruit flies; Phorid flies; Drosophila; moth flies; black flies; Tabanid flies; and Stomoxys calcitrans); thrips pests (e.g. southern yellow thrips, western flower thrips, onion thrips, Thrips hawaiiesis, yellow tea thrips, Frankliniella intonsa, and Ponticulothrips diospyrosi); plant-parasitic nematodes (e.g. root-knot nematode; Pratylenchus spp; cyst nematodes; Aphelenchoides such as white-tip nematode of rice; and pine wood nematode); round worm, and nematodes such as Brugia malayi. Examples of more preferred insect species to be controlled include lepidopteran pests, hemipteran pests, coleopteran pests, hymenopteran pests, dipteran pests, thrips pests, plant-parasitic nematodes. Examples of still more preferred insect species to be controlled include hemipteran pests such as cotton aphid, tobacco whitefly, brown planthopper, and brown winged green stink bug, thrips pests such as southern yellow thrips, lepidopteran pests such as diamondback moth and rice stem borer, and coleopteran pests such as rice water weevil, Anomala rufocuprea, striped flea beetle, western corn root worm, southern corn root worm, Inedorooimushi, and western bean cutworms.

The present invention also provides use of the compound represented by formula (1) of the present invention or the agriculturally and horticulturally acceptable acid addition salts thereof as the active ingredient of agricultural and horticultural insecticides.

In use of the compound represented by formula (1) as an agricultural and horticultural insecticide, the compound represented by formula (1) may be used as it is, or may be mixed with a suitable solid carrier, liquid carrier, gaseous carrier, surfactant, dispersing agent, and other formulation aids or the like so as to prepare an agrochemical formulation. Examples of the preferred agrochemical formulation include an emulsion, an EW formulation, a solution, a suspension, a wettable powder, wettable granules, powders, DL powder, particulates, granules, tablets, an oil solution, an aerosol, a flowable formulation, a dry flowable formulation, and microcapsules. These dosage forms may be optionally selected for use as agrochemical formulation. In the present invention, a carrier refers to a solid carrier, a liquid carrier, or a gaseous carrier or the like.

Examples of the solid carrier include talc, bentonite, clay, kaolin, diatomaceous earth, vermiculite, white carbon, calcium carbonate, acid clay, silica sand, silica stone, zeolite, perlite, attapulgite, pumice, ammonium sulfate, sodium sulfate, and urea.

Examples of the liquid carrier include alcohols such as methanol, ethanol, n-hexanol, ethylene glycol and propylene glycol; ketones such as acetone, methyl ethyl ketone and cyclohexanone; aliphatic hydrocarbons such as n-hexane, kerosene and lamp oil, aromatic hydrocarbons such as toluene, xylene and methylnaphthalene; ethers such as diethyl ether, dioxane and tetrahydrofuran; esters such as ethyl acetate; nitriles such as acetonitrile and isobutyronitrile, acid amides such as dimethylformamide and dimethylacetamide; vegetable oils such as soybean oil and cottonseed oil; dimethyl sulfoxide; and water.

Also, examples of the gaseous carrier include LPG, air, nitrogen, carbon dioxide, and dimethyl ether.

Examples of the surfactant and the dispersant include alkyl sulfates, alkyl(aryl)sulfonates, polyoxyalkylene alkyl(aryl) ethers, polyhydric alcohol esters, lignin sulfonates, alkyl sulfosuccinates, formalin condensates of alkylnaphthalene sulfonates, polycarboxylates, POE polystyrylphenyl ether sulfates and phosphates, and POE/POP block polymers.

Furthermore, examples of the formulation aid include carboxymethyl cellulose, hydroxypropyl cellulose, polyvinyl alcohol, xanthan gum, alpha-starch, gum arabic, polyvinyl pyrrolidone, an ethylene-acrylic acid copolymer, an ethylene-vinyl acetate copolymer, polyethylene glycol, liquid paraffin, calcium stearate, and defoamers and preservatives.

The various carriers, surfactants, dispersants, and formulation aids described above may be used alone or in combination on an as needed basis.

The content of the compound represented by formula (1) as active ingredient in the agrochemical formulation is preferably 1 to 75 wt% in emulsions, 0.3 to 25 wt% in powder, 1 to 90 wt% in wettable powder, and 0.1 to 10 wt% in granules, though not particularly limited thereto.

The agricultural and horticultural insecticides of the present invention may be directly used or diluted for use. Further, the agricultural and horticultural insecticides of the present invention may be mixed with or used in combination with other insecticides, fungicides, miticides, herbicides, plant growth regulators, fertilizers and the like. Examples of the chemicals that can be mixed or used in combination include ones described in Pesticide Manual, 16th edition, published by The British Crop Protection Council, SHIBUYA INDEX, 16th Edition, 2011, published by SHIBUYA INDEX RESEARCH GROUP and SHIBUYA INDEX 17th edition, 2012, published by SHIBUYA INDEX RESEARCH GROUP, and Mode of Action Classification, version 7.3, published by IRAC and Mode of Action of Fungicide, 2015-year version, published by FRAC.

More specifically, examples of the insecticides may include:

carbamate compounds such as alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, metolcarb, fenothiocarb, and fenoxycarb;
organic phosphoric acid ester compounds such as acephate, azamethiphos, azinphos-ethyl, azinphos-methyl, ethylthiometon, chlorethoxyfos, cadusafos, chlorethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, EPN, ethion, etoprophos, famphur, fenamifos, fenitrothion, fenthion, fosthiazate, heptenophos, imicyafos, isofenphos, isopropyl O-(methoxyaminothiophosphoryl)salicylate, isoxathion, malathion, mecarbam, methamidophos, methidathion, mevinphos, monocrotophos, naled, omethoate, dioxydemeton ethyl, parathion, parathion-methyl, PAP, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, chlorpyrifos-ethyl, disulfoton, sulprofos, flupyrazophos, phenthoate, fonofos, and tribufos;
organochlorine compounds such as endosulfan, alpha-endosulfan, gamma-HCH, dicofol, chlordane, dieldrin, and methoxyclor;
phenylpyrazole compounds such as acetoprole, fipronil, ethiprole, pyrafluprole, pyriprole, and flufiprole;
meta-diamide compounds such as broflanilide;
isoxazoline compounds such as afoxolaner, fluralaner, and sarolaner;
pyrethroid compounds such as acrinathrin, allethrin, d-cis-trans allethrin, d-trans allethrin, bifenthrin, kappa-bifenthrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, ethofenprox, fenpropathrin, fenvalerate, flucythrinate, flumethrin, tau-fluvalinate, halfenprox, imiprothrin, kadethrin, permethrin, phenothrin, prallethrin, pyrethrin, resmethrin, silafluofen, tefluthrin, kappa-tefluthrin, phthalthrin, tetramethrin, tralomethrin, transfluthrin, metoxadiazone, metofluthrin, profluthrin, pyrethrum, terallethrin, momfluorothrin, heptafluthrin, meperfluthrin, tetramethylfluthrin, dimefluthrin, and protrifenbut;
neonicotinoid compounds such as acetamiprid, clothianidin, dinotefuran, imidacloprid, nitenpyram, thiacloprid, and thiamethoxam;
sulfoxamine compounds such as sulfoxaflor;
butenolide compounds such as flupyradifurone;
mesoionic compounds such as triflumezopyrim and dicloromezotiaz;
spinosyn compounds such as spinosad and spinetoram;
macrolide compounds such as abamectin, ivermectin, emamectin benzoate, milbemectin, and lepimectin;
juvenile hormone-like compounds such as hydroprene, quinoprene, Diofenolan, and methoprene;
4-phenoxyphenoxy compounds such as pyriproxyfene;
pyridine azomethine compounds such as pymetrozine;
pyridinecarboxamide compounds such as flonicamid;
oxazole compounds such as ethoxazole;
Bacillus thuringiensis and Bacillus sphaericus agents such as B.t. subsp. israelensis, B.t. subsp.aizawai, B.t. subsp. kurstaki, B.t. subsp. tenebrionis and insecticidal proteins they produce;
insecticidal proteins produced by Bt crops corresponding to the above;
thiourea compounds such as diafenthiuron;
organic metal compounds such as azocyclotin, cyhexatin, fenbutatin oxide;
sulfurous acid ester/diphenyl ether compounds such as propargite;
diphenyl sulfone compounds such as tetradifon;
pyrrole compounds such as chlorfenapyr and tralopyril;
dinitro compounds such as DNOC;
nereistoxin analogs such as bensultap, cartap, thiocyclam, thiosultap, and thiosultap sodium;
benzoyl urea compounds such as bistrifluron, chlorfluazuron, diflubenzuron, flucycloxuron, flufenoxuron, hexaflumuron, lufenuron, novaluron, noviflumuron, teflubenzuron, triflumuron, and bistrifluron;
thiadiazine compounds such as buprofezin;
triazole compounds such as cyromazine;
diacyl hydrazine compounds such as chromafenozide, halofenozide, methoxyfenozide, and tebufenozide;
amidine compounds such as amitraz;
amidinohydrazone compounds such as hydramethylnon;
naphthoquinone compounds such as acequinocyl;
strobilurin compounds such as fluacrypyrim, pyriminostrobin, and flufenoxystrobin;
quinazoline compounds such as fenazaquin;
phenoxypyrazole compounds such as fenpyroxymate;

phenoxyethylamine compounds such as pyrimidifen;
pyridazinone compounds such as pyridaben;
pyrazolecarboxamide compounds such as tebufenpyrad, tolfenpyrad, and pyflubumide;
hydrazinecarboxamide compounds such as metaflumizone;
tetronic acid and tetramic acid compounds such as spirodiclofen, spirotetaramat, and spiromesifen;
beta-ketonitrile compounds such as cyflumetofen and cyenopyrafen;
phthalic acid amide compounds such as flubendiamide;
anthranilic acid amide compounds such as chlorantraniliprole, cyantraniliprole, cyclaniliprole, and tetraniliprole;
quinoxaline compounds such as quinomethionate;
thiazolidinone compounds such as hexythiazox;
hydrazine compounds such as bifenazate ;
pyridinamine compounds such as flufenerim;
aminoquinazoline compounds such as pyrifluquinazon;
6-phenoxy quinoline compounds such as flometoquin;
pyridinylethylbenzamide compounds such as fluopyram; and
sulfonamide compounds such as 8-chloro-N-((2-chloro-5-methoxyphenyl)sulfonyl)-6-(trifluoromethyl)imidazo[1,2-a]pyridine-2-carboxamide and amidoflumet.

Further, examples of other insecticides include nicotine, chloropicrin, sulfuryl fluoride, crylotie, clofentezine, diflovidazin, rotenone, indoxacarb, piperonyl butoxide, chlordimeform, pyridalyl, azadirachtin, benzoxymate, afidopyropen, fluhexafon, tioxazafen, fluensulfone, benclothiaz, carzole, insecticidal soap, dimehypo, nithiazine, borate salt, metaaldehyde, ryanodine, sulfluramid, (E)-N-(1-((6-chloropyridin-3-yl)methyl)pyridine-2(1H)-ylidene)-2,2,2-trifluoroacetamide, and 5-(trifluoromethyl)-2-(4-(4-(trifluoromethyl)phenoxy)piperidin-1-yl)pyridine. In addition, agricultural and horticultural insecticides of the present invention can may be mixed or used in combination with microbial pesticides such as insect pathogenic bacteria, insect-pathogenic virus and insect pathogenic fungi.
Examples of the fungicides for use include:

phenylamide compounds such as metalaxyl, metalaxyl-M, oxadixyl, ofurase, benalaxyl, benalaxyl-M, kiralaxyl, ofurace, furalaxyl, and cyprofuram;
hydroxypyrimidine compounds such as bupyrimate, dimethilimol, and ethilimol;
isoxazole compounds such as hymexazole and hydroxyisoxazole;
piperidinylthiazole isoxazoline compounds such as oxathiapiprolin;
isothiazolone compounds such as octhilinone;
carboxylic acid compounds such as oxolinic acid;
benzimidazole thiophanate compounds such as benomyl, thiophanate-methyl, carbendazole, fuberidazole, thiabendazole, and debacarb;
N- phenyl carbamate compounds such as diethofencarb;
toluamide compounds such as zoxamide;
ethylamino thiazolecarboxamide compounds such as ethaboxam;
phenylurea compounds such as pencycuron;
pyridinylmethylbenzamide compounds such as fluopicolide;
pyrimidinamine compounds such as diflumetorim and bupirimate;
pyrazolecarboxamide compounds such as tolfenpyrad;
benzanilide compounds such as benodanil, flutolanil, and mepronil;
phenyloxoethyl thiophene amide compounds such as isofetamid;
pyridinyl ethylbenzamide compounds such as fluopyram;
furancarboxamide compounds such as fenfuram;
oxathiincarboxamide compounds such as oxycarboxin and carboxin;
thiazolecarboxamide compounds such as thifluzamide;
pyrazole-4-carboxamide compounds such as fluxapyroxad, furametpyr, penflufen, penthiopyrad, benzovindiflupyr, bixafen, isopyrazam, and sedaxane;
pyridinecarboxamide compounds such as boscalid;
strobilurin compounds such as azoxystrobin, coumetoxystrobin, kresoxym-methyl, trifloxystrobin, picoxystrobin, pyraclostrobin, dimoxystrobin, metominostrobin, orysastrobin, fluoxastrobin, pyraoxystrobin, pyrametostrobin, flufenoxystrobin, fenaminstrobin, enoxastrobin, coumoxystrobin, mandestrobin, and triclopyricarb;
oxazolidinedione compounds such as famoxadon;
imidazolinone compounds such as fenamidone;
benzylcarbamate compounds such as triclopyricarab and pyribencarb;

cyano imidazole compounds such as cyazofamid;
sulfamoyltriazole compounds such as amisulbrom;
dinitrophenylcroton compounds such as binapacryl, meptyldinocap, dinocap;
2,6-dinitroaniline compounds such as fluazinam;
pyrimidinone hydrazone compounds such as ferimzone;
organic and inorganic metal compounds such as fentin-acetate, fentin chloride, fentin hydroxide, fenthin hydroxide, fenthin acetate and oxine copper;
thiophenecarboxamide compounds such as silthiofam;
triazolopyrimidine amine compounds such as ametoctradin;
anilinopyrimidine compounds such as mepanipyrim, nitrapyrin, pyrimethanil and cyprodinil;
enopiranuron acid antibiotics such as blasticidin-S;
hexopyranosyl antibiotics such as kasugamycin, kasugamycin hydrochloride hydrate;
glucopyranosyl antibiotics such as streptomycin;
tetracycline antibiotics such as oxytetracycline;
allyloxyquinoline compounds such as quinoxyfen;
quinazoline compounds such as proquinazid;
cyanopyrrole compounds such as fludioxonil and fenpiclonil;
dicarboximide compounds such as fluoroimid, procymidone, iprodione, and vinchlozolin;
phosphorothiorate compounds such as edifenphos, iprobenfos and pyrazophos;
dithiolane compounds such as isoprothiolane;
propylcarbamate compounds such as propamocarb and propamocarb hydrochloride;
genus Bacillus such as Bacillus subtilis and produced bactericidal proteins including QST713, FZB24, MBI600, D747 strains and
bactericidal protein compounds produced by the Bt crops described above;
terpene hydrocarbons and terpene alcohols such as extracts of tee tree;
piperazine compounds such as triforine;
pyridine compounds such as pyrifenox and pyrisoxazole;
pyrimidine compounds such as fenarimol and nuarimol;
azole compounds such as azaconazole, bromuconazole, diniconazole, diniconazole-M, epoxyconazole, fluquinconazole, oxpoconazole, pefurazoate, difenoconazole, fenbuconazole, imibenconazole, ipconazole, metconazole, tetraconazole, triadimefon, triadimenol, triticonazole, uniconazole, imazalil, bitertanol, triflumizole, etaconazole, propiconazole, penconazole, flusilazole, flutriafol, myclobutanil, paclobutrazol, prothioconazole, cyproconazole, tebuconazole, hexaconazole, prochloraz, and simeconazole;
morpholine compounds such as aldimorph, dodemorph, dodemorph acetate, tridemorph, fenpropimorph, dimethomorph, flumorph, and pyrimorph;
piperidine compounds such as piperalin and fenpropidin;
spiroketalamine compounds such as spiroxamine;
hydroxyanilide compounds such as fenhexamid;
amino pyrazolinon compounds such as fenpyrazamine;
thiocarbamate/dithiocarbamate compounds such as ferbam, metam, metasulphocarb, metiram, thiram, mancozeb, maneb, zineb, ziram, polycarbamate, propineb, thiuram and pyributicarb;
glucopyranosyl antibiotics such as validamycin;
nucleoside antibiotics such as mildiomycin and polyoxin;
valine amide carbamate-based compounds such as benthiavalicarb, benthiavalicarb-isopropyl, valifenalate, and iprovalicarb;
mandelic acid amide compounds such as mandipropamid;
isobenzofuranon compounds such as fthalide;
pyrroloquinolinon compounds such as pyroquilone;
triazolobenzothiazole compounds such as tricyclazole;
cyclopropanecarboxamide compounds such as carpropamid;
carboxamide compounds such as diclocymet;
propionamide compounds such as fenoxanil;
benzothiadiazole compounds such as acibenzolar-S-methyl;
benzisothiazole compounds such as probenazole;
thiadiazole carboxamide compounds such as tiadinil;
isothiazolecarboxamide compounds such as isotianil;
cyanoacetamide oxime compounds such as cymoxanil;
ethylphosphonate compounds such as fosetyl;

phthalamic acid compounds such as techlophthalam;
benzotriazine compounds such as triazoxide;
benzenesulfonic acid compounds such as flusulfamide;
pyridazinone compounds such as diclomezine;
phenylacetamide compounds such as cyflufenamide;
benzophenone compounds such as metrafenone;
benzoylpyridine compounds such as pyriofenone;
cyanomethylene thiazolidine compounds such as flutianil;
4-quinolyl acetic acid compounds such as tebufloquin;
organic phosphoric acid compounds such as fosetyl-aluminium and tolclofos-methyl;
1,2,4-thiadiazole compounds such as echlomezole;
trifluoroethyl carbamate compounds such as tolprocarb;
copper-based compounds such as Bordeaux mixture, copper acetate, basic copper sulfate, oxy copper chloride, copper hydroxide, and oxine-copper;
inorganic compounds such as copper and sulfur;
N- halogenothioalkyl-based compounds such as captan, captafol, and folpet;
organochlorine compounds such as anilazine, chlorothalonil, dichlorophen, pentachlorophenol and salts thereof, hexachlorobenzene, and quintozene;
guanidine compounds such as iminoctadine triacetate salt, iminoctadine albesilate, guanidine, dodine, dodine free base, guazatine, guazatine acetate salt, and albesilate;
anthraquinone compounds such as dithianon;
quinoxaline compounds such as quinomethionate;
maleimide compounds such as fluoroimide;
sulfenic acid compounds such as tolylfluanid and dichlofluanid;
dinitrophenol compounds such as dinobuton; and
cyclic dithiocarbamate compounds such as dazomet.

Examples of other fungicides include, dipymetitrone, pyraziflumid, picarbutrazox, tecnazen, nitrthal-isopropyl, dicyclomet, acibenzolar, prohexadione-calcium, bronopol, diphenylamine, flumetover, bethoxazin, biphenyl, chloroneb, CNA, iodcarb, prothiocarb, N-(1-(2,4-dichlorophenyl)-1-methoxypropan-2-yl)-2-iodobenzamide bromide, 3-(5-fluoro-3,3,4,4-tetramethyl-3,4-dihydroisoquinolin-1-yl)quinoline, 3-(4,4-difluoro-3,3-dimethyl-3,4-dihydroisoquinolin-1-yl)quinoline, and 3-(4,4,5-trifluoro-3,3-dimethyl-3,4-dihydroisoquinolin-1-yl)quinoline.

The compounds represented by the formula (1) or acid addition salts thereof which are agriculturally and horticulturally acceptable and acceptable as animal drugs of the present invention in an effective amount can be applied to plants, soil or animals for use in controlling target harmful organisms. In other words, a method for controlling harmful organisms in these fields is provided. The controlling method of the present invention may also include a method for applying the compounds represented by formula (1) or agriculturally and horticulturally acceptable acid addition salts thereof by smoking treatment in a sealed space.

The compounds represented by the formula (1) or agriculturally and horticulturally acceptable acid addition salts thereof of the present invention in a biologically effective amount may be processed to or brought into contact with crops, seeds from which crops grow, or crop seats so as to enhance the vitality of crops.

For target seeds to be processed or contacted, the amount of the compound represented by formula (1) or agriculturally and horticulturally acceptable acid addition salts thereof of the present invention is not limited, and the compound represented by formula (1) or agriculturally and horticulturally acceptable acid addition salts thereof of the present invention in an amount of approximately 0.0001 mass% to approximately 1 mass% relative to the whole seeds after treatment is preferred.

The present invention further provides use of the compound represented by formula (1) or an acid addition salt thereof of the present invention as active ingredient of a composition for protecting animals from harmful parasitic invertebrate organisms. The composition comprises the compound represented by the formula (1) or an acid addition salt thereof of the present invention in an amount parasiticidally effective without harmful to subject animals. In the use, the compound represented by the formula (1) or an acid addition salt thereof of the present invention in a biologically effective amount is brought into contact with the harmful invertebrate organisms or the habitat thereof, so that the harmful invertebrate organisms are controlled.

Examples

<Synthesis Examples>

Hereinafter, the present invention is specifically described with reference to examples, though the present invention is not limited thereto. In NMR data, "S" represents a singlet, "d" represents a doublet, "t" represents a triplet, "q" represents a quartet, and "m" represents a multiplet.

Preparation of 2-hydroxy-4-oxo-1-(2-cyanoethyl)-3-(3-toluyl)-4H-pyrido[1,2-a]pyrimidinium inner salt (Compound 3)

Step A: Preparation of N-t-butoxycarbonyl-2-aminopyridine

To di-t- butyl dicarbonate (27.83 g, 127.51 mmol), t-butanol (150 mL) was added, and then 2-aminopyridine was slowly added thereto under stirring. After stirring for 30 minutes at room temperature, t-butanol was distilled off under reduced pressure with an evaporator to obtain white solid. This was purified by silica gel chromatography, so that the title compound (14.63 g, 70.9%, white crystal) was obtained.
$^1$H-NMR (CDCl3) $\delta$ 8.80-9.00 (1H, broad), 8.32 (1H, dd, J = 5.1, 0.9 Hz), 7.98 (1H, d, J = 8.7 Hz), 7.55-7.75 (1H, m), 6.94 (ddd, 1H, J = 7.2, 5.1, 0.9 Hz), 1.55 (s, 9H)

Step B: Preparation of 3-(pyridin-2-ylamino)propanenitrile

The product in the step A was dissolved in dimethyl sulfoxide (60 mL), and sodium hydride (55%, 1.45 g, 33.23 mmol) was added thereto at room temperature. The mixture was stirred for 30 minutes at 60°C. Subsequently, 3-bromopropanenitrile (2.57 mL, 31.46 mmol) was added thereto, and the mixture was stirred for 1 hour at 60°C. The mixture was then cooled to room temperature, and water was added thereto. The mixture was extracted three times with ethyl acetate and washed three times with a saturated saline solution. The extracted ethyl acetate layer was dried over anhydrous magnesium sulfate and filtered. Ethyl acetate was then distilled off under reduced pressure with an evaporator. The resulting crude product was purified by silica gel chromatography, so that t-butyl (2-cyanoethyl) (pyridin-2-yl)carbamate (5.41 g, 73.0%, colorless oily state) was obtained.

To t- butyl-(2-cyanoethyl)-(pyridin-2-yl)carbamate (7.69 g, 31.10 mmol), THF (40 mL) and 6 N hydrochloric acid (40 mL) were added and stirred for 2 hours at room temperature. A sodium hydroxide aqueous solution was added thereto, so that a neutral to alkaline solution was obtained. The reaction solution was extracted twice with ethyl acetate and dried over magnesium sulfate. Ethyl acetate was then distilled off under reduced pressure with an evaporator, so that the title compound (3.41 g, 23.17 mmol, 74.5%) was obtained. $^1$H-NMR (CDCl3) $\delta$ 8.09 (1H, dd, J = 4.8, 0.9 Hz), 7.35-7.47 (1H, m), 6.62 (1H, ddd, J = 6.3, 5.1, 0.9 Hz), 6.44 (1H, d, J = 8.4), 4.70-4.90 (1H, broad), 3.70 (2H, q, J = 6.3 Hz), 2.74 (2H, t, J = 6.3 Hz)

Step C: Preparation of 1,3-diethyl 2-(3-toluyl)propanedioate

To 1,4-dioxane (100 mL), 1,10-phenanthroline (1.00 g, 5.55 mmol) and copper(I) iodide (1.01 g, 5.30 mmol) were added, then cesium carbonate (18.67 g, 57.30 mmol), diethyl malonate (8.08 g, 50.45 mmol) and 3-iodotoluene (10.00 g, 45.86 mmol) were added thereto. After stirring for 15 hours under reflux, the reaction mixture was cooled to room temperature. Aqueous 1 N hydrochloric acid was added to the reaction mixture, which was filtered with a filter aid to remove solids. The filtrate was extracted twice with ethyl acetate, dried over magnesium sulfate, filtered, and the filtrate was distilled off under reduced pressure by an evaporator. The resulting crude product was purified by silica gel chromatography to produce the title compound (7.02 g, 61.2%).
$^1$H-NMR (CDCl3) $\delta$ 7.10-7.30 (4H, m), 4.57 (s, 1H), 4.10-4.30 (4H, m), 2.35 (3H, s), 1.26 (6H, t, J = 6.9 Hz)

Step D: Preparation of bis(2,4,6-trichlorophenyl)-2-(3-toluyl)propanedioate

The product in the step C was added to a mixture of ethanol (35 mL) and 17% sodium hydroxide aqueous solution (132 g), and the reaction mixture was vigorously stirred for 30 minutes at 60°C under nitrogen atmosphere. Subsequently, hydrochloric acid was added to the reaction mixture until the pH reached 1. The reaction solution was extracted 3 times with ethyl acetate, dried over magnesium sulfate and filtered, and ethyl acetate was distilled off under reduced pressure by an evaporator. To the resulting white solid (5.69 g), dichloromethane (120 mL) was added, and oxalyl chloride (4.81 mL, 56.09 mmol) and N,N-dimethylformamide (1.2 mL) were added thereto. The reaction mixture was stirred for 2 hours at room temperature, and 2,4,6-trichlorophenol (11.09 g, 56.17 mmol) was added thereto. After stirring overnight at room temperature, the reaction mixture was concentrated under reduced pressure. Methanol was

added to the resulting residue, so that solids precipitated out slowly from the solution. The precipitated solids were collected by filtration so as to obtain the title compound (9.55 g, 61.6%, white solid).
$^1$H-NMR (CDCl3) δ 7.28-7.47 (7H, m), 7.23 (1H, d, J = 7.8 Hz), 5.27 (1H, s), 2.39 (3H, s)

Step E: Preparation of 2-hydroxy-4-oxo-3-(3-toluyl)-1-(2-cyanoethyl)-4H-pyrido[1,2-a]pyrimidinium inner salt (Compound 3)

Into toluene (10 mL), the product in the step D (0.81 g, 1.47 mmol) and the product in the step B (0.22 g, 1.50 mmol) were added. The reaction mixture was heated at 110°C for 2 hours, so that yellow solids precipitated out from the solution. The reaction mixture was cooled to room temperature and then filtered. The crystals collected by filtration were dried at 60°C by hot air, so that the title product (0.28 g, 62.6%, yellow solid) was obtained. $^1$H-NMR (DMSO-d6) δ 9.31 (1H, dd, J = 6.6, 1.2 Hz), 8.28-8.38 (1H, m), 8.08 (1H, d, J = 9.0 Hz), 7.54 (1H, t, J = 6.9 Hz), 7.48 (1H, s), 7.45 (1H, d, J = 7.8 Hz), 7.19 (1H, t, J = 7.5 Hz), 6.98 (1H, d, J = 7.5 Hz), 4.59 (2H, t, J = 6.9 Hz), 2.94 (2H, t, J = 6.9 Hz), 2.30 (3H, s)
Alternative method for preparing 3-(pyridin-2-ylamino)propanenitrile in Step B:
In N-methyl-2-pyrrolidone (200 mL), 3-amino-propionitrile (54.3 g, 775 mmol), 2-bromopyridine (40.8 g, 258 mmol), potassium carbonate (35.9 g, 260 mmol), and copper iodide (4.95 g, 26.0 mmol) were dissolved and the mixture was stirred at 140°C for 2 hours. After cooling to room temperature, the reaction solution was poured into water and filtered with a filter aid, so that insoluble material was removed. The filtrate was extracted twice with ethyl acetate, washed twice with saturated brine, dried over magnesium sulfate, and then filtered to remove the magnesium sulfate. Ethyl acetate was distilled off under reduced pressure with an evaporator, and the resulting crude product was purified by silica gel chromatography so as to obtain the title compound (9.68 g, 65.77 mmol, 25.5%).

Preparation of 1-(2-cyanoethyl)-2-hydroxy-4-oxo-3-(3-trifluoromethylphenyl)-4H-pyrido[1,2-a]pyrimidinium inner salt (Compound 51)

Step A: Preparation of 1,3-diethyl-2-(3-trifluoromethylphenyl)propanedioate

To 1,4-dioxane (200 mL), 1,10-phenanthroline(2.52 g, 13.98 mmol) and copper(I) iodide (2.53 g, 13.28 mmol) were added, and cesium carbonate (36.16 g, 111.0 mmol), diethyl malonate (17.76 g, 111.0 mmol) and 3-iodobenzotrifluoride (25.12 g, 92.35 mmol) were then added thereto. After stirring under reflux for 7.5 hours, the reaction mixture was cooled to room temperature. To the reaction mixture, 1 N hydrochloric acid was added, and the mixture was filtered with a filter aid to remove solids. The filtrate was extracted twice with ethyl acetate, dried over magnesium sulfate, filtered, and the filtrate was distilled off under reduced pressure with an evaporator. The resulting crude product was purified by silica gel chromatography to obtain the title compound (21.26 g, 75.7%).
$^1$H-NMR (CDCl3) δ 7.72-7.57 (3H, m), 7.49 (t, 1H, J = 7.8 Hz), 4.66 (1H, s), 4.32-4.15 (4H, m), 1.32-1.22 (6H, m)

Step B: Preparation of bis(2,4,6-trichlorophenyl)-2-(3-trifluoromethylphenyl)propanedioate

The product in the step A (18.70 g, 61.46 mmol) was added to 18% sodium hydroxide aqueous solution (219 g), and the reaction mixture was stirred vigorously at 50°C for 1 hour under nitrogen atmosphere. Hydrochloric acid was then added to the reaction solution, until the pH reached 1. The reaction solution was extracted 3 times with ethyl acetate, dried over magnesium sulfate, and filtered, and ethyl acetate was distilled off under reduced pressure with an evaporator. To the resulting white solid (13.18 g), dichloromethane (200 mL) was added, and oxalyl chloride (15.8 mL, 184.2 mmol) and N,N-dimethylformamide (4.0 mL) were then added thereto. The reaction mixture was stirred at room temperature for 3 hours and concentrated under reduced pressure with an evaporator. To the resulting residue, dichloromethane (200 mL) was added, and 2,4,6-trichlorophenol (24.36 g, 123.4 mmol) was added thereto. After stirring overnight at room temperature, the reaction mixture was concentrated under reduced pressure. Methanol was added to the resulting residue, so that a solid precipitated out slowly from the solution. The precipitated solid collected by filtration was dried at 50°C, so that the title compound (12.84 g, 34.4%, white solid) was obtained.
$^1$H-NMR (CDCl3) δ 7.92 (1H, s), 7.85 (1H, d, J = 7.8 Hz), 7.71 (1H, d, J = 7.8 Hz), 7.60 (1H, t, J = 7.8 Hz), 7.38 (4H, s), 5.38 (1H, s)

Step C: Preparation of 2-hydroxy-4-oxo-3-(3-trifluoromethylphenyl)-1-(2-cyanoethyl)-4H-pyrido[1,2-a]pyrimidinium inner salt (Compound 51)

In toluene (210 mL), 3-(pyridin-2-ylamino)propanenitrile (7.00 g, 47.56 mmol) and the product in the step B (28.88 g, 47.58 mmol) were added. The reaction mixture was heated at 110°C for 2 hours, so that yellow solids precipitated out from the solution. The reaction mixture was cooled to room temperature and filtered. The crystals collected by filtration

were dried at 60°C by hot air, so that the title product (41.94 g, 88.2%, yellow solid) was obtained.
$^1$H-NMR (DMSO-d6) δ 9.60 (1H, ddd, J = 6.9, 1.8, 0.6 Hz), 8.28-8.19 (1H, m), 8.15-8.10 (1H, m), 8.05-7.97 (1H, m), 7.75 (1H, d, J = 8.7 Hz), 7.56-7.49 (2H, m), 7.47 (1H, td, J = 7.2, 0.9 Hz), 4.66 (2H, t, J = 6.3 Hz), 3.02 (2H, t, J = 6.6 Hz)

Synthesis Example: Alternative method for preparing bis(2,4,6-trichlorophenyl)-2-(3-trifluoromethylphenyl)propanedioate in the step B for Compound 51

To 1,4-dioxane (200 mL), potassium phosphate (23.50 g, 110.7 mmol), dimethyl malonate (14.70 g, 111.3 mmol), 3- iodobenzotrifluoride (25.10 g, 92.28 mmol), picolinic acid (2.50 g, 20.3 mmol) and copper(I) iodide (2.50 g, 13.1 mmol) were added. After stirring the reaction mixture under reflux for 5 hours, potassium phosphate (23.50 g, 110.7 mmol) was added thereto, and the mixture was further stirred under reflux for 2.5 hours. After cooling of the reaction liquid to room temperature, the reaction mixture was adjusted to pH 3 with hydrochloric acid, and filtered by a filter aid to remove solids. The filtrate was extracted twice with ethyl acetate, washed once each with a saturated sodium hydrogen carbonate aqueous solution and saturated brine, dried over magnesium sulfate, and filtered. The filtrate was distilled off under reduced pressure by an evaporator. A brown oily crude product (27.93 g) was obtained.
The resulting crude product (16.90 g) was added to 11% sodium hydroxide aqueous solution (112 g) and methanol (25 mL), and the reaction mixture was stirred from room temperature to 60°C for 2 hours. Hydrochloric acid was then added to the reaction solution until the pH reached 1. The solution was extracted twice with ethyl acetate, dried over magnesium sulfate, and filtered. Ethyl acetate was distilled off under reduced pressure by an evaporator. To the resulting slightly brown solid (15.70 g), dichloromethane (160 mL) was added, and oxalyl chloride (15.8 mL, 184.2 mmol) and N,N-dimethylformamide (0.8 mL) were then added thereto. After stirring of the reaction mixture at room temperature for 1 hour, 2,4,6-trichlorophenol (24.30 g, 123.1 mmol) was added thereto and further stirred for 1.5 hours. The reaction mixture was concentrated under reduced pressure, and methanol was added to the residue obtained, so that solids precipitated out slowly from the solution. The precipitated solids were collected by filtration and dried at 60°C, so that the title compound (21.50 g, 63.3%, white solid) was obtained.

Synthesis Example: Alternative method for preparing Compound 51

To toluene (7.5 mL), 2-(3-trifluoromethylphenyl)propane diacid (0.72 g, 2.90 mmol) was added, and oxalyl chloride (0.58 mL, 6.76 mmol) and DMF in a catalytic amount were added thereto and stirred at room temperature for 2 hours. The mixture was then cooled in an ice bath, and 3-(pyridin-2-ylamino)propanenitrile (0.43 g, 2.92 mmol) dissolved in toluene (4 mL) was added thereto. Subsequently, triethylamine (0.80 mL, 5.77 mmol) was added thereto, and the mixture was stirred for 20 hours at room temperature. Normal hexane was added to the reaction mixture, and the mixture was stirred for one hour. The precipitated crude crystals were filtered, washed with normal hexane. To the crude crystals obtained, a mixture solution of t-butyl methyl ether and ethyl acetate in a ratio of 7:1 was added and stirred. After filtration, the crystals were washed with t-butyl methyl ether. The crystals obtained were dissolved in ethyl acetate and a small amount of methanol, washed twice with 1 N-hydrochloric acid and once with water, dried over magnesium sulfate, and filtered. The filtrate was concentrated by an evaporator, so that the title compound (0.974 mmol, 33.6%) was obtained.
The compounds of formulas (i) and (ii) described in Tables 3 and 4 which were synthesized by the same method as described above had proton NMR spectral data and melting points as shown in Tables 5 and 6, respectively.

[Formula 15]

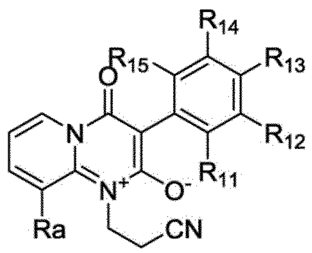

(i)

[Table 3]

| Compound Number | Ra | $R_{11}$ | $R_{12}$ | $R_{13}$ | $R_{14}$ | $R_{15}$ |
|---|---|---|---|---|---|---|
| 1 | H | H | H | H | H | H |
| 3 | H | H | Me | H | H | H |
| 6 | H | H | Et | H | H | H |
| 12 | H | H | *i*Pr | H | H | H |
| 15 | H | H | *t*Bu | H | H | H |
| 18 | H | H | OMe | H | H | H |
| 21 | H | H | OEt | H | H | H |
| 24 | H | H | O*n*Pr | H | H | H |
| 27 | H | H | O*i*Pr | H | H | H |
| 30 | H | H | O*n*Bu | H | H | H |
| 36 | H | H | O*n*Ren | H | H | H |
| 38 | H | F | H | H | H | H |
| 39 | H | H | F | H | H | H |
| 42 | H | H | Cl | H | H | H |
| 45 | H | H | Br | H | H | H |
| 48 | H | H | $CHF_2$ | H | H | H |
| 51 | H | H | $CF_3$ | H | H | H |
| 54 | H | H | $OCHF_2$ | H | H | H |
| 57 | H | H | $OCF_3$ | H | H | H |
| 58 | H | H | H | $OCF_3$ | H | H |
| 60 | H | H | $OCH_2CF_3$ | H | H | H |
| 63 | H | H | SMe | H | H | H |
| 66 | H | H | SEt | H | H | H |
| 75 | H | H | $NH_2$ | H | H | H |
| 78 | H | H | NHAc | H | H | H |
| 84 | H | H | NMeAc | H | H | H |
| 90 | H | H | $CH=CH_2$ | H | H | H |
| 100 | H | H | Me | H | Me | H |
| 103 | H | OMe | H | H | OMe | H |
| 112 | H | H | Cl | H | Cl | H |
| 140 | H | H | Me | F | H | H |
| 168 | H | H | Cl | H | H | OMe |
| 183 | H | Cl | H | H | $CF_3$ | H |
| 200 | H | H | $CF_3$ | Me | H | H |
| 223 | Me | H | Me | H | H | H |
| 241 | Me | H | OEt | H | H | H |
| 271 | Me | H | $CF_3$ | H | H | H |
| 333 | Me | H | Cl | H | Cl | H |

[Formula 16]

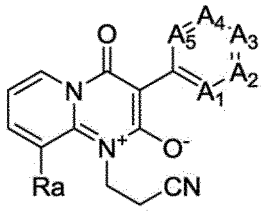

(i i)

[Table 4]

| Compound Number | Ra | A1 | A2 | A3 | A4 | A5 |
|---|---|---|---|---|---|---|
| 367 | H | N | C-H | C-H | C-H | C-H |

[Table 5-1]

| Compound Number | $^1$H-NMR | | Melting point [°C] |
|---|---|---|---|
| | Deuterated solvent | 300MHz, δppm | |
| 1 | DMSO-d6 | 9.32(1H, dd, J=6.9, 1.2Hz), 8. 39-8. 28(1H, m), 8.09(1H, d, J=9.0Hz), 7.73-7.64(2H, m), 7.54(1H, td, J=6.9, 0.9Hz), 7.35-7.26(2H, m), 7.17 (1H, tt, J=8. 1, 1.2Hz), 4.60(2H, t, J=6.9Hz), 2.94(2H, t, J=6.9Hz) | 224~226 |
| 3 | DMSO-d6 | 9.31(1H, dd, J=6.6, 1.2Hz), 8. 38-8. 28 (1H, m), 8.08(1H, d, J=9.0Hz), 7.54(1H, t, J=6.9Hz), 7.48(1H, s), 7.45(1H, d, J=7.8Hz), 7.19(1H, t, J=7.5Hz), 6.98(1H, d, J=7.5Hz), 4.59(2H, t, J=6.9Hz), 2.94(2H, t, J=6.9Hz), 2.30(3H, s) | 185~187 |
| 6 | $CDCl_3$ | 9.58(1H, dd, J=6.9, 1.5Hz), 8.22-8.10 (1H, m), 7.69(1H, d, J=9.0Hz), 7.51(1H, s), 7.53(1H, d, J=7.8Hz), 7.40(1H, td, J=6.9, 1.8Hz), 7.32 (1H, t, J=7.5Hz), 7.10(1H, d, J=7.8Hz), 4.62(2H, t, J=6.3Hz), 2.99 (2H, t, J=6.3Hz), 2.69(2H, q, J=7.5Hz), 1.26(3H, t, J=7.5Hz) | 190~195 |
| 12 | $CDCl_3$ | 9.58(1H, dd, J=6.6, 1.2Hz), 8.20-8.10(1H, m), 7.70(1H, d, J=9.0Hz), 7.61(1H, s), 7.54(1H, d, J=7.8Hz), 7.40(1H, t, J=6.9Hz), 7.33(1H, t, J=7.5Hz), 7.13(1H, d, J=7.8Hz), 4.63(2H, t, J=6.6Hz), 3.04-2.85(3H, m), 1.28(6H, d, J=6.9Hz) | 76~84 |
| 15 | $CDCl_3$ | 9.58(1H, dd, J=6.9, 1.5Hz), 8.19-8.09(1H, m), 7.79(1H, t, J=1.8Hz), 7.69(1H, d, J=8.7Hz), 7.53(1H, dt, J=7.2, 1.5Hz), 7.39(1H, td, J=6.9, 0.9Hz), 7.37-7.26 (2H, m), 4.63 (2H, t, J=6.6Hz), 2.98(2H, t, J=6.6Hz), 1.35(9H, s) | 90~102 |
| 18 | DMSO-d6 | 9.32(1H, d, J=5.7Hz), 8. 38-8. 28 (1H, m), 8.08(1H, d, J=9.0Hz), 7.54 (1H, t, J=7.5Hz), 7. 34-7. 26 (2H, m), 7.21(1H, t, J=8.1Hz), 6.79-6.72 (1H, m), 4.59(2H, t, J=6.9Hz), 3.74(3H, s), 2.94(2H, t, J=6.9Hz) | 186~189 |
| 21 | $CDCl_3$ | 9.54(1H, d, J=6.6Hz), 8.18-8.06(1H, m), 7.66(1H, d, J=8.7Hz), 7.37 (1H, t, J=6.9Hz), 7.35-7.22(3H, m), 6.78(1H, dt, J=7.5, 1.8Hz), 4.59 (2H, t, J=6.6Hz), 4.05(2H, q, J=7.2Hz), 2.95(2H, t, J=6.6Hz), 1.40 (3H, t, J=7.2Hz) | 166~170 |

[Table 5-2]

| | | | |
|---|---|---|---|
| 24 | $CDCl_3$ | 9.54(1H, dd, J=6.9, 1.2Hz), 8. 20-8.06 (1H, m), 7.66(1H, d, J=9.0Hz), 7.37(1H, td, J=6.9, 0.9Hz), 7.23-7. 34 (3H, m), 6.79(1H, dt, J=7.2, 2.1Hz), 4.59(2H, t, J=6.6Hz), 3.94(2H, t, J=6.6Hz), 2.95(2H, t, J=6.6Hz), 1.74-1.90(2H, m), 1.02(3H, t, J=7.2Hz) | 140~143 |
| 27 | $CDCl_3$ | 9.57(1H, dd, J=6.9, 1.2Hz), 8.20-8.11(1H, m), 7.68(1H, d, J=9.0Hz), 7.40(1H, td, J=6.9, 0.9Hz), 7.24-7.35(3H, m), 6.79(1H, dt, J=7.2, 2.1Hz), 4.70-4.50(3H, m), 2.98(2H, t, J=6.6Hz), 1.34(6H, d, J=6.0Hz) | 75~83 |
| 30 | $CDCl_3$ | 9.52(1H, dd, J=6.9, 1.5Hz), 8. 13-8. 03 (1H, m), 7.64(1H, d, J=9.0Hz), 7.35(1H, td, J=6.9, 0.6Hz), 7.32-7. 22 (3H, m), 6.77(1H, dt, J=7.5, 2.1Hz), 4.58(2H, t, J=6.6Hz), 3.97(2H, t, J=6.6Hz), 2.93(2H, t, J=6.6Hz), 1.82-1.69(2H, m), 1.56-1.40 (2H, m), 0.96(3H, t, J=7.5Hz) | 74~87 |
| 36 | $CDCl_3$ | 9.55(1H, dd, J=6.9, 1.2Hz), 8.17-8.07(1H, m), 7.67(1H, d, J=9.0Hz), 7.38(1H, td, J=6.9, 0.9Hz), 7.34-7.24(3H, m), 6.79(1H, dt, J=6.9, 2.4Hz), 4.60(2H, t, J=6.6Hz), 3.97(2H, t, J=6.6Hz), 2.96(2H, t, J=6.6Hz), 1.85-1.71(2H, m), 1.51-1.30(4H, m), 0.92(3H, t, J=6.9Hz) | 60~62 |
| 38 | DMSO-d6 | 9.28(1H, dd, J=6.9, 1.5Hz), 8.43-8.31(1H, m), 8.11(1H, d, J=9.0Hz), 7.55(1H, t, J=6.9Hz), 7.42(1H, td, J=8.1, 2.1Hz), 7.36-7.25(1H, m), 7.09-7.21(2H, m), 4.58 (2H, t, J=6.9Hz), 2.93(2H, t, J=6.9Hz) | 264~266 |
| 39 | DMSO-d6 | 9.33(1H, dd, J=5.7, 1.2Hz), 8.41-8.31(1H, m), 8.10(1H, d, J=9.0Hz), 7.65(1H, d, J=8.1Hz), 7.51-7.63 (2H, m), 7.34(1H, q, J=8.1Hz), 6.98(1H, td, J=8.1, 1.8Hz), 4.60(2H, t, J=6.9Hz), 2.94(2H, t, J=6.9Hz) | 224~225 |
| 42 | $CDCl_3$ | 9.56(1H, dd, J=6.9, 1.5Hz), 8.24-8.14(1H, m), 7.79(1H, t, J=2.1Hz), 7.74-7.64 (2H, m), 7.43(1H, t, 7.2Hz), 7.32(1H, t, J=7.8Hz), 7.25-7.18(1H, m), 4.62(2H, t, J=6.6Hz), 2.99(2H, t, 6.6Hz) | 219~222 |
| 45 | $CDCl_3$ | 9.56(1H, dd, J=6.9, 1.2Hz), 8.23-8.13(1H, m), 7.94(1H, t, J=1.5Hz), 7.75-7.66 (2H, m), 7.42(1H, td, J=6.9, 0.9Hz), 7.37(1H, m), 7.25(1H, t, J=7.8Hz), 4.61(2H, t, 6.6Hz), 2.98(2H, t, 6.6Hz) | 190~193 |

[Table 5-3]

| | | | |
|---|---|---|---|
| 48 | $CDCl_3$ | 9.57 (1H, dd, J=6. 9, 1.2Hz), 8.15-8.22 (1H, m), 7.85-7.96(2H, m), 7.71(1H, d, 8.7Hz), 7. 37-7. 53 (3H, m), 6.68(1H, t, J=56.7Hz), 4.63(2H, t, J=6.6Hz), 2.99(2H, t, 6.6Hz) | 175~ 178 |
| 51 | $CDCl_3$ | 9.60(1H, ddd, J=6.9, 1.8, 0.6Hz), 8.28-8.19(1H, m), 8.15-8.10(1H, m), 8.05-7.97(1H, m), 7.75(1H, d, J=8.7Hz), 7.56-7.49(2H, m), 7.47(1H, td, J=7.2, 0.9Hz), 4.66(2H, t, J=6.3Hz), 3.02(2H, t, J=6.6Hz) | 197~ 199 |
| 54 | DMSO-d6 | 9.33(1H, dd, J=6.9, 1.5Hz), 8.40-8.31(1H, m), 8.10(1H, d, J=9.0), 7.67(1H, dt, J=8.1, 1.5Hz), 7.60(1H, t, J=1.8Hz), 7.55(1H, td, J= 6.9, 0.6Hz), 7.35(1H, t, J=8. 1Hz), 7.17 (1H, t, J=74. 4Hz), 6.98(1H, dd, J=8.1, 2.4Hz), 4.60(2H, t, J=6.9Hz), 2.94(2H, t, J=6.9Hz) | 190~ 194 |
| 57 | $CDCl_3$ | 9.58(1H, dd, J=6.9, 1.5Hz), 8.25-8.16(1H, m), 7.81-7.68(3H, m), 7.43(1H, td, J=6.9, 0.9Hz), 7.40(1H, t, J=8.1Hz), 7.10(1H, dt, J=8.1, 1.8Hz), 4. 63 (2H, t, J=6.6Hz), 3.00 (2H, t, J=6.3Hz) | 157~ 160 |
| 58 | $CDCl_3$ | 9.57(1H, dd, J=6.9, 1.5Hz), 8.24-8.15(1H, m), 7.85-7.78(2H, m), 7.72(1H, d, J=9.0Hz), 7.44(1H, td, J=6.9, 0.6Hz), 7.26-7.18(2H, m), 4.63(2H, t, J=6.6Hz), 3.00(2H, t, J=6.3Hz) | 234~ 236 |
| 60 | $CDCl_3$ | 9.57(1H, dd, J=6.9, 1.2Hz), 8. 23-8.15 (1H, m), 7.71(1H, d, J=9.00), 7.50-7.39(3H, m), 7.34(1H, t, J=7.8Hz), 6.86(1H, ddd, J=8.10, 2.7, 0.9Hz), 4.62(2H, t, J=6.6Hz), 4.38(q, 2H, 8.1Hz), 2.99(2H, t, J=6.3Hz) | 137~ 140 |

(continued)

| | | | |
|---|---|---|---|
| 63 | $CDCl_3$ | 9.58(1H, dd, J=6.9, 1.2Hz), 8. 23-8.13 (1H, m), 7.67-7.40(2H, m), 7.54(1H, dt, J=7.8, 1.2Hz), 7.42(1H, td, J=7.2, 0.9Hz), 7.32(1H, t, J=6.9Hz), 7.15-7.22(1H, m), 4.63(2H, t, J=6.6Hz), 3.00(2H, t, J=6.6Hz), 2.50(3H, s) | 162~ 164 |
| 66 | $CDCl_3$ | 9.52(1H, dd, J=6.9, 1.5Hz), 8.16-8.06(1H, m), 7.74(1H, t, J=1.5Hz), 7.66(1H, d, J=9. 0Hz), 7.57(1H, dt, J=7.8, 1.5Hz), 7.37(1H, td, J=6.9, 0.6Hz), 7.29(1H, t, J=7.8Hz), 7.20(1H, dt, J=8.1, 1.5Hz), 4.59(2H, t, J=6.6Hz), 3. 00-2. 89 (4H, m), 1.32(3H, t, J=7.5Hz) | 57~5 9 |

[Table 5-4]

| | | | |
|---|---|---|---|
| 75 | $CD_3OD$ | 9.42(1H, d, J=6. 6Hz), 8.33(1H, t, 7.8Hz), 8.04(1H, d, J=9.0Hz), 7.55(1H, t, J=7.8Hz), 7.12(1H, t, J=7.8Hz), 7.01-6.90(1H, m), 6.65(1H, d, J=7.5Hz), 4.69(2H, t, J=6.9Hz), 2.98 (2H, t, J=6.9Hz) | 194~ 198 |
| 78 | $CO(CD_3)_2$ | 9.51(1H, ddd, J=6.9, 1.5, 0.6Hz), 8.99(1H, t, 1.8Hz), 8. 52-8. 44 (2H, m), 8,23(1H, d, J=9.3Hz), 8.04(1H, ddd, J=7.2, 2.4, 0.9Hz), 7.66(1H, td, J-6.9, 1.2Hz), 7.60(1H, t, J=8.1Hz), 4.82(2H, t, J=7.2Hz), 3.08(2H, t, J=7.2Hz), 2.77(3H, s) | 245~ 247 |
| 84 | $CDCl_3$ | 9.58(1H, dd, J=6.6, 1.2Hz), 8.26-8.17(1H, m), 7.79(1H, d, J=8.1Hz), 7.74(1H, d, J=9.0Hz), 7.66(1H, t, J=1.8Hz), 7.38-7.49(2H, m), 7.10-7.03(1H, m), 4.64(2H, t, J=6.6Hz), 3.30(3H, s), 3.01(2H, t, J=6.6Hz), 1.98(3H, s) | 229~ 230 |
| 90 | $CDCl_3$ | 9.56(1H, dd, J=6.9, 1.5Hz), 8.19-8.10 (1H, m), 7.78(1H, s), 7.68(1H, d, J=9.0Hz), 7.63 (1H, dt, J=7.2, 1.5Hz), 7. 44-7. 28 (3H, m), 6.75(1H, dd, J=17.7, 10.8Hz), 5.74(1H, dd, J=17.7, 0.9Hz), 5.20(1H, dd, J=10.8, 0.9Hz), 4.61(2H, t, J=6.6Hz), 2.98(2H, t, J=6.6Hz) | 169~ 169 |
| 100 | DMSO-d6 | 9.30(1H, dd, J=6.9, 1.5Hz), 8.38-8.29(1H, m), 8.08(1H, d, J=9.0Hz), 7.54(1H, td, J=6.9, 0.9Hz), 7.25(2H, s), 6.81(1H, s), 4.58(2H, t, J=6.9Hz), 2.94(2H, t, J=6.9Hz), 2.26(6H, s) | 239~ 240 |
| 103 | DMSO-d6 | 9.26(1H, dd, J=6.9, 1.2Hz), 8.39-8. 30(1H, m), 8.08(1H, d J=9.0Hz), 7.53(1H, t, J-¯6.6Hz), 6.92(1H, d, 8. 7Hz), 6.82(1H, dd, J=8.7, 3.0Hz), 6.77(1H, d, J=3.0Hz), 4.57(2H, t, 7.2Hz), 3.69(3H, s), 3.63(3H, s), 2.92(2H, t, J=6.9Hz) | 110~ 130 |
| 112 | DMSO-d6 | 9.32(1H, dd, J=6.9, 1.5Hz), 8. 43-8. 33 (1H, m), 8.11(1H, d, J=8.7Hz), 7.92(2H, d, J=1.8Hz), 7.58(1H, td, J=6.9, 0.9Hz), 7.36(1H, t, J=2.1Hz), 4.60(2H, t, J=6.9Hz), 2.95 (2H, t, 6.9Hz) | 278~ 281 |
| 140 | $CDCl_3$ | 9.32(1H, dd, J=6.9, 1.2Hz), 8.23-8.14 (1H, m), 7.71(1H, d, J=9.0Hz), 7.60-7.47(2H, m), 7.42(1H, td, J=7.2, 1.2Hz), 7.03(1H, t, J=9.0Hz), 4.62(2H, t, J=6.6Hz), 2.99(2H, t, 6.6Hz), 2.30(3H, s, J=1.8Hz) | 231~ 234 |

[Table 5-5]

| | | | |
|---|---|---|---|
| 168 | DMSO-d6 | 9.25(1H, d, J=6.3Hz), 8.35(1H, t, J=7.8Hz), 8.10(1H, d, J=9.0Hz), 7.54(1H, t, J=6.9Hz), 7.29(1H, dd, J=9.0, 2.7Hz), 7.20(1H, d, J=2.4Hz), 7.03(1H, d, J=9.0Hz), 4.57(2H, t, J=5.7Hz), 3.68(3H, s), 2.93(2H, t, J=6.6Hz) | 249~253 |
| 183 | $CDCl_3$ | 9.53(1H, dd, J=6.6, 1.5Hz), 8.27-8.17 (1H, m), 7. 79-7. 72 (2H, m), 7.59(1H, d, J=8.4Hz), 7.51(1H, dd, J=8.7, 2.1Hz), 7.45(1H, t, J=6.6Hz) 4.63(2H, t, J=6.6Hz), 2.98(2H, t, J=6.6Hz) | 113~123 |
| 200 | $CDCl_3$ | 9.58(1H, dd, J=6.9, 1.8Hz), 8.23-8.14(1H, m), 8.06(1H, d, J=1.2Hz), 7.84(1H, dd, J=8.1, 1.2Hz), 7.72(1H, t, J=8.7Hz), 7.43(1H, td, J=6.9, 0.9Hz), 7.30(1H, t, J=7.8Hz), 4.63(2H, t, J=6.6Hz), 2.99(2H, t, J=6.6Hz), 2.49(3H, d, J=1.5Hz) | 209~214 |
| 223 | $CDCl_3$ | 9.52(1H, dd, J=6.6, 1.2Hz), 7.97(1H, dd, J=7.2, 0.9Hz), 7.54(1H, s), 7.51(1H, d, J=7.8Hz), 7.35(1H, t, J=7.2Hz), 7.29(1H, t, J=7.5Hz), 7.06(1H, d, J=7.5Hz), 4.66(2H, t, J=6.6Hz), 3.02(2H, t, J=6.6Hz), 2.81(3H, s), 2.38(3H, s) | 141~143 |

(continued)

| | | | |
|---|---|---|---|
| 241 | $CDCl_3$ | 9.52(1H, dd, J=6.9, 1.2Hz), 7.97(1H, dd, J=7.2, 0.9Hz), 7.39-7.26(4H, m), 6.83-6.77(1H, m), 4.66(2H, t, J=6.9Hz), 4.07(2H, q, J=6.9Hz), 3.01(2H, t, J=6. 9Hz), 2.81(3H, s), 1.41(3H, t, J=6.9Hz) | 125~128 |
| 271 | $CDCl_3$ | 9.50(1H, dd, J=6.9, 1.2Hz), 8.11(1H, s), 8.04-7.96(2H, m), 7.45-7.54(2H, m), 7.37(1H, t, J=7.2Hz), 4.66(2H, t, J=6.9Hz), 3.00(2H, t, J=6.9Hz), 2.81(3H, s) | 109~112 |
| 333 | $CDCl_3$ | 9.46(1H, dd, J=6.9, 1.5Hz), 8.00(1H, dd, J=7.5, 0.9Hz), 7.76(2H, d, J=2.1), 7.38 (1H, t, J=7.2Hz), 7.20(1H, t, J=1.8Hz), 4.63(2H, t, J=6.9Hz), 2.99(2H, t, J=6.9Hz), 2.80(3H, s) | 185~189 |

[Table 6]

| Compound Number | $^1$H-NMR | | Melting point [°C] |
|---|---|---|---|
| | Deuterated solvent | 300MHz, $\delta$ppm | |
| 441 | $CO(CD_3)_2$ | 9.39(1H, dd, J=6.9, 1. 5Hz), 8. 56-8. 62 (1H, m), 8.45-8.35(1H, m), 8.16 (1H, d, J=9.0Hz), 7.70(1H, td, J=7.8, 1.8Hz), 7. 62-7. 52 (2H, m), 7.20-7.11(1H, m), 4.74(2H, t, J=7.2), 3.04(2H, t, J=7.2Hz) | 204~206 |

<Formulation Example>

Furthermore, formulation examples of the agricultural chemicals containing the compound represented by the formula (1) of the present invention as active ingredient are described as follows.

Formulation Example 1 [wettable powder]

Compound 1: 30 wt%
Clay: 30 wt%
Diatomaceous earth: 35 wt%
SAN X P252 (product name of calcium lignosulfonate, manufactured by Nippon Paper Chemicals Co., Ltd.): 4 wt%
SORPOL 8070 (product name of sodium laurylsulfate, manufactured by Toho Chemical Industry Co., Ltd.): 1 wt%

The components described above were uniformly mixed and pulverized to obtain a wettable powder.

Formulation Example 2 [wettable powder 2]

Compound 1: 2 wt%
Kaolin HA: 51 wt%
CARPLEX #80 (product name of white carbon, manufactured by DSL Japan Co., Ltd.): 35 wt%
DEMOL T (product name of sodium $\beta$-naphthalene sulfonate formaldehyde condensate, manufactured by Kao Corporation): 5 wt%
NEWKALGEN NX-405H (product name of POE alkyl phenyl ether sulfoacetate sodium, manufactured by Takemoto Oil and Fat Co., Ltd.): 7 wt%

The components described above were uniformly mixed and pulverized to obtain a wettable powder.

Formulation Example 3 [powder]

Compound 1: 2 wt%
Clay: 90 wt%
Talc: 7 wt%
Calcium stearate: 1 wt%

The components described above were uniformly mixed to obtain a powder

Formulation Example 4 [emulsion]

Compound 1: 20 wt%
N,N-dimethyl formamide: 20 wt%
SOLVESSO 150 (product name of aromatic solvent manufactured by Exxon Mobil Corporation): 50 wt%
NEWKALGEN CL-H (product name of POE alkyl phenyl ether, manufactured by Takemoto Oil and Fat Co., Ltd.): 10 wt%

The components described above were uniformly mixed and dissolved to obtain an emulsion.

Formulation Example 5 [emulsion 2]

Compound 1: 5 wt%
Xylene: 42.5 wt%
DMSO: 42.5 wt%
NEWKALGEN 2003 (product name of the mixture of POE allyl phenyl ether formaldehyde condensate and metal alkyl benzene sulfonate manufactured by Takemoto Oil and Fat Co., Ltd.): 10 wt%

The components described above were uniformly mixed and dissolved to obtain an emulsion.

Formulation Example 6 [Flowable formulation]

Compound 1: 25 wt%
SORPOL 7556 (product name of POE styrylphenyl ether sulfate, manufactured by Toho Chemical Industry Co., Ltd.): 5 wt%
Propylene glycol: 6 wt%
Bentonite: 1 wt%
1% xanthan gum aqueous solution: 3 wt%
Water: 60 wt%

The whole amount of formulation other than 1% xanthan gum aqueous solution and a suitable amount of water was premixed and then pulverized by a wet pulverizer. Subsequently, the 1% xanthan gum aqueous solution and the remaining water were added to the pulverized product thus obtained, so that 100 wt% flowable formulation was produced.

Formulation Example 7 [Particulates]

Compound 1: 5 wt%
Bentonite: 40 wt%
Talc: 10 wt%
Clay: 43 wt%
SAN X P252 (product name of calcium lignosulfonate, manufactured by Nippon Paper Chemicals Co., Ltd.): 2 wt%

The components described above were uniformly pulverized and mixed. Water was added to the mixture, which

was then well kneaded, granulated and dried, so that granules were produced.

<Biological Test Examples>

The following tests demonstrate the control effect of the compounds of the present invention against a certain broad range of harmful organisms. "Control effect" represents the growth inhibition in harmful invertebrate organisms to significantly reduce eating including the mortality. The harmful organisms as target to be controlled by the compounds, however, are not limited to these species. The numerical numbers for identifying the compounds of the present invention refer to the number of compounds in Tables 1 and 2.

<Biological Test Examples>

Biological Test Example 1: Test (foliar spraying) for controlling cotton aphid (Aphis gossypii)

A cucumber leaf cut to a diameter of 3.5 cm was placed on cotton wool moistened with water. Two adult cotton aphids were released there for offspring for 24 hours and the adult insects were then removed. The diluent of the test compounds diluted to 200 ppm in an amount of 2 mL was sprayed onto the cucumber leaves with a spray tower. After air-drying, the sample with cotton wool was placed in a plastic cup, which was covered with a lid and kept in a chamber at a constant temperature of 25°C for rearing. On 5 days after the treatment, the life or death was observed to calculate the mortality.

As a result, the compounds 1, 3, 6, 12, 15, 18, 21, 24, 27, 30, 36, 38, 39, 42, 45, 48, 51, 54, 57, 58, 60, 63, 66, 78, 84, 90, 100, 103, 140, 168, 183, 200, 223 and 241 exhibited a mortality of 80% or more.

Biological Test Example 2: Test (foliar spraying) for controlling sweetpotato whitefly (Bemisia tabaci)

A cucumber leaf cut to a diameter of 6.0 cm was placed on cotton wool moistened with water. The diluent of the test compounds diluted to 200 ppm in an amount of 2 mL was sprayed onto the cucumber leaves with a spray tower. After air-drying, the cucumber leaf was placed in a plastic cup, and 20 female adult sweetpotato whiteflies were released there. The cup was covered with a lid and kept in a chamber at a constant temperature of 25°C for rearing. On 5 days after the treatment, the life or death was observed to calculate the mortality.

As a result, Compounds 3 and 48 exhibited a mortality of 80% or more.

Biological Test Example 3: Test (foliar dipping) for controlling diamondback moth (Plutella xylostella)

A cabbage leaf cut to a diameter of 5.0 cm was dipped in the diluent of the test compounds diluted to 200 ppm in an amount of 20 mL and air-dried. After air-drying, the cabbage leaf was placed in a plastic cup, and 10 larvae of diamondback moths in the third instar larva were released there. The cup was covered with a lid and kept in a chamber at a constant temperature of 25°C for rearing. On 3 days after the treatment, the life or death of the larvae was observed to calculate the mortality.

As a result, Compounds 3, 42, 48, 51 and 57 exhibited a mortality of 80% or more.

Biological Test Example 4: Test (foliar dipping) for controlling brown planthopper (Nilaparvata lugens)

Ten sampled rice seedlings were dipped in in the chemical solution of the test compounds diluted to 200 ppm in an amount of 20 mL and air-dried. After air-drying, the rice seedlings held with urethane in a glass cylinder having an inner diameter of 4.5 cm and a length of 14 cm were set up in a plastic cup containing 40 mL of water. Larvae of brown planthoppers in the third instar larva were released there. The cylinder was covered with a lid of medicine paper and kept in a chamber at a constant temperature of 25°C for rearing. On 5 days after the treatment, the life or death of the larvae was observed to calculate the mortality.

As a result, Compounds 1, 3, 6, 12, 15, 18, 21, 24, 27, 30, 36, 38, 39, 42, 45, 48, 51, 57, 60, 63, 66, 78, 84, 90, 100, 103, 140, 168, 183, 200, 223 and 241 exhibited a mortality of 80% or more.

Biological Test Example 5: Test (foliar spraying) for controlling southern yellow thrips (Thrips palmi)

A cucumber leaf cut to a diameter of 1.5 cm was placed on cotton wool moistened with water. The diluent of the test compounds diluted to 200 ppm in an amount of 2 mL was sprayed onto the cucumber leaves with a spray tower. After air-drying, 5 larvae of southern yellow thrips in the first instar larva were released there. The sample with cotton wool was placed in a plastic cup, which was covered with a lid and kept in a chamber at a constant temperature of 25°C

for rearing. On 3 days after the treatment, the life or death was observed to calculate the mortality.

As a result, Compounds 1, 3, 12, 18, 21, 27, 38, 39, 42, 51, 57, 58, 60, 66, 84, 90, 100, 103, 112, 140, 168, 183 and 200 exhibited a mortality of 80% or more.

Test Example 1: Test (foliar spraying) for controlling cotton aphid (Aphis gossypii)

In the Biological Test Example 1, for example, Compound 51 of the present invention described in Table 7 exhibited more excellent effect in comparison with the prior art.

[Table 7]

| | Concentration (ppm) | Mortality of *Aphis gossypii* (%) |
|---|---|---|
| Compound 51 of the present invention 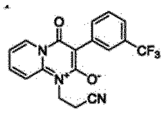 | 0.1 | 100 |
| WO2011/017342 Compound number 113 Dicloromezotiaz 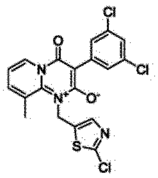 | 200<br>50<br>12.5 | 100<br>90<br>0 |
| WO2012/092115 Compound number 3 Triflumezopyrim 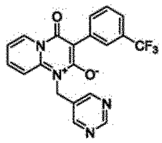 | 0.78<br>0.39<br>0.20 | 85<br>60<br>0 |
| WO2009/099929 Compound number 12 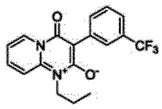 | 200<br>50 | 20<br>0 |
| WO2009/099929 Compound number 370 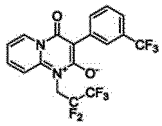 | 200 | 0 |

Test Example 2: Test (foliar spraying) for controlling brown planthopper (Nilaparvata lugens)

In the Biological Test Example 4, for example, Compounds 1, 3, 18, 21 and 48 of the present invention described in Table 8 exhibited more excellent effect in comparison with the prior art.

[Table 8]

| | Concentration (ppm) | Mortality of *Nilaparvata lugens* (%) |
|---|---|---|
| Compound 1 of the present invention 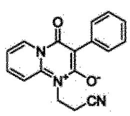 | 0.049<br>0.024<br>0.012 | 70<br>60<br>50 |
| Compound 3 of the present invention | 0.05 | 80 |

(continued)

| | Concentration (ppm) | Mortality of *Nilaparvata lugens* (%) |
|---|---|---|
| 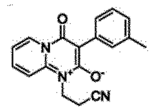 | 0.02<br>0.01 | 40<br>10 |
| Compound 18 of the present invention<br>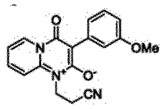 | 0.05<br>0.02<br>0.01 | 100<br>20<br>10 |
| Compound 21 of the present invention<br>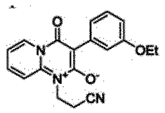 | 0.05<br>0.02<br>0.01 | 80<br>70<br>10 |
| Compound 48 of the present invention<br>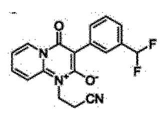 | 0.05<br>0.02<br>0.01 | 100<br>20<br>0 |
| WO2011/017342 Compound number 113 Dicloromezotiaz<br>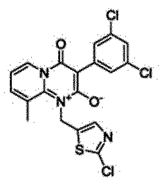 | 200 | 0 |
| WO2012/092115 Compound number 3 Triflumezopyrim<br>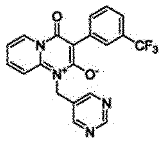 | 0.2<br>0.1 | 70<br>10 |

Test Example 3: Test (root dipping) for controlling brown planthopper (Nilaparvata lugens)

Ten sampled rice seedlings held with urethane in a glass cylinder having an inner diameter of 4.5 cm and a length of 14 cm were set up in a plastic cup containing 40 mL of chemical solution diluted to a desired concentration. After 2 days, 10 larvae of brown planthoppers in the third instar larva were released in the glass cylinder. The glass cylinder was covered with a lid of medicine paper and kept in a chamber at a constant temperature of 25°C for rearing. On 5 days after the treatment, the life or death of the larvae was observed to calculate the mortality.

In the present comparison test, for example, Compound 3 of the present invention described in Table 9 exhibited more excellent effect in comparison with the prior art.

[Table 9]

| | Concentration (ppm) | Mortality of *Nilaparvata lugens* (%) |
|---|---|---|
| Compound 3 of the present invention<br>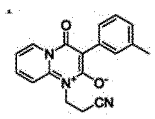 | 0.1<br>0.05<br>0.025 | 100<br>60<br>50 |

(continued)

| | Concentration (ppm) | Mortality of *Nilaparvata lugens* (%) |
|---|---|---|
| WO2011/017342 Compound number 113 Dicloromezotiaz 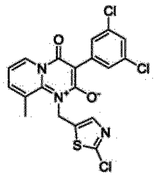 | 200 | 0 |
| WO2012/092115 Compound number 3 Triflumezopyrim 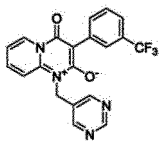 | 0.8<br>0.4<br>0.2<br>0.1<br>0.05 | 100<br>70<br>70<br>70<br>0 |

Test Example 4: Test (foliar spraying) for controlling cotton aphid (Aphis gossypii)

In Biological Test Example 1, for example, the compounds to which a straight-chain cyanoethyl group is bonded such as Compounds 3 and 51 described in Table 10 exhibited more excellent effect compared with compounds such as Comparative Compounds 1 to 4 having a relatively long carbon chain at the 1-position, compounds such as Comparative Compound 5 having a carbon chain with one carbon atom fewer at the 1-position, and compounds such as Comparative Compounds 6 and 7 having a branched carbon chain at the 1-position.

[Table 10]

| | Concentration (ppm) | Mortality of *Aphis gossypii* (%) |
|---|---|---|
| Compound 3 of the present invention 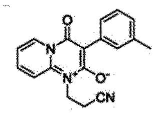 | 0.8 | 100 |
| Compound 51 of the present invention 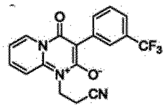 | 0.8 | 100 |
| Comparative compound 1 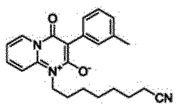 | 200 | 0 |
| Comparative compound 2 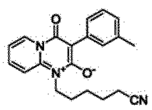 | 200<br>50 | 20<br>0 |
| Comparative compound 3 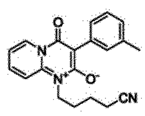 | 200<br>50<br>12.5 | 100<br>40<br>0 |
| Comparative compound 4 | 12.5 | 75 |

(continued)

| | Concentration (ppm) | Mortality of *Aphis gossypii* (%) |
|---|---|---|
| 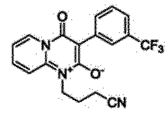 | 3.1 | 0 |
| Comparative compound 5 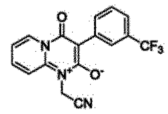 | 12.5<br>3.1 | 10<br>0 |
| Comparative compound 6 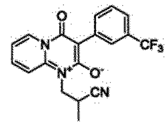 | 50<br>12.5<br>3.1 | 100<br>50<br>0 |
| Comparative compound 7 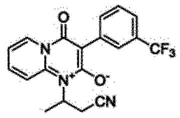 | 200 | 0 |

Test Example 5: Test (foliar spraying) for controlling southern yellow thrips (Thrips palmi)

In Biological Test Example 5, for example, Compound 18 of the present invention described in Table 11 exhibited more excellent effect in comparison with the prior art.

[Table 11]

| | Concentration (ppm) | Mortality of *Thrips palmi* (%) |
|---|---|---|
| Compound 18 of the present invention 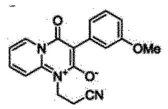 | 50<br>12.5<br>3.1 | 100<br>100<br>100 |
| WO2011/017342 Compound number 13 Dicloromezotiaz 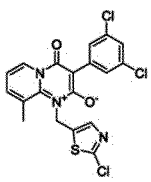 | 200 | 0 |
| WO2012/092115 Compound number 3 Triflumezopyrim 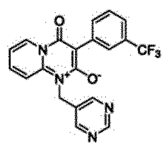 | 200<br>50<br>12.5 | 100<br>60<br>0 |

Test Example 6: Test (root dipping) for controlling rice leaf beetle (Oulema oryzae)

Ten sampled rice seedlings held with urethane in a glass cylinder having an inner diameter of 4.5 cm and a length of 14 cm were set up in a plastic cup containing 40 mL of chemical solution diluted to a desired concentration.

After 2 days, 4 larvae of rice leaf beetle in the late instar larva were released in the glass cylinder. The glass cylinder was covered with a lid of medicine paper and kept in a chamber at a constant temperature of 25°C for rearing. On 4 days after the treatment, the life or death and the abnormality of the larvae were observed to calculate the abnormality of the larvae.

In the present comparison test, for example, Compounds 3 and 51 of the present invention described in Table 12 exhibited more excellent effect in comparison with the prior art.

[Table 12]

| | Concentration (ppm) | Mortality of *Oulema oryzae* (%) |
|---|---|---|
| Compound 3 of the present invention 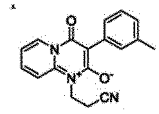 | 1.6<br>0.8 | 100<br>100 |
| Compound 51 of the present invention 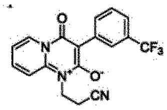 | 1.6<br>0.8<br>0.4 | 100<br>100<br>100 |
| WO2012/092115 Compound number 3 Triflumezopyrim 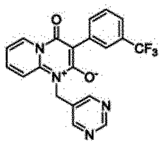 | 1.6 | 0 |

Test Example 7: Test (pot test) for controlling rice stem borer (Chilo suppressalis)

The roots of five sampled rice seedlings were washed with water. The wettable powder prepared in accordance with Formulation Example 2 described above was wrapped in the roots to be planted in a Wagner pot of 1/10000a. After 4 days from the treatment, a total of 25 hatchlings of rice stem borer were released per strain and reared in a plastic greenhouse with a room temperature set at 25°C. On 14 days after the treatment, the ratio of browning stem of seedlings for each strain and the ratio to untreated ones were calculated. Double system.

In the present comparison test, for example, Compounds 3, 51 and 57 of the present invention described in Table 13 exhibited more excellent effect in comparison with the prior art.

[Table 13]

| | Treatment chemical amount * | Browning stem ratio (%) | Ratio to untreated (%) |
|---|---|---|---|
| Compound 3 of the present invention 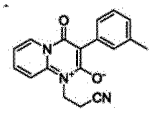 | 500g/ha | 0 | 0 |
| Compound 51 of the present invention 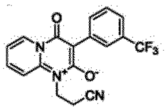 | 500g/ha | 10 | 12 |
| Compound 57 of the present invention 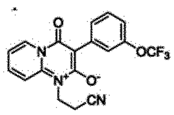 | 500g/ha | 0 | 0 |

(continued)

| | Treatment chemical amount * | Browning stem ratio (%) | Ratio to untreated (%) |
|---|---|---|---|
| WO2012/092115 Compound Number 3 Triflumezopyrim 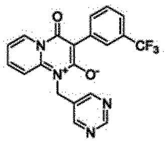 | 500g/ha | 40 | 49 |
| Untreated | - | 82 | 100 |
| * Gram number represents the weight of each test compound. | | | |

Test Example 8: Test (pot test) for controlling inedorooimushi (Oulema oryzae)

The roots of five sampled rice seedlings were washed with water. The wettable powder prepared in accordance with Formulation Example 2 described above was wrapped in the roots to be planted in a Wagner pot of 1/10000a. After 20 days from the treatment, 3 larvae of inedorooimushi in the late instar larva were released per strain and reared in a plastic greenhouse with a room temperature set at 25°C. On 2 days after the releasing of the insects, the mortality of the insect was calculated. In the present comparison test, for example, Compounds 3 and 51 of the present invention described in Table 14 exhibited more excellent effect in comparison with the prior art.

[Table 14]

| | Treatment chemical amount * | Mortality of *Oulema oryzae* (%) |
|---|---|---|
| Compound 3 of the present invention 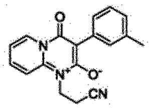 | 500g/ha<br>250g/ha<br>125g/ha | 100<br>50.0<br>33.3 |
| Compound 51 of the present invention 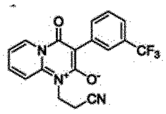 | 500g/ha<br>250g/ha<br>125g/ha | 100<br>100<br>83.3 |
| WO2012/092115 Compound Number 3 Triflumezopyrim 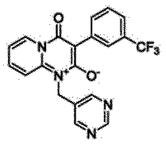 | 500g/ha<br>250g/ha<br>125g/ha | 66.7<br>50.0<br>16.7 |
| Untreated | - | 0 |
| * Gram number represents the weight of each test compound. | | |

Test Example 9: Residual effect test (field experiment) for brown planthopper (Nilaparvata lugens)

The roots of five sampled rice seedlings were washed with water. Subsequently, 25 mg of the wettable powder prepared in accordance with Formulation Example 2 described above was wrapped in the roots. Each strain was made from the five seedlings. In outdoor artificial paddy fields made from wooden frames having a size of 60 cm by 90 cm, 10 strains were planted, respectively. The artificial paddy field was shielded by an insect screen with a mesh size of 1 mm, so that invasion of harmful insects from the outside was prevented. Into the test system, 40 to 100 adult brown

planthoppers were released at fixed intervals so as to cause damage to the rice. On 16 to 51 days after the treatment, the numbers of surviving insects and dead insects were measured to calculate the density index. The density index was obtained from the following expression.

[Expression 1]

$$\text{Density index} = \frac{\text{Number of surviving insects after lapse of specified days in evaluation area}}{\text{Number of surviving insects after lapse of specified days in untreated area}} \times 100$$

In the present comparison test, for example, Compounds 3, 51 and 57 of the present invention described in Table 15 exhibited more excellent effect in comparison with the prior art.

[Table 15]

| | Density index of *Nilaparvata lugens* | | | | | |
|---|---|---|---|---|---|---|
| | After 16 days | After 23 days | After 30 days | After 37 days | After 44 days | After 51 days |
| Compound 3 of the present invention 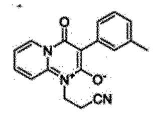 | 7.7 | 0 | 28.4 | 23.8 | 13.0 | 88.5 |
| Compound 51 of the present invention 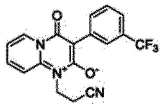 | 0 | 2.6 | 21.7 | 43.1 | 37.1 | 76.2 |
| Compound 57 of the present invention 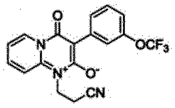 | 0 | 0 | 25.4 | 50.4 | 33.0 | 80.7 |
| WO2012/092115 Compound Number 3 Triflumezopyrim 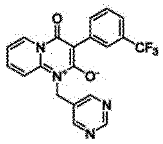 | 2.5 | 5.4 | 45.1 | 56.7 | 63.9 | 92.2 |
| Fipronil | 0 | 5.4 | 56.2 | 73.9 | 105 | 98.4 |

Test Example 10: Test (greenhouse test) for controlling brown winged green stink bug (Plautia stali)

Using a commercially available hand spray, a chemical solution diluted to a concentration of 100 ppm was sprayed onto an apple fruit (cultivar: Fuji). After air-drying, the apple fruit was placed in a plastic cup, which was allowed to stand still for a specified number of days in a plastic greenhouse with a room temperature set at 25°C and with the natural light shining thereon. On 7 days after the treatment, 5 adult brown winged green stink bugs (without distinction between male and female) were released onto the treated fruit. On 4 days after the release of insects, the numbers of death and abnormality were observed and recorded. Triple system. Also, the fruit after examination was immersed in an acidic 0.5% fuchsine solution for 30 minutes, and then washed with water to measure the total number of sapping scars.

In the present comparison test, for example, Compounds 3 and 51 of the present invention described in Table

16 exhibited more excellent effect in comparison with the prior art.

[Table 16]

| | Number of normal insects | Number of abnormal insects | Number of dead insects | Proportion of abnormal insects and dead insects on 4 days after releasing insects (%) | Total number of sheaths formed by stylet insertion |
|---|---|---|---|---|---|
| Compound 3 of the present invention 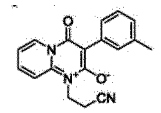 | 2 | 7 | 6 | 86.7 | 5 |
| Compound 51 of the present invention 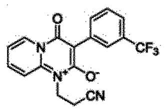 | 0 | 7 | 8 | 100 | 1 |
| WO2012/092115 Compound Number 3 Triflumezopyrim 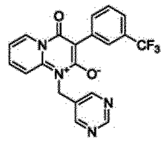 | 6 | 5 | 4 | 60.0 | 10 |
| Untreated | 14 | 0 | 1 | 6.7 | 26 |

**Claims**

**1.** A compound represented by a formula (1) or a salt thereof:

[Formula 1]

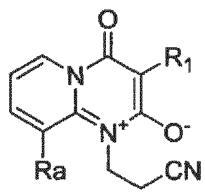

(1)

wherein

Ra represents a hydrogen atom, a halogen atom or a C1 alkyl group, wherein the C1 alkyl group is optionally substituted with one or more of the same or different halogen atoms;
$R_1$ represents a phenyl group or a pyridyl group, which is optionally substituted with up to three substituents $R_2$; wherein
each $R_2$ represents a halogen atom;
a cyano group;
a C1-C4 alkyl group,

wherein the C1-C4 alkyl group is optionally substituted with
one or more of the same or different halogen atoms, and/or
a C1-C3 alkyloxy group which is optionally substituted with one or more of the same or different halogen atoms, and/or
a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms;
a C2-C3 alkenyl group,
wherein the C2-C3 alkenyl group is optionally substituted with one or more of the same or different halogen atoms;
a C2-C3 alkynyl group,
wherein the C2-C3 alkynyl group is optionally substituted with one or more of the same or different halogen atoms;
a C1-C5 alkyloxy group,
wherein the C1-C5 alkyloxy group is optionally substituted with
one or more of the same or different halogen atoms, and/or
a C1-C3 alkyloxy group which is optionally substituted with one or more of the same or different halogen atoms, and/or
a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms;
an amino group having two substituents selected from a hydrogen atom, a C1-C4 alkyl group, and a C2 acyl group,
wherein the C1-C4 alkyl group is optionally substituted with
one or more of the same or different halogen atoms, and/or
a C1-C3 alkyloxy group which is optionally substituted with one or more of the same or different halogen atoms, and/or
a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms; and
wherein the C2 acyl group is optionally substituted with one or more of the same or different halogen atoms; or
a C1-C4 alkylthio group, a C1-C4 alkylsulfoxy group, or a C1-C4 alkylsulfonyl group,
wherein the C1-C4 alkylthio group, the C1-C4 alkylsulfoxy group, or the C1-C4 alkylsulfonyl group may be substituted with a substituent selected from the group consisting of:

one or more of the same or different halogen atoms, and/or
a C1-C3 alkyloxy group which is optionally substituted with one or more of the same or different halogen atoms, and/or

a C1-C3 alkylthio group which is optionally substituted with one or more of the same or different halogen atoms, and/or
a C1-C3 alkylsulfoxy group which is optionally substituted with one or more of the same or different halogen atoms, and/or
a C1-C3 alkylsulfonyl group which is optionally substituted with one or more of the same or different halogen atoms.

**2.** The compound or the salt thereof according to claim 1, wherein
Ra is a hydrogen atom or a methyl group; and
$R_1$ is a phenyl group which is optionally substituted with up to two substituents $R_2$; wherein
each $R_2$ is independently selected from the group consisting of:

a halogen atom,

a C1-C4 alkyl group which is optionally substituted with one or more of the same or different halogen atoms,
a C2-C3 alkenyl group which is optionally substituted with one or more of the same or different halogen atoms,
a C2-C3 alkynyl group which is optionally substituted with one or more of the same or different halogen atoms,
a C1-C5 alkyloxy group which is optionally substituted with one or more of the same or different halogen atoms,
an N-alkylacetamide group, wherein the alkyl group is C1-C4 and is optionally substituted with one or more of the same or different halogen atoms,
an N-alkyltrifluoroacetamide group, wherein the alkyl group is C1-C4 and is optionally substituted with one or more of the same or different halogen atoms,
an acetamide group,
a trifluoroacetamide group,
a C1-C4 alkylthio group which is optionally substituted with one or more of the same or different halogen atoms,
a C1-C4 alkylsulfoxy group which is optionally substituted with one or more of the same or different halogen atoms, and
a C1-C4 alkylsulfonyl group which is optionally substituted with one or more of the same or different halogen atoms.

**3.** The compound or the salt thereof according to claim 1, wherein
Ra is a hydrogen atom or a methyl group; and
$R_1$ is a phenyl group which is optionally substituted with up to two substituents $R_2$; wherein
each $R_2$ is:
a fluorine atom, a chlorine atom, a bromine atom, a methyl group, an ethyl group, an n-propyl group, an i-propyl group, a c-propyl group, an n-butyl group, an i-butyl, an s-butyl group, a t-butyl group, a fluoromethyl group, a difluoromethyl group, a trifluoromethyl group, a methyloxy group, an ethyloxy group, an n-propyloxy group, an i-propyloxy group, a c-propyloxy group, an n-butyloxy group, an i-butyloxy group, an s-butyloxy group, a t-butyloxy group, an n-pentyloxy group, a trifluoromethyl group, a 2,2,2-trifluoroethyloxy group, a methylthio group, a methylsulfoxy group, a methylsulfonyl group, a vinyl group, or an ethynyl group.

**4.** A compound or a salt thereof according to claim 1, wherein the compound represented by the formula (1) is selected from the group consisting of:

1-(2-cyanoethyl)-2-hydroxy-4-oxo-3-phenyl-4H-pyrido[1,2-a]pyrimidinium inner salt;
1-(2-cyanoethyl)-2-hydroxy-4-oxo-3 -(3-toluyl)-4H-pyrido[1,2-a]pyrimidinium inner salt;
1-(2-cyanoethyl)-3-(3-ethylphenyl)-2-hydroxy-4-oxo-4H-pyrido[1,2-a]pyrimidinium inner salt;
1-(2-cyanoethyl)-2-hydroxy-3-(3-isopropylphenyl)-4-oxo-4H-pyrido[1,2-a]pyrimidinium inner salt;
1-(2-cyanoethyl)-2-hydroxy-3-(3-tert-butylphenyl)-4-oxo-4H-pyrido[1,2-a]pyrimidinium inner salt;
1-(2-cyanoethyl)-2-hydroxy-3-(3-methoxyphenyl)-4-oxo-4H-pyrido[1,2-a]pyrimidinium inner salt;
1-(2-cyanoethyl)-3-(3-ethoxyphenyl)-2-hydroxy-4-oxo-4H-pyrido[1,2-a]pyrimidinium inner salt;
1-(2-cyanoethyl)-2-hydroxy-(3-n-propoxyphenyl)-4-oxo-3-4H-pyrido[1,2-a]pyrimidinium inner salt;
1-(2-cyanoethyl)-2-hydroxy-3-(3-isopropoxyphenyl)-4-oxo-4H-pyrido[1,2-a]pyrimidinium inner salt;
1-(2-cyanoethyl)-2-hydroxy-3-(3-n-butoxyphenyl)-4-oxo-4H-pyrido[1,2-a]pyrimidinium inner salt;
1-(2-cyanoethyl)-2-hydroxy-3-(3-n-pentyloxyphenyl)-4-oxo-4H-pyrido[1,2-a]pyrimidinium inner salt;
1-(2-cyanoethyl)-3-(2-fluorophenyl)-2-hydroxy-4-oxo-4H-pyrido[1,2-a]pyrimidinium inner salt;
1-(2-cyanoethyl)-3-(3-fluorophenyl)-2-hydroxy-4-oxo-4H-pyrido[1,2-a]pyrimidinium inner salt;
3-(3-chlorophenyl)-1-(2-cyanoethyl)-2-hydroxy-4-oxo-4H-pyrido[1,2-a]pyrimidinium inner salt;
3-(3-bromophenyl)-1-(2-cyanoethyl)-2-hydroxy-4-oxo-4H-pyrido[1,2-a]pyrimidinium inner salt;
1-(2-cyanoethyl)-3-(3-difluoromethylphenyl)-2-hydroxy-4-oxo-4H-pyrido[1,2-a]pyrimidinium inner salt;
1-(2-cyanoethyl)-2-hydroxy-4-oxo-3-(3-trifluoromethylphenyl)-4H-pyrido[1,2-a]pyrimidinium inner salt;
1-(2-cyanoethyl)-3-(3-difluoromethoxyphenyl)-2-hydroxy-4-oxo-4H-pyrido[1,2-a]pyrimidinium inner salt;
1-(2-cyanoethyl)-2-hydroxy-4-oxo-3-(3-trifluoromethoxyphenyl)-4H-pyrido[1,2-a]pyrimidinium inner salt;
1-(2-cyanoethyl)-2-hydroxy-4-oxo-3-(4-trifluoromethoxyphenyl)-4H-pyrido[1,2-a]pyrimidinium inner salt;
1-(2-cyanoethyl)-2-hydroxy-4-oxo-3-(3-(2,2,2-trifluoroethoxy)phenyl)-4H-pyrido[1,2-a]pyrimidinium inner salt;
1-(2-cyanoethyl)-2-hydroxy-3-(3-methylthiophenyl)-4-oxo-4H-pyrido[1,2-a]pyrimidinium inner salt;
1-(2-cyanoethyl)-2-hydroxy-3-(3-ethylthiophenyl)-4-oxo-4H-pyrido[1,2-a]pyrimidinium inner salt;
3 -(3 -acetamidophenyl)-1-(2-cyanoethyl)-2-hydroxy-4-oxo-4H-pyrido[1,2-a]pyrimidinium inner salt;
1-(2-cyanoethyl)-2-hydroxy-3-(3-(N-methylacetamide)phenyl)-4-oxo-4H-pyrido[1,2-a]pyrimidinium inner salt;
1-(2-cyanoethyl)-2-hydroxy-4-oxo-3-(3-vinylphenyl)-4H-pyrido[1,2-a]pyrimidinium inner salt;
1-(2-cyanoethyl)-2-hydroxy-3-(3,5-dimethylphenyl)-4-oxo-4H-pyrido[1,2-a]pyrimidinium inner salt;
1-(2-cyanoethyl)-2-hydroxy-3-(2,5-dimethoxyphenyl)-4-oxo-4H-pyrido[1,2-a]pyrimidinium inner salt;

3-(3,5-dichlorophenyl)-1-(2-cyanoethyl)-2-hydroxy-4-oxo-4H-pyrido[1,2-a]pyrimidinium inner salt;
1-(2-cyanoethyl)-3-(4-fluoro-3-methylphenyl)-2-hydroxy-4-oxo-4H-pyrido[1,2-a]pyrimidinium inner salt;
3-(3-chloro-6-methoxyphenyl)-1-(2-cyanoethyl)-2-hydroxy-4-oxo-4H-pyrido[1,2-a]pyrimidinium inner salt;
3-(2-chloro-5-trifluoromethylphenyl)-l-(2-cyanoethyl)-2-hydroxy-4-oxo-4H-pyrido[1,2-a]pyrimidinium inner salt;
1-(2-cyanoethyl)-2-hydroxy-3-(4-methyl-3-trifluoromethylphenyl)-4-oxo-4H-pyrido[1,2-a]pyrimidinium inner salt;
1-(2-cyanoethyl)-2-hydroxy-8-methyl-4-oxo-3-(3-toluyl)-4H-pyrido[1,2-a]pyrimidinium inner salt;
1-(2-cyanoethyl)-3-(3-ethoxyphenyl)-2-hydroxy-8-methyl-4-oxo-4H-pyrido[1,2-a]pyrimidinium inner salt;
1-(2-cyanoethyl)-2-hydroxy-8-methyl-4-oxo-3-(3-trifluoromethylphenyl)-4H-pyrido[1,2-a]pyrimidinium inner salt; and
1-(2-cyanoethyl)-3-(3,5-dichlorophenyl)-2-hydroxy-8-methyl-4-oxo-4H-pyrido[1,2-a]pyrimidinium inner salt.

**5.** A compound or a salt thereof according to claim 1, wherein the compound represented by the formula (1) is selected from the group consisting of:

1-(2-cyanoethyl)-2-hydroxy-4-oxo-3-(3-toluyl)-4H-pyrido[1,2-a]pyrimidinium inner salt;
1-(2-cyanoethyl)-2-hydroxy-3-(3-methoxyphenyl)-4-oxo-4H-pyrido[1,2-a]pyrimidinium inner salt;
1-(2-cyanoethyl)-3-(3-ethoxyphenyl)-2-hydroxy-4-oxo-4H-pyrido[1,2-a]pyrimidinium inner salt; and
1-(2-cyanoethyl)-2-hydroxy-4-oxo-3-(3-trifluoromethylphenyl)-4H-pyrido[1,2-a]pyrimidinium inner salt.

**6.** An insecticidal composition comprising the compound or the salt thereof according to any one of claims 1 to 5 and at least one component selected from the group consisting of a surfactant, a solid diluent and a liquid diluent.

**7.** The insecticidal composition according to claim 6, further comprising at least one biologically active compound or chemical agent.

**8.** The composition according to claim 7, wherein the at least one biologically active compound or chemical agent is selected from the group consisting of:
alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, metolcarb, fenothiocarb, fenoxycarb, acephate, azamethiphos, azinphos-ethyl, azinphos-methyl, ethylthiometon, chloroethoxyfos, cadusafos, chlorethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, EPN, ethion, ethoprophos, famphur, fenamiphos, fenitrothion, fenthion, fosthiazate, heptenophos, imicyafos, isofenphos, isopropyl=O-(methoxy aminothio phosphoryl), salicylates, isoxathion, malathion, mecarbam, methamidophos, methidathion, mevinphos, monocrotophos, naled, omethoate, oxydemeton ethyl, parathion, parathion-methyl, PAP, phorate, phosalone, phosmet, phosphamidon, phoxim, pirimiphos-methyl, profenofos-propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, chlorpyrifos-ethyl, disulfoton, sulprofos, flupyrazofos, phenthoate, fonofos, tribufos, endosulfan, alpha-endosulfan, gamma-HCH, dicofol, chlordane, dieldrin, methoxychlor, acetoprole, fipronil, ethiprole, pyrafluprole, pyriprole, flufiprole, broflanilide, afoxolaner, fluralaner, sarolarner, acrinathrin, allethrin, d-cis-trans allethrin, d-trans allethrin, bifenthrin, kappa-bifenthrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, ethofenprox, fenpropathrin, fenvalerate, flucythrinate, flumethrin, tau-fluvalinate, halfenprox, imiprothrin, kadethrin, permethrin, phenothrin, prallethrin, pyrethrin, resmethrin, silafluofen, tefluthrin, kappa-tefluthrin, phthalthrin, tetramethrin, tralomethrin, transfluthrin, methoxadiazone, metofluthrin, profluthrin, pyrethrum, terallethrin, momfluorothrin, heptafluthrin, meperfluthrin, tetramethylfluthrin, dimefluthrin, protrifenbute, acetamiprid, clothianidin, dinotefuran, imidacloprid, nitenpyram, thiacloprid, thiamethoxam, sulfoxaflor, flupyradifurone, triflumezopyrim, dicloromezotiaz, spinosad, spinetoram, abamectin, ivermectin, emamectin benzoate, milbemectin, lepimectin, hydroprene, kinoprene, diofenolan, methoprene, pyriproxyfen, pymetrozine, flonicamid, etoxazole, diafenthiuron, azocyclotin, cyhexatin, fenbutatin oxide, propargite, tetradifon, chlorfenapyr, tralopyril, DNOC, bensultap, cartap, thiocyclam, thiosultap, thiosultap-sodium, bistrifluron, chlorfluazuron, diflubenzuron, flucycloxuron, flufenoxuron, hexaflumuron, lufenuron, novaluron, noviflumuron, teflubenzuron, triflumuron, bistrifluron, buprofezin, cyromazine, chromafenozide, halofenozide, methoxyfenozide, tebufenozide, amitraz, hydramethylnon, acequinocyl, fluacrypyrim, pyriminostrobin, flufenoxystrobin, fenazaquin, fenpyroximate, pyrimidifen, pyridaben, tebufenpyrad, tolfenpyrad, pyflubumide, spirodiclofen, spirotetramat, spiromesifen, cyflumetofen, cyenopyrafen, flubendiamide, chlorantraniliprole, cyantraniliprole, cyclaniliprole, tetraniliprole, quinomethionate, hexythiazox, bifenazate, flufenerim, pyrifluquinazon, flometoquin, fluopyram, 8-chloro-N-((2-chloro-5-methoxyphenyl)sulfonyl)-6-(trifluoromethyl)imidazo[1,2-a]pyridine-2-carboxamide, amidoflumet, nicotine, chloropicrin, sulfuryl fluoride, chlorothie, clofentezine, diflovidazin, rotenone, indoxacarb, piperonyl butoxide, chlordimeform, pyridalyl, azadirachtin, benzoxymate, afidopiropen, fluhexafon, tioxazafen, fluensulfone, benclothiaz, carzole, an insecticidal soap, dimehypo, nithiazine, a borate, metaldehyde, ryanodine, sulfluramid, (E)-N-(1-((6-chloropyridin-3-yl)methyl)pyridine-2(1H)-ylidene)-2,2,2-trifluoroacetamide, 5-(trifluoromethyl)-2-(4-(4-(trifluoromethyl)phenoxy)piperidin-1-yl)pyridine, metalaxyl, metalaxyl-M, oxadixyl, ofurace, benalaxyl, benalaxyl-M, kiralaxyl, ofurace, furalaxyl, cyprofuram, bupirimate, dimethirimol, ethirimol, hymexazol, hydroxyisoxazole, oxathiapiprolin, octhilinone, oxolinic acid, benomyl, thiophanate-methyl, carbendazim, fuberidazole, thiabendazole, debacarb, diethofencarb, zoxamide, ethaboxam, pencycuron, fluopicolide, diflumetorim, bupirimate, benodanil, flutolanil, mepronil, isofetamid, fenfuram, oxycarboxin, carboxin, thifluzamide, fluxapyroxad, furametpyr, penflufen, penthiopyrad, benzovindiflupyr, bixafen, isopyrazam, sedaxane, boscalid, azoxystrobin, coumetoxystrobin, kresoxym-methyl, trifloxystrobin, picoxystrobin, pyraclostrobin, dimoxystrobin, metominostrobin, orysastrobin, fluoxastrobin, pyraoxystrobin, pyrametostrobin, flufenoxystrobin, fenaminstrobin, enoxastrobin, coumoxystrobin, mandestrobin, triclopyricarb, famoxadon, fenamidone, triclopyricarb, pyribencarb, cyazofamid, amisulbrom, binapacryl, meptyldinocarb, dinocap, fluazinam, ferimzone, fentin-acetate, fentin chloride, fentin hydroxide, triphenyltin hydroxide, triphenyltin acetate, oxine-copper, silthiofam, ametoctradin, mepanipyrim, nitrapyrin, pyrimethanil, cyprodinil, blasticidin S, kasugamycin, kasugamycin hydrochloride hydrate, streptomycin, oxytetracyclin, quinoxyfen, proquinazid, fludioxonil, fenpiclonil, fluoroimide, procymidone, iprodione, vinclozolin, edifenphos, iprobenfos, pyrazophos, isoprothiolane, propamocarb, propamocarb hydrochloride, tea tree oil extract, triforine, pyrifenox, pyrisoxazole, fenarimol, nuarimol, azaconazole, bromuconazole, diniconazole, diniconazole-M, epoxiconazole, fluquinconazole, oxpoconazole, pefurazoate, difenoconazole, fenbuconazole, imibenconazole, ipconazole, metconazole, tetraconazole, triadimefon, triadimenol, triticonazole, uniconazole, imazalil, bitertanol, triflumizole, etaconazole, propiconazole, penconazole, flusilazole, flutriafol, myclobutanil, paclobutrazol, prothioconazole, cyproconazole, tebuconazole, hexaconazole, prochloraz, simeconazole, aldimorph, dodemorph, dodemorph acetate, tridemorph, fenpropimorph, dimethomorph, flumorph, pyrimorph, piperalin, fenpropidin, spiroxamine, fenhexamid, fenpyrazamine, ferbam, metam, methasulfocarb, metiram, thiram, manzeb, maneb, zineb, ziram, polycarbamate, propineb, thiuram, pyributicarb, validamycin, mildiomycin, polyoxin, benthiavalicarb, benthiavalicarb-isopropyl, valifenalate, iprovalicarb, mandipropamid, fthalide, pyroquilon, tricyclazole, carpropamid, diclocymet, fenoxanil, acibenzolar-S-methyl, probenazole, tiadinil, isotianil, cymoxanil, fosetyl, tecloftalam, triazoxide, flusulfamide, diclomezine, cyflufenamid, metrafenone, pyriofenon, flutianil, tebufloquin, fosetyl aluminum, tolclofos-methyl, echlomezol, tolprocarb, Bordeaux mixture, copper acetate, basic copper sulfate, copper oxychloride, cupric hydroxide, oxyquinoline copper, copper, sulfur, captan, captafol, folpet, anilazine, chlorothalonil, dichlorophene, pentachlorophenol and salts thereof, hexachlorobenzene, quintozene, iminoctadine acetate, iminoctadine albesilate, guanidine, dodine, dodine free base, guazatine, guazatine acetate, albesilate, dithianon, quinomethionate, fluoroimide, tolylfluanid, dichlofluanid, dinobuton, dazomet, dipymetitrone, pyraziflumid, picarbutrazox, tecnazene, nitrothal-isopropyl, dicyclomet, acibenzolar, prohexadione-calcium, bronopol, diphenylamine, flumetover, bentoxazin, biphenyl, chloroneb, CNA, iodocarb, prothiocarb, N-(1-(2,4-dichlorophenyl)-1-methoxypropan-2-yl)-2-iodobenzamide, bromide, 3-(5-fluoro-3,3,4,4-tetramethyl-3,4-dihydroisoquinolin-1-yl)quinoline, 3-(4,4-difluoro-3,3-dimethyl-3,4-dihydroisoquinolin-1-yl)quinoline, 3-(4,4,5-trifluoro-3,3-dimethyl-3,4-dihydroisoquinolin-1-yl)quinoline, Bacillus species and insecticidal proteins and bactericidal proteins produced therefrom, and insecticidal proteins, bactericidal proteins, entomopathogenic bacteria, entomopathogenic viruses and entomopathogenic fungi produced from Bt crops.

**9.** A composition for protecting animals from harmful parasitic invertebrate organisms, comprising the compound or the salt thereof according to claim 1 in a parasiticidally effective amount and at least one carrier.

**10.** A method for controlling harmful invertebrate organisms, comprising bringing the harmful invertebrate organisms or the environment thereof into contact with the compound or the salt thereof according to claim 1 in a biologically effective amount, wherein the environment thereof does not encompass the human or animal body.

**11.** A method for enhancing the vitality of crops, comprising bringing the crops, seeds from which crops will grow, or the base of crops into contact with the compound or the salt thereof according to claim 1 in a biologically effective amount.

**12.** A treated seed comprising the compound or the salt thereof according to claim 1 in an amount of about 0.0001 to about 1 mass% relative to the seed after treatment.

## Patentansprüche

1. Verbindung, dargestellt durch eine Formel (1) oder ein Salz davon:

[Formel 1]

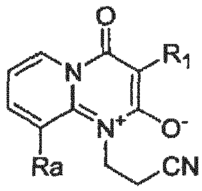

(1)

wobei

Ra ein Wasserstoffatom, ein Halogenatom oder eine Cl-Alkylgruppe darstellt, wobei die Cl-Alkylgruppe optional mit einem oder mehreren der gleichen oder unterschiedlichen Halogenatome substituiert ist;
$R_1$ eine Phenylgruppe oder eine Pyridylgruppe darstellt, die optional mit bis zu drei Substituenten $R_2$ substituiert ist, wobei
jedes $R_2$ ein Halogenatom darstellt;
eine Cyanogruppe;
eine C1-C4-Alkylgruppe,
wobei die C1-C4-Alkylgruppe optional mit
einem oder mehreren der gleichen oder unterschiedlichen Halogenatome substituiert ist, und/oder
eine C1-C3-Alkyloxygruppe, die optional mit einem oder mehreren der gleichen oder unterschiedlichen Halogenatome substituiert ist, und/oder
eine C1-C3-Alkylthiogruppe, die optional mit einem oder mehreren der gleichen oder unterschiedlichen Halogenatome substituiert ist, und/oder
eine C1-C3-Alkylsulfoxygruppe, die optional mit einem oder mehreren der gleichen oder unterschiedlichen Halogenatome substituiert ist, und/oder
eine C1-C3-Alkylsulfonylgruppe, die optional mit einem oder mehreren der gleichen oder unterschiedlichen Halogenatome substituiert ist;
eine C2-C3-Alkenylgruppe,
wobei die C2-C3-Alkenylgruppe optional mit einem oder mehreren der gleichen oder unterschiedlichen Halogenatome substituiert ist;
eine C2-C3-Alkynylgruppe,
wobei die C2-C3-Alkynylgruppe optional mit einem oder mehreren der gleichen oder unterschiedlichen Halogenatome substituiert ist;
eine C1-C5-Alkyloxygruppe,
wobei die C1-C5-Alkyloxygruppe optional mit
einem oder mehreren der gleichen oder unterschiedlichen Halogenatome substituiert ist, und/oder
eine C1-C3-Alkyloxygruppe, die optional mit einem oder mehreren der gleichen oder unterschiedlichen Halogenatome substituiert ist, und/oder
eine C1-C3-Alkylthiogruppe, die optional mit einem oder mehreren der gleichen oder unterschiedlichen Halogenatome substituiert ist, und/oder
eine C1-C3-Alkylsulfoxygruppe, die optional mit einem oder mehreren der gleichen oder unterschiedlichen Halogenatome substituiert ist, und/oder
eine C1-C3-Alkylsulfonylgruppe, die optional mit einem oder mehreren der gleichen oder unterschiedlichen Halogenatome substituiert ist;
eine Aminogruppe mit zwei Substituenten, ausgewählt aus einem Wasserstoffatom, einer C1-C4-Alkylgruppe und einer C2-Acylgruppe,
wobei die C1-C4-Alkylgruppe optional mit
einem oder mehreren der gleichen oder unterschiedlichen Halogenatome substituiert ist, und/oder
eine C1-C3-Alkyloxygruppe, die optional mit einem oder mehreren der gleichen oder unterschiedlichen Halogenatome substituiert ist, und/oder
eine C1-C3-Alkylthiogruppe, die optional mit einem oder mehreren der gleichen oder unterschiedlichen Halogenatome substituiert ist, und/oder
eine C1-C3-Alkylsulfoxygruppe, die optional mit einem oder mehreren der gleichen oder unterschiedlichen Halogenatome substituiert ist, und/oder
eine C1-C3-Alkylsulfonylgruppe, die optional mit einem oder mehreren der gleichen oder unterschiedlichen Halogenatome substituiert ist; und
wobei die C2-Acylgruppe optional mit einem oder mehreren der gleichen oder unterschiedlichen Halogenatome substituiert ist; oder
eine C1-C4-Alkylthiogruppe, eine C1-C4-Alkylsulfoxygruppe oder eine C1-C4-Alkylsulfonylgruppe,
wobei die C1-C4-Alkylthiogruppe, die C1-C4-Alkylsulfoxygruppe oder die C1-C4-Alkylsulfonylgruppe mit einem Substituenten substituiert sein kann, der ausgewählt ist aus der Gruppe bestehend aus:

einem oder mehreren der gleichen oder unterschiedlichen Halogenatome, und/oder
einer C1-C3-Alkyloxygruppe, die optional mit einem oder mehreren der gleichen oder unterschiedlichen Halogenatome substituiert ist, und/oder
einer C1-C3-Alkylthiogruppe, die optional mit einem oder mehreren der gleichen oder unterschiedlichen Halogenatome substituiert ist, und/oder
einer C1-C3-Alkylsulfoxygruppe, die optional mit einem oder mehreren der gleichen oder unterschiedlichen Halogenatome substituiert ist, und/oder
einer C1-C3-Alkylsulfonylgruppe, die optional mit einem oder mehreren der gleichen oder unterschiedlichen Halogenatome substituiert ist.

**2.** Verbindung oder das Salz davon nach Anspruch 1, wobei
Ra ein Wasserstoffatom oder eine Methylgruppe ist; und
$R_1$ eine Phenylgruppe ist, die optional mit bis zu zwei Substituenten $R_2$ substituiert ist; wobei
jedes $R_2$ unabhängig ausgewählt ist aus der Gruppe bestehend aus:

einem Halogenatom,
einer C1-C4-Alkylgruppe, die optional mit einem oder mehreren der gleichen oder unterschiedlichen Halogenatome substituiert ist,
einer C2-C3-Alkenylgruppe, die optional mit einem oder mehreren der gleichen oder unterschiedlichen Halogenatome substituiert ist,
einer C2-C3-Alkynylgruppe, die optional mit einem oder mehreren der gleichen oder unterschiedlichen Halogenatome substituiert ist,
einer C1-C5-Alkyloxygruppe, die optional mit einem oder mehreren der gleichen oder unterschiedlichen Halogenatome substituiert ist,
einer N-Alkylacetamidgruppe, wobei die Alkylgruppe C1-C4 ist und optional mit einem oder mehreren der gleichen oder unterschiedlichen Halogenatome substituiert ist,
einer N-Alkyltrifluoracetamidgruppe, wobei die Alkylgruppe C1-C4 ist und optional mit einem oder mehreren der gleichen oder unterschiedlichen Halogenatome substituiert ist,
einer Acetamidgruppe,
einer Trifluoracetamidgruppe,
einer C1-C4-Alkylthiogruppe, die optional mit einem oder mehreren der gleichen oder unterschiedlichen Halogenatome substituiert ist,
einer C1-C4-Alkylsulfoxygruppe, die optional mit einem oder mehreren der gleichen oder unterschiedlichen Halogenatome substituiert ist, und
einer C1-C4-Alkylsulfonylgruppe, die optional mit einem oder mehreren der gleichen oder unterschiedlichen Halogenatome substituiert ist.

**3.** Verbindung oder das Salz davon nach Anspruch 1, wobei
Ra ein Wasserstoffatom oder eine Methylgruppe ist; und
$R_1$ eine Phenylgruppe ist, die optional mit bis zu zwei Substituenten $R_2$ substituiert ist; wobei
jedes $R_2$:
ein Fluoratom, ein Chloratom, ein Bromatom, eine Methylgruppe, eine Ethylgruppe, eine n-Propylgruppe, eine i-Propylgruppe, eine c-Propylgruppe, eine n-Butylgruppe, eine i-Butylgruppe, eine s-Butylgruppe, eine t-Butylgruppe, eine Fluormethylgruppe, eine Difluormethylgruppe, eine Trifluormethylgruppe, eine Methyloxygruppe, eine Ethyloxygruppe, eine n-Propyloxygruppe, eine i-Propyloxygruppe, eine c-Propyloxygruppe, eine n-Butyloxygruppe, eine i-Butyloxygruppe, eine s-Butyloxygruppe, eine t-Butyloxygruppe, eine n-Pentyloxygruppe, eine Trifluormethylgruppe, eine 2,2,2-Trifluorethyloxygruppe, eine Methylthiogruppe, eine Methylsulfoxygruppe, eine Methylsulfonylgruppe,

eine Vinylgruppe oder eine Ethinylgruppe ist.

4. Verbindung oder ein Salz davon nach Anspruch 1, wobei die durch die Formel (1) dargestellte Verbindung ausgewählt ist aus der Gruppe bestehend aus:

1-(2-Cyanethyl)-2-hydroxy-4-oxo-3-phenyl-4H-pyrido[1,2-a]pyrimidinium-Zwitterion;
1-(2-Cyanethyl)-2-hydroxy-4-oxo-3-(3-Toluyl)-4H-pyrido[1,2-a]pyrimidinium - Zwitterion;
1-(2-Cyanethyl)-3-(3-Ethylphenyl)-2-hydroxy-4-oxo-4H-pyrido[1,2-a]pyrimidinium-Zwitterion;
1-(2-Cyanethyl)-2-hydroxy-3-(3-Isopropylphenyl)-4-oxo-4H-pyrido[1,2-a]pyrimidinium-Zwitterion;
1-(2-Cyanethyl)-2-hydroxy-3-(3-Tert-Butylphenyl)-4-oxo-4H-pyrido[1,2-a]pyrimidinium-Zwitterion;
1-(2-Cyanethyl)-2-hydroxy-3-(3-Methoxyphenyl)-4-oxo-4H-pyrido[1,2-a]pyrimidinium-Zwitterion;
1-(2-Cyanethyl)-3-(3-Ethoxyphenyl)-2-hydroxy-4-oxo-4H-pyrido[1,2-a]pyrimidinium-Zwitterion;
1-(2-Cyanethyl)-2-hydroxy-(3-N-Propoxyphenyl)-4-oxo-3-4H-pyrido[1,2-a]pyrimidinium-Zwitterion;
1-(2-Cyanethyl)-2-hydroxy-3-(3-Isopropoxyphenyl)-4-oxo-4H-pyrido[1,2-a]pyrimidinium-Zwitterion;
1-(2-Cyanethyl)-2-hydroxy-3-(3-N-Butoxyphenyl)-4-oxo-4H-pyrido[1,2-a]pyrimidinium-Zwitterion;
1-(2-Cyanethyl)-2-hydroxy-3-(3-N-Pentyloxyphenyl)-4-oxo-4H-pyrido[1,2-a]pyrimidinium-Zwitterion;
1-(2-Cyanethyl)-3-(2-Fluorphenyl)-2-hydroxy-4-oxo-4H-pyrido[1,2-a]pyrimidinium-Zwitterion;
1-(2-Cyanethyl)-3-(3-Fluorphenyl)-2-hydroxy-4-oxo-4H-pyrido[1,2-a]pyrimidinium-Zwitterion;
3-(3-Chlorphenyl)-1-(2-Cyanethyl)-2-hydroxy-4-oxo-4H-pyrido[1,2-a]pyrimidinium-Zwitterion;
3-(3-Bromophenyl)-1-(2-Cyanethyl)-2-hydroxy-4-oxo-4H-pyrido[1,2-a]pyrimidinium-Zwitterion;
1-(2-Cyanethyl)-3-(3-Difluormethylphenyl)-2-hydroxy-4-oxo-4H-pyrido[1,2-a]pyrimidinium-Zwitterion;
1-(2-Cyanethyl)-2-hydroxy-4-oxo-3-(3-Trifluormethylphenyl)-4H-pyrido[1,2-a]pyrimidinium-Zwitterion;
1-(2-Cyanethyl)-3-(3-Difluormethoxyphenyl)-2-hydroxy-4-oxo-4H-pyrido[1,2-a]pyrimidinium-Zwitterion;
1-(2-Cyanethyl)-2-hydroxy-4-oxo-3-(3-Trifluormethoxyphenyl)-4H-pyrido(1,2-a]pyrimidinium-Zwitterion;
1-(2-Cyanethyl)-2-hydroxy-4-oxo-3-(4-Trifluormethoxyphenyl)-4H-pyrido[1,2-a]pyrimidinium-Zwitterion;
1-(2-Cyanethyl)-2-hydroxy-4-oxo-3-(3-(2,2,2-Trifluorethoxy)phenyl)-4H-pyrido[1,2-a]pyrimidinium-Zwitterion;
1-(2-Cyanethyl)-2-hydroxy-3-(3-Methylthiophenyl)-4-oxo-4H-pyrido[1,2-a]pyrimidinium-Zwitterion;
1-(2-Cyanethyl)-2-hydroxy-3-(3-Ethylthiophenyl)-4-oxo-4H-pyrido[1,2-a]pyrimidinium-Zwitterion;
3-(3-Acetamidophenyl)-1-(2-Cyanethyl)-2-hydroxy-4-oxo-4H-pyrido[1,2-a]pyrimidinium-Zwitterion;
1-(2-Cyanethyl)-2-hydroxy-3-(3-(N-Methylacetamid)phenyl)-4-oxo-4H-pyrido[1,2-a]pyrimidinium-Zwitterion;
1-(2-Cyanethyl)-2-hydroxy-4-oxo-3-(3-Vinylphenyl)-4H-pyrido[1,2-a]pyrimidinium-Zwitterion;
1-(2-Cyanethyl)-2-hydroxy-3-(3,5-Dimethylphenyl)-4-oxo-4H-pyrido[1,2-a]pyrimidinium-Zwitterion;
1-(2-Cyanethyl)-2-hydroxy-3-(2,5-Dimethoxyphenyl)-4-oxo-4H-pyrido[1,2-a]pyrimidinium-Zwitterion;
3-(3,5-Dichlorphenyl)-1-(2-Cyanethyl)-2-hydroxy-4-oxo-4H-pyrido[1,2-a]pyrimidinium-Zwitterion;
1-(2-Cyanethyl)-3-(4-Fluor-3-Methylphenyl)-2-hydroxy-4-oxo-4H-pyrido[1,2-a]pyrimidinium-Zwitterion;
3-(3-chlor-6-Methoxyphenyl)-1-(2-Cyanethyl)-2-hydroxy-4-oxo-4H-pyrido[1,2-a]pyrimidinium-Zwitterion;
3-(2-Chlor-5-trifluormethylphenyl)-1-(2-Cyanethyl)-2-hydroxy-4-oxo-4H-pyrido[1,2-a]pyrimidinium-Zwitterion;
1-(2-Cyanethyl)-2-hydroxy-3-(4-Methyl-3-Trifluormethylphenyl)-4-oxo-4H-pyrido[1,2-a]pyrimidinium-Zwitterion;
1-(2-Cyanethyl)-2-hydroxy-8-methyl-4-oxo-3-(3-Toluyl)-4H-pyrido[1,2-a]pyrimidmium-Zwitterion;
1-(2-Cyanethyl)-3-(3-Ethoxyphenyl)-2-hydroxy-8-methyl-4-oxo-4H-pyrido[1,2-a]pyrimidinium-Zwitterion;
1-(2-Cyanethyl)-2-hydroxy-8-methyl-4-oxo-3-(3-Trifluormethylphenyl)-4H-pyrido[1,2-a]pyrimidinium-Zwitterion; und
1-(2-Cyanethyl)-3-(3,5-Dichlorphenyl)-2-hydroxy-8-methyl-4-oxo-4H-pyrido[1,2-a]pyrimidinium-Zwitterion;

5. Verbindung oder ein Salz davon nach Anspruch 1, wobei die durch die Formel (1) dargestellte Verbindung ausgewählt ist aus der Gruppe bestehend aus:

1-(2-Cyanethyl)-2-hydroxy-4-oxo-3-(3-Toluyl)-4H-pyrido[1,2-a]pyrimidinium-Zwitterion;
1-(2-Cyanethyl)-2-hydroxy-3-(3-Methoxyphenyl)-4-oxo-4H-pyrido[1,2-a]pyrimidinium-Zwitterion;
1-(2-Cyanethyl)-3-(3-Ethoxyphenyl)-2-hydroxy-4-oxo-4H-pyrido[1,2-a]pyrimidinium-Zwitterion; und
1-(2-Cyanethyl)-2-hydroxy-4-oxo-3-(3-Trifluormethylphenyl)-4H-pyrido[1,2-a]pyrimidinium-Zwitterion.

6. Insektizide Zusammensetzung, umfassend die Verbindung oder das Salz davon nach einem der Ansprüche 1 bis 5 und mindestens eine Komponente, die ausgewählt ist aus der Gruppe bestehend aus einem Tensid, einem festen Verdünnungsmittel und einem flüssigen Verdünnungsmittel.

7. Insektizide Zusammensetzung nach Anspruch 6, weiter umfassend mindestens eine biologisch aktive Verbindung

oder ein chemisches Mittel.

8. Zusammensetzung nach Anspruch 7, wobei die mindestens eine biologisch aktive Verbindung oder das chemische Mittel ausgewählt ist aus der Gruppe bestehend aus: Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanat, Furathiocarb, Isoprocarb, Methiocarb, Methomyl, Oxamyl, Pirimicarb, Propoxur, Thiophodicarb, Thiofanox, Triazamat, Trimethacarb, XMC, Xylylcarb, Metolcarb, Fenothiocarb, Fenoxycarb, Acephat, Azamethiphos, Azinphosethyl, Azinphosmethyl, Ethylthiometon, Chlorethoxyfos, Cadusafos, Chlorethoxyfos, Chlorfenvinphos, Chlormephos, Chlorpyrifos, Chlorpyrifosmethyl, Kumaphos, Cyanophos, Demeton-S-methyl, Diazinon, Dichlorvos, Dicrotophos, Dimethoat, Dimethylvinphos, EPN, Ethion, Ethoprophos, Famphur, Fenamiphos, Fenitrothion, Fenthion, Fosthiazat, Heptenophos, Imicyafos, Isofenphos, Isopropyl=O-(Methoxy-aminothiophosphoryl), Salicylat, Isoxathion, Malathion, Mecarbam, Methamidophos, Methidathion, Mevinphos, Monocrotophos, Naled, Methoat, Oxidemeton Ethyl, Parathion, Parathionmethyl, PAP, Phorat, Phosalon, Phosmet, Phosphamidon, Phoxim, Pirimiphosmethyl, Profenofos-Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon, Vamidothion, Chlorpyrifos-Ethyl, Disulfoton, Sulprofos, Flupyrazofos, Phenthoat, Fonofos, Tribufos, Endosulfan, Alpha-Endosulfan, Gamma-HCH, Dicofol, Chlordan, Dieldrin, Methoxychlor, Acetoprol, Fipronil, Ethiprol, Pyrafluprol, Pyriprol, Flufiprol, Broflanilid, Foxolaner, Floralaner, Sarolamer, Akrinathrin, Allethrin, d-cis-trans-Allethrin, d-trans-Allethrin, Bifenthrin, Kappa-Bifenthrin, Bioallethrin S-Cyclopentenyl, Biosmethrin, Cycloprothrin, Cycluthrin, Beta-Cycluthrin, Chalothrin, Lambda-Chalothrin, Gamma-Chalothrin, Cypermethrin, Alpha-Cypermethrin, Beta-Cypermethrin, Theta-Cypermethrin, Zeta-Cypermethrin, Cypphenothrin, Deltamethrin, Empenthrin, Esfenvalerat, Ethofenprox, Fenpropathrin, Fenvalerat, Flucythrinat, Flumethrin, Tau-Fluvalinat, Halfenprox, Imiprothrin, Kadethrin, Permethrin, Phenothrin, Prallethrin, Pyrethrin, Resmethrin, Silafluofen, Tefluthrin, Kappa-Tefluthrin, Phthalthrin, Tetramethrin, Tralomethrin, Transfluthrin, Methoxadiazon, Metofluthrin, Profluthrin, Pyrethrum, Terallethrin, Momfluorthrin, Heptafluthrin, Meperfluthrin, Tetramethylfluthrin, Dimefluthrin, Protrifenbut, Acetamiprid, Clothianidin, Dinotefuran, Imidacloprid, Nitenpyram, Thiacloprid, Thiamethoxam, Sulfoxaflor, Flupyradifuron, Triflumezopyrim, Dichlormezotiaz, Spinosad, Spinetoram, Abamectin, Ivermectin, Emamectinbenzoat, Milbemectin, Lepimectin, Hydropren, Kinopren, Diofenolan, Methopren, Pyriproxyfen, Pymetrozin, Flonamid, Etoxazol, Diafenthiuron, Azocyclotin, Cyhexatin, Fenbutatinoxid, Propargit, Tradifon, Chlorfenapyr, Trachopyril, DNOC, Bensultap, Cartap, Thiocyclam, Thiosultap, Thiosultapnatrium, Bistrifluron, Chlorfluazuron, Diflubenzuron, Flucycloxuron, Flufenoxuron, Hexaflumuron, lufenuron, Novaluron, Noviflumuron, Teflubenzuron, Triflumuron, Bistrifluron, Buprofezin, Cromazin, Chromafenozid, Halogenofenozid, Methoxyfenozid, Tebufenozid, Ammitraz, Hydramethylnon, Acequinocyl, Fluacrypyrim, Pyriminostrobin, Flufenoxystrobin, Fenazaquin, Fenpyroximat, Pyrimidifen, Pyridaben, Tebufenpyrad, Tolfenpyrad, Pyflubumid, Spirodiclofen, Spirotetramat, Spiromesifen, Cyflumetofen, Cyenopyrafen, Flubendiamid, Chlorantraniliprol, Cyantraniliprol, Cyclaniliprol, Tetraniliprol, Chinomethionat, Hexythiazox, Bifenazat, Flufenerim, Pyrifluquinazon, Flometoquin, Fluopyram, 8-Chlor-N-((2-Chlor-5-methoxyphenyl)sulfonyl)-6-(trifluormethyl)imidazo[1,2-a]pyridin-2-carboxamid, Amidoflumet, Nikotin, Chlorpikrin, Sulfurylfluorid, Chlorthie, Clofentezin, Diflovidazin, Rotenon, Indoxacarb, Piperonylbutoxid, Chlordimeform, Pyridalyl, Azadirachtin, Benzoxymat, Fidopiropen, Fluhexafon, Tioxazafen, Fluensulfon, Benclothiaz, Carzol, eine insektizide Seife, Dimehypo, Nithiazin, ein Borat, Metallaldehyd, Ryanodin, Schwefelfluramid, (E)-N-(1-((6-Chlorpyridin-3-yl)methyl)pyridin-2(1H)-yliden)-2,2,2-trifluoracetamid, 5-(trifluormethyl)-2-(4-(4-(trifluormethyl)phenoxy)piperidin-1-yl)pyridin, Metalaxyl, Metalaxyl-M, Oxadixyl, Ofurac, Benalaxyl, Benalaxyl-M, Kiralaxyl, Ofurac, Furalaxyl, Cyprofuram, Bupirimat, Dimethiricinol, Ethirimol, Hymexazol, Hydroxyisoxazol, Oxathiaprolin, Othilinon, Oxolinsäure, Benomyl, Thiophanatmethyl, Carbendazim, Fuberidazol, Thiabendazol, Debacarb, Diethofencarb, Zoxamid, Ethaboxam, Pencycuron, Fluopicolid, Diflumetorim, Bupirimat, Benodanil, Flutolanil, Mepronil, Isofetamid, Fenfuram, Oxycarboxin, Carboxin, Thifluzamid, Fluxapyroxad, Furametpyr, Penflufen, Penthiopyrad, Benzovindiflupyr, Bixafen, Isopyrazam, Sedaxan, Boscalid, Azoxystrobin, Coumetoxystrobin, Kresoxymethyl, Trifloxystrobin, Picoxystrobin, Pyraclostrobin, Dimoxystrobin, Metominostrobin, Orysastrobin, Fluoxastrobin, Pyraoxystrobin, Pyrametostrobin, Flufenoxystrobin, Fenaminstrobin, Enoxastrobin, Coumoxystrobin, Mandestrobin, Triclopyricarb, Famoxadon, Fenamidon, Triclopyricarb, Pyribencarb, Cyazofamid, Amisulbrom, Binapacryl, Meptyldinocarb, Dinocap, Fluazinam, Ferimzon, Fentinacetat, Fentinchlorid, Fentinhydroxid, Triphenylzinhydroxid, Triphenyltinacetat, Oxinkupfer, Silthiofam, Ametoctradin, Mepanipyrim, Nitrapyrin, Pyrimethanil, Cyprodinil, Blasticidin-S, Kasugamycin, Kasugamycin-Hydrochloridhydrat, Streptomycin, Oxytetracyclin, Chinoxyfen, Proquinazid, Fludioxonil, Fenpiclonil, Fluorimid, Procymidon, Iprodion, Vinclozolin, Edifenphos, Iprobenfos, Pyrazophos, Isoprothiolan, Propamocarb, Propamocarb Hydrochlorid, Teebaumölextrakt, Triforin, Pyrifenox, Pyrisoxazol, Fenarimol, Nuarimol, Azaconazol, Bromnazol, Diniconazol, Diniconazol-M, Epoxiconazol, Fluquinconazol, Oxpoconazol, Pefurazoat, Difenoconazol, Fenbuconazol, Imibenconazol, Iconazol, Metconazol, Tetraconazol, Triadimefon, Triadimenol, Triticonazol, Uniconazol, Imazalil, Bitertanol, Triflumizol, Etaconazol, Propiconazol, Penconazol, Flusilazol, Flutriafol, Mycobutanil, Paclobutrazol, Prothioconazol, Cyproconazol, Tebuconazol, Hexaconazol,

Prochloraz, Simeconazol, Aldimorph, Dodemorph, Dodemorphacetat, Tridemorph, Fenpropimorph, Dimethomorph, Flumorph, Pyrimorph, Piperalin, Fenpropidin, Spiroxamin, Fenhexamid, Fenpyrazamin, Ferbam, Metam, Methasulfocarb, Metiram, Thiram, Manzeb, Maneb, Zineb, Ziram, Polycarbamate, Propineb, Thiuram, Pyributicarb, Validamycin, Mildiomycin, Polyoxin, Benthiavalicarb, Benthiavalicarb-Isopropyl, Valifenalat, Iprovalicarb, Mandipropamid, Phthalid, Pyroquilon, Tricyclazol, Carpropamid, Diclocymet, Fenoxanil, Acibenzolar-S-Methyl, Probenazol, Thiadinil, Isotianil, Cymoxanil, Fosetyl, Tecloftalam, Triazoxid, Flusulfamid, Diclomezin, Cyflufenamid, Metrafenon, Pyriofenon, Flutianil, Tebufloquin, Fosetyl Aluminium, Tolclofos-Methyl, Echlomezol, Tolprocarb, Bordeaux-Mischung, Kupferacetat, basisches Kupfersulfat, Kupferoxychlorid, Kupferhydroxid, Oxyquinolinkupfer, Kupfer, Schwefel, Captan, Captafol, Falpet, Anilazin, Chlorthalonil, Dichlorphen, Pentachlorphenol und Salze davon, Hexachlorbenzol, Chintozen, Iminoctadin-Acetat, Iminoctadin-Albesilat, Guanidin, Dodin, Dodin-freie Base, Guazat, Guazat-Acetat, Albesilat, Dithianon, Chinomethionat, Fluorimid, Tolylfluanid, Dichlofluanid, Dinobuton, Dazomet, Dipymetitron, Pyraziflumid, Picarbutrazox, Tecnazen, Nitrothal-Isopropyl, Dicyclomet, Acibenzolar, Prohexadion-Calcium, Bronopol, Diphenylamin, Flumetover, Bentoxazin, Biphenyl, Chloroneb, CNA, Iodocarb, Prothiocarb, N-(1-(2,4-Dichlorphenyl)-1-Methoxypropan-2-yl)-2-Iobenzamid, Bromid, 3-(5-Fluor-3,3,4,4-tetramethyl-3,4-dihydroisoquinolin-1-yl)quinolin, 3-(4,4-Difluor-3,3-dimethyl-3,4-dihydroisoquinolin-1-yl)quinolin, 3-(4,4,5-Trifluor-3,3-dimethyl-3,4-dihydroisoquinolin-1-yl)quinolin, Bacillusarten und insektizide Proteine und bakterizide Proteine, die daraus hergestellt wurden , und insektizide Proteine, bakterizide Proteine, entomopathogene Bakterien, entomopathogene Viren und entomopathogene Pilze, die aus Bt-Pflanzen hergestellt wurden.

**9.** Zusammensetzung zum Schutz von Tieren vor schädlichen parasitären wirbellosen Organismen, umfassend die Verbindung oder das Salz davon nach Anspruch 1 in einer parasitizid wirksamen Menge und mindestens einen Träger.

**10.** Verfahren zur Bekämpfung schädlicher wirbelloser Organismen, umfassend das Inkontaktbringen der schädlichen wirbellosen Organismen oder der Umgebung davon mit der Verbindung oder dem Salz davon nach Anspruch 1 in einer biologisch wirksamen Menge, wobei die Umgebung davon den menschlichen oder tierischen Körper nicht umschließt.

**11.** Verfahren zum Erhöhen der Vitalität von Nutzpflanzen, umfassend das Inkontaktbringen der Nutzpflanzen, der Samen, aus denen Nutzpflanzen wachsen werden, oder der Basis von Nutzpflanzen mit der Verbindung oder dem Salz davon nach Anspruch 1 in einer biologisch wirksamen Menge.

**12.** Behandelter Samen, umfassend die Verbindung oder das Salz davon nach Anspruch 1 in einer Menge von etwa 0,0001 bis etwa 1 Masse-%, bezogen auf den Samen nach der Behandlung.

## Revendications

**1.** Composé représenté par une formule (1) ou un sel de celui-ci :

[Formule 1]

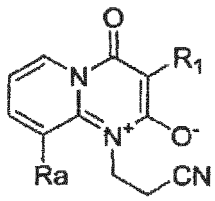

(1)

dans lequel

Ra représente un atome d’hydrogène, un atome d’halogène ou un groupe alkyle en C1, dans lequel le groupe alkyle en C1 est facultativement substitué par un ou plusieurs atomes du même halogène, ou d’un ou plusieurs halogènes différents ;

$R_1$ représente un groupe phényle ou un groupe pyridyle, qui est facultativement substitué par jusqu’à trois

substituants $R_2$; dans lequel
chaque $R_2$ représente un atome d'halogène ;
un groupe cyano ;
un groupe alkyle en C1-C4,
dans lequel le groupe alkyle en C1-C4 est facultativement substitué par
un ou plusieurs atomes du même halogène, ou d'un ou plusieurs halogènes différents, et/ou
un groupe alkyloxy en C1-C3 qui est facultativement substitué par un ou plusieurs atomes du même halogène, ou d'un ou plusieurs halogènes différents, et/ou
un groupe alkylthio en C1-C3 qui est facultativement substitué par un ou plusieurs atomes du même halogène, ou d'un ou plusieurs halogènes différents, et/ou
un groupe alkylsulfoxy en C1-C3 qui est facultativement substitué par un ou plusieurs atomes du même halogène, ou d'un ou plusieurs halogènes différents, et/ou
un groupe alkylsulfonyle en C1-C3 qui est facultativement substitué par un ou plusieurs atomes du même halogène, ou d'un ou plusieurs halogènes différents;
un groupe alcényle en C2-C3,
dans lequel le groupe alcényle en C2-C3 est facultativement substitué par un ou plusieurs atomes du même halogène, ou d'un ou plusieurs halogènes différents ;
un groupe alcynyle en C2-C3,
dans lequel le groupe alcynyle en C2-C3 est facultativement substitué par un ou plusieurs atomes du même halogène, ou d'un ou plusieurs halogènes différents;
un groupe alkyloxy en C1-C5,
dans lequel le groupe alkyloxy en C1-C5 est facultativement substitué par un ou plusieurs atomes du même halogène, ou d'un ou plusieurs halogènes différents, et/ou
un groupe alkyloxy en C1-C3 qui est facultativement substitué par un ou plusieurs atomes du même halogène, ou d'un ou plusieurs halogènes différents, et/ou
un groupe alkylthio en C1-C3 qui est facultativement substitué par un ou plusieurs atomes du même halogène, ou d'un ou plusieurs halogènes différents, et/ou
un groupe alkylsulfoxy en C1-C3 qui est facultativement substitué par un ou plusieurs atomes du même halogène, ou d'un ou plusieurs halogènes différents, et/ou
un groupe alkylsulfonyle en C1-C3 qui est facultativement substitué par un ou plusieurs atomes du même halogène, ou d'un ou plusieurs halogènes différents ;
un groupe amino ayant deux substituants sélectionnés à partir d'un atome d'hydrogène, d'un groupe alkyle en C1-C4 et d'un groupe acyle en C2,
dans lequel le groupe alkyle en C1-C4 est facultativement substitué par
un ou plusieurs atomes du même halogène, ou d'un ou plusieurs halogènes différents, et/ou
un groupe alkyloxy en C1-C3 qui est facultativement substitué par un ou plusieurs atomes du même halogène, ou d'un ou plusieurs halogènes différents, et/ou
un groupe alkylthio en C1-C3 qui est facultativement substitué par un ou plusieurs atomes du même halogène, ou d'un ou plusieurs halogènes différents, et/ou
un groupe alkylsulfoxy en C1-C3 qui est facultativement substitué par un ou plusieurs atomes du même halogène, ou d'un ou plusieurs halogènes différents, et/ou
un groupe alkylsulfonyle en C1-C3 qui est facultativement substitué par un ou plusieurs atomes du même halogène, ou d'un ou plusieurs halogènes différents; et
dans lequel le groupe acyle en C2 est facultativement substitué par un ou plusieurs atomes du même halogène, ou d'un ou plusieurs halogènes différents ; ou
un groupe alkylthio en C1-C4, un groupe alkylsulfoxy en C1-C4 ou un groupe alkylsulfonyle en C1-C4,
dans lequel le groupe alkylthio en C1-C4, le groupe alkylsulfoxy en C1-C4 ou le groupe alkylsulfonyle en C1-C4 peut être substitué par un substituant sélectionné à partir d'un groupe constitué par :

> un ou plusieurs atomes du même halogène, ou d'un ou plusieurs halogènes différents, et/ou
> un groupe alkyloxy en C1-C3 qui est facultativement substitué par un ou plusieurs atomes du même halogène, ou d'un ou plusieurs halogènes différents, et/ou

un groupe alkylthio en C1-C3 qui est facultativement substitué par un ou plusieurs atomes du même halogène, ou d'un ou plusieurs halogènes différents, et/ou
un groupe alkylsulfoxy en C1-C3 qui est facultativement substitué par un ou plusieurs atomes du même halogène, ou d'un ou plusieurs halogènes différents, et/ou
un groupe alkylsulfonyle en C1-C3 qui est facultativement substitué par un ou plusieurs atomes du même

halogène, ou d'un ou plusieurs halogènes différents.

**2.** Composé ou sel de celui-ci selon la revendication 1, dans lequel Ra est un atome d'hydrogène ou un groupe méthyle ; et $R_1$ est un groupe phényle qui est facultativement substitué par jusqu'à deux substituants $R_2$ ; dans lequel chaque $R_2$ est indépendamment sélectionné à partir du groupe constitué par :

un atome d'halogène,
un groupe alkyle en C1-C4 qui est facultativement substitué par un ou plusieurs atomes du même halogène, ou d'un ou plusieurs halogènes différents,
un groupe alcényle en C2-C3 qui est facultativement substitué par un ou plusieurs atomes du même halogène, ou d'un ou plusieurs halogènes différents,
un groupe alcynyle en C2-C3 qui est facultativement substitué par un ou plusieurs atomes du même halogène, ou d'un ou plusieurs halogènes différents,
un groupe alkyloxy en C1-C5 qui est facultativement substitué par un ou plusieurs atomes du même halogène, ou d'un ou plusieurs halogènes différents,
un groupe N-alkylacétamide, dans lequel le groupe alkyle est en C1-C4 et est facultativement substitué par un ou plusieurs atomes du même halogène, ou d'un ou plusieurs halogènes différents,
un groupe N-alkyltrifluoroacétamide, dans lequel le groupe alkyle est en C1-C4 et est facultativement substitué par un ou plusieurs atomes du même halogène, ou d'un ou plusieurs halogènes différents,
un groupe acétamide,
un groupe trifluoroacétamide,
un groupe alkylthio en C1-C4 qui est facultativement substitué par un ou plusieurs atomes du même halogène, ou d'un ou plusieurs halogènes différents,
un groupe alkylsulfoxy en C1-C4 qui est facultativement substitué par un ou plusieurs atomes du même halogène, ou d'un ou plusieurs halogènes différents, et
un groupe alkylsulfonyle en C1-C4 qui est facultativement substitué par un ou plusieurs atomes du même halogène, ou d'un ou plusieurs halogènes différents.

**3.** Composé ou sel de celui-ci selon la revendication 1, dans lequel Ra est un atome d'hydrogène ou un groupe méthyle ; et $R_1$ est un groupe phényle qui est facultativement substitué par jusqu'à deux substituants $R_2$ dans lequel chaque $R_2$ est : un atome de fluor, un atome de chlore, un atome de brome, un groupe méthyle, un groupe éthyle, un groupe n-propyle, un groupe i-propyle, un groupe c-propyle, un groupe n-butyle, un i-butyle, un groupe s-butyle, un groupe t-butyle, un groupe fluorométhyle, un groupe difluorométhyle, un groupe trifluorométhyle, un groupe méthyloxy, un groupe éthyloxy, un groupe n-propyloxy, un groupe i-propyloxy, un groupe c-propyloxy, un groupe n-butyloxy, un groupe i-butyloxy, un groupe s-butyloxy, un groupe t-butyloxy, un groupe n-pentyloxy, un groupe trifluorométhyle, un groupe 2,2,2-trifluoroéthyloxy, un groupe méthylthio, un groupe méthylsulfoxy, un groupe méthylsulfonyle, un groupe vinyle ou un groupe éthynyle.

**4.** Composé ou sel de celui-ci selon la revendication 1, dans lequel le composé représenté par la formule (1) est sélectionné à partir du groupe constitué par :

sel interne de 1-(2-cyanoéthyl)-2-hydroxy-4-oxo-3-phényl-4H-pyrido[1,2-a]pyrimidinium ;
sel interne de 1-(2-cyanoéthyl)-2-hydroxy-4-oxo-3-(3-toluyl)-4H-pyrido[1,2-a]pyrimidinium ;
sel interne de 1-(2-cyanoéthyl)-3-(3-éthylphényl)-2-hydroxy-4-oxo-4H-pyrido[1,2-a]pyrimidinium ;
sel interne de 1-(2-cyanoéthyl)-2-hydroxy-3-(3-isopropylphényl)-4-oxo-4H-pyrido[1,2-a]pyrimidinium;
sel interne de 1-(2-cyanoéthyl)-2-hydroxy-3-(3-tert-butylphényl)-4-oxo-4H-pyrido[1,2-a]pyrimidinium;
sel interne de 1-(2-cyanoéthyl)-2-hydroxy-3-(3-méthoxyphényl)-4-oxo-4H-pyrido[1,2-a]pyrimidinium ;
sel interne de 1-(2-cyanoéthyl)-3-(3-éthoxyphényl)-2-hydroxy-4-oxo-4H-pyrido[1,2-a]pyrimidinium;
sel interne de 1-(2-cyanoéthyl)-2-hydroxy(3-n-propoxyphényl)-4-oxo-3-4H-pyrido[1,2-a]pyrimidinium;
sel interne de 1-(2-cyanoéthyl)-2-hydroxy-3-(3-isopropoxyphényl)-4-oxo-4H-pyrido[1,2-a]pyrimidinium;
sel interne de 1-(2-cyanoéthyl)-2-hydroxy-3-(3-n-butoxyphényl)-4-oxo-4H-pyrido[1,2-a]pyrimidinium;
sel interne de 1-(2-cyanoéthyl)-2-hydroxy-3-(3-n-pentyloxyphényl)-4-oxo-4H-pyrido[1,2-a]pyrimidinium;
sel interne de 1-(2-cyanoéthyl)-3-(2-fluorophényl)-2-hydroxy-4-oxo-4H-pyrido[1,2-a]pyrimidinium ;
sel interne de 1-(2-cyanoéthyl)-3-(3-fluorophényl)-2-hydroxy-4-oxo-4H-pyrido[1,2-a]pyrimidinium ;
sel interne de 3-(3-chlorophényl)-1-2-cyanoéthyl)-2-hydroxy-4-oxo-4H-pyrido[1,2-a]pyrimidinium;

sel interne de 3-(3-bromophényl)-1-(2-cyanoéthyl)-2-hydroxy-4-oxo-4H-pyrido[1,2-a]pyrimidinium;
sel interne de 1-(2-cyanoéthyl)-3-(3-difluorométhylphényl)-2-hydroxy-4-oxo-4H-pyrido[1,2-a]pyrimidinium;
sel interne de 1-(2-cyanoéthyl)-2-hydroxy-4-oxo-3-(3-trifluorométhylphényl)-4H-pyrido[1,2-a]pyrimidinium;
sel interne de 1-(2-cyanoéthyl)-3-(3-difluorométhoxyphényl)-2-hydroxy-4-oxo-4H-pyrido[1,2-a]pyrimidinium;
sel interne de 1-(2-cyanoéthyl)-2-hydroxy-4-oxo-3-(3-trifluorométhoxyphényl)-4H-pyrido[1,2-a]pyrimidinium;
sel interne de 1-(2-cyanoéthyl)-2-hydroxy-4-oxo-3-(4-trifluorométhoxyphényl)-4H-pyrido[1,2-a]pyrimidinium;
sel interne de 1-(2-cyanoéthyl)-2-hydroxy-4-oxo-3-(3-(2,2,2-trifluoroéthoxy)phényl)-4H-pyrido[1,2-a]pyrimidinium;
sel interne de 1-(2-cyanoéthyl)-2-hydroxy-3-(3-méthylthiophényl)-4-oxo-4H-pyrido [1,2-a]pyrimidinium;
sel interne de 1-(2-cyanoéthyl)-2-hydroxy-3-(3-éthylthiophényl)-4-oxo-4H-pyrido[1,2-a]pyrimidinium;
sel interne de 3-(3-acétamidophényl)-1-(2-cyanoéthyl)-2-hydroxy-4-oxo-4H-pyrido[1,2-a]pyrimidinium;
sel interne de 1-(2-cyanoéthyl)-2-hydroxy-3-(3-(N-méthylacétamide)phényl)-4-oxo-4H-pyrido[1,2-a]pyrimidinium;
sel interne de 1-(2-cyanoéthyl)-2-hydroxy-4-oxo-3(3-vinylphényl)-4H-pyrido[1,2-a]pyrimidinium;
sel interne de 1-(2-cyanoéthyl)-2-hydroxyl-3-(3,5-diméthylphényl)-4-oxo-4H-pyrido[1,2-a]pyrimidinium;
sel interne de 1-(2-cyanoéthyl)-2-hydroxy-3-(2,5-diméthoxyphényl)-4-oxo-4H-pyrido[1,2-a]pyrimidinium;
sel interne de 3-(3,5-dichlorophényl)-1-(2-cyanoéthyl)-2-hydroxy-4-oxo-4H-pyrido[1,2-a]pyrimidinium;
sel interne de 1-(2-cyanoéthyl)-3-(4-fluoro-3-méthylphényl)-2-hydroxy-4-oxo-4H-pyrido[1,2-a]pyrimidinium;
sel interne de 3-(3-chloro-6-méthoxyphényl)-1-2-cyanoéthyl)-2-hydroxy-4-oxo-4H-pyrido[1,2-a]pyrimidinium;
sel interne de 3-(2-chloro-5-trifluorométhylphényl)-1-(2-cyanoéthyl)-2-hydroxy-4-oxo-4H-pyrido[1,2-a]pyrimidinium;
sel interne de 1-(2-cyanoéthyl)-2-hydroxy-3-(4-méthyl-3-trifluorométhylphényl)-4-oxo-4H-pyrido[1,2-a]pyrimidinium;
sel interne de 1-(2-cyanoéthyl)-2-hydroxy-8-méthyl-4-oxo-3-(3-toluyl)-4H-pyrido[1,2-a]pyrimidinium;
sel interne de 1-(2-cyanoéthyl)-3-(3-éthoxyphényl)-2-hydroxy-8-méthyl-4-oxo-4H-pyrido[1,2-a]pyrimidinium;
sel interne de 1-(2-cyanoéthyl)-2-hydroxy-8-méthyl-4-oxo-3-(3-trifluorométhylphényl)-4H-pyrido[1,2-a]pyrimidinium; et
sel interne de 1-(2-cyanoéthyle)-3-(3,5-dichlorophényl)-2-hydroxy-8-méthyl-4-oxo-4H-pyrido[1,2-a]pyrimidinium.

**5.** Composé ou sel de celui-ci selon la revendication 1, dans lequel le composé représenté par la formule (1) est sélectionné à partir du groupe constitué par :

sel interne de 1-(2-cyanoéthyl)-2-hydroxy-4-oxo-3-(3-toluyl)-4H-pyrido[1,2-a]pyrimidinium ;
sel interne de 1-(2-cyanoéthyl)-2-hydroxy-3-(3-méthoxyphényl)-4-oxo-4H-pyrido[1,2-a]pyrimidinium;
sel interne de 1-(2-cyanoéthyl)-3-(3-éthoxyphényl)-2-hydroxy-4-oxo-4H-pyrido[1,2-a]pyrimidinium; et
sel interne de 1-(2-cyanoéthyl)-2-hydroxy-4-oxo-3-(3-trifluorométhylphényl)-4H-pyrido[1,2-a]pyrimidinium.

**6.** Composition insecticide comprenant le composé ou le sel de celui-ci selon l'une quelconque des revendications 1 à 5 et au moins un composant sélectionné à partir du groupe constitué par un tensioactif, un diluant solide et un diluant liquide.

**7.** Composition insecticide selon la revendication 6, comprenant en outre au moins un composé biologiquement actif ou un agent chimique.

**8.** Composition selon la revendication 7, dans laquelle le au moins un composé ou agent chimique biologiquement actif est sélectionné à partir du groupe constitué par :
alanycarbe, aldicarbe, bendiocarbe, benfuracarbe, butocarboxime, butoxycarboxime, carbaryl, carbofuran, carbosulfan, éthiofencarbe, fénobucarbe, formétanate, furathiocarb, isoprocarbe, méthiocarbe, méthomyle, oxamyle, pirimicarbe, propoxur, thiodicarbe, thiofanox, triazamate, triméthacarbe, XMC, xylylcarbe, métolcarbe, fénothiocarbe, fénoxycarbe, acéphate, azaméthiphos, azinphos-éthyle, azinphos-méthyle, éthylthiométon, chloréthoxyphos, cadusafos, chloréthoxyphos, chlorfenvinphos, chlorméphos, chlorpyrifos, chlorpyrifos-méthyle, coumaphos, cyanophos, déméton-S-méthyle, diazinon, dichlorvos, dicrotophos, diméthoate, diméthylvinphos, EPN, éthion, éthoprophos, famphur, fenamiphos, fénitrothion, fenthion, fosthiazate, hepténophos, imicyafos, isofenphos, isopropyl=O-(méthoxy-aminothio-phosphoryle), salicylates, isoxathion, malathion, mécarbame, méthamidophos, méthidathion, mevinphos, monocrotophos, naled, ométhoate, oxydéméton-éthyle, parathion, parathion-méthyle, PAP, phorate, phosalon, phosmet, phosphamidon, phoxime, pirimiphos-méthyl, profénophos-propétamphos, prothiofos, pyraclofos, pyridaphenthion, quinalphos, sulfotep, tébupirimfos, téméphos, terbufos, tétrachlorvinphos, thiométon,

triazophos, trichlorfon, vamidothion, chlorpyrifos-éthyle, disulfoton, sulprofos, flupyrafos, phentoate, fonofos, tribufos, endosulfan, alpha-endosulfan, gamma-HCH, dicofol, chlordane, dieldrine, méthoxychlore, acétoprole, fipronil, éthiprole, pyrafluprole, pyriprole, flufiprole, broflanilide, afoxolaner, fluralaner, sarolarner, acrinathrine, alléthrine, d-cis-trans alléthrine, d-trans alléthrine, bifenthrine, kappa-bifenthrine, bioalléthrine-S-cyclopentenyl, bioresméthrine, cycloprothrine, cyfluthrine, bêta-cyfluthrine, cyhalothrine, lambda-cyhalothrine, gamma-cyhalothrine, cyperméthrine, alpha-cyperméthrine, bêta-cyperméthrine, thêta-cyperméthrine, zêta-cyperméthrine, cyphénothrine, deltaméthrine, empenthrine, esfenvalérate, éthofenprox, fenpropathrine, fenvalérate, flucythrinate, fluméthrine, tau-fluvalinate, halfenprox, imiprothrine, kadéthrine, perméthrine, phénothrine, pralléthrine, pyréthrine, resméthrine, silafluofen, téfluthrine, kappa-téfluthrine, phtalthrine, tétraméthrine, tralométhrine, transfluthrine, méthoxadiazone, métofluthrine, profluthrine, pyrethrum, teralléthrine, momfluorothrine, heptafluthrine, meperfluthrine, tétraméthylfluthrine, diméfluthrine, protibenbute, acétamipride, clothianidine, dinotéfurane, imidaclopride, nitenpyrame, thiaclopride, thiaméthoxame, sulfoxafor, flupyradifurone, triflumézopyrime, dicloromézotiaz, spinosade, spinétorame, abamectine, iverméctine, benzoate d'émamectine, milbemectine, lepimetcine, hydroprène, kinoprène, diofénolane, méthoprène, pyriproxyfen, pymétrozine, flonicamide, étoxazole, diafenthiuron, azocyclotine, cyhexatine, oxyde de fenbutatine, propargite, tétradifon, chlorfénapyr, tralopyril, DNOC, bensultap, cartap, thiocyclam, thiosultap, thiosultap-sodium, bistrifluron, chlorfluazuron, diflubenzuron, flucycloxuron, flufénoxuron, hexaflumuron, lufénuron, novaluron, noviflumuron, teflubenzuron, triflumuron, bistrifluron, buprofézine, cyromazine, chromafénozide, halofénozide, méthoxyfénozide, tébufénozide, amitraz, hydraméthylnone, acéquinocyl, fluacrypyrime, pyriminostrobine, flufenoxystrobine, fénazaquine, fenpyroximate, pyrimidifène, pyridabène, tébufenpyrad, tolfenpyrad, pyflubumide, spirodiclofène, spirotetramat, spiromésifène, cyflumétofène, cyenopyrafen, flubendiamide, chlorantraniliprole, cyantraniliprole, cyclaniliprole, tétraniliprole, quinométhionate, hexythiazox, bifénazate, flufénérim, pyrifluquinazone, flométoquine, fluopyram, 8-chloro-N-((2-chloro-5-méthoxyphényl)sulfonyl)-6-(trifluorométhyl)imidazo[1,2-a]pyridine-2-carboxamide, amidoflumet, nicotine, chloropicrine, fluorure de sulfuryle, chlorothié, clofentézine, diflovidazine, roténone, indoxacarb, butoxyde de pipéronyle, chlordiméform, pyridalyl, azadirachtine, benzoxymate, afidopiropen, fluhexafon, tioxazafène, fluensulfone, benclothiaz, carzole, un savon insecticide, diméhypo, nithiazine, un borate, métaldéhyde, ryanodine, sulfuramide, (E)-N-(1-((6-chloropyridin-3-yl)méthyl)pyridine-2(1H)-ylidène)-2,2,2-trifluoroacétamide, 5-(trifluorométhyl)-2-(4-(4-(trifluorométhyl)phénoxy)pipéridine 1-yl)pyridine, métalaxyl, métalaxyl-M, oxadixyl, ofurace, bénalaxyl, bénalaxyl-M, kiralaxyl, ofurace, furalaxyl, cyprofuram, bupirimate, diméthirimol, éthirimol, hyxémazol, hydroxyisoxazole, oxathiapiproline, octhilinone, acide oxolinique, bénomyl, thiophanate-méthyle, carbendazim, fubéridazole, thiabendazole, débacarb, diéthofencarb, zoxamide, éthaboxam, pencycuron, fluopicolide, diflumétorim, bupirimate, bénodanil, flutolanil, mépronil, isofétamide, fenfuram, oxycarboxin, carboxin, thifluzamide, fluxapyroxad, furamétpyr, penflufène, penthiopyrad, benzovindiflupyr, bixafen, isopyrazam, sedaxane, boscalid, azoxystrobine, coumétoxystrobine, krésoxym-méthyle, trifloxystrobine, picoxystrobine, pyraclostrobine, dimoxystrobine, métominostrobine, orysastrobine, fluoxastrobine, pyraoxystrobine, pyramétostrobine, flufenoxystrobine, fénaminstrobine, énoxastrobine, coumoxystrobine, mandestrobine, triclopyricarb, famoxadone, fenamidone, triclopyricarb, pyribencarb, cyazofamide, amisulbrom, binapacryl, meptyldinocarb, dinocap, fluaziname , ferimzone, acétate de fentine, chlorure de fentine, hydroxyde de fentine, hydroxyde de triphényltine, acétate de triphényltine, oxine-cuivre, silthiofam, amétocradine, mépanipyrim, nitrapyrin, pyriméthanil, cyprodinil, blasticidine S, kasugamycin, kasugamycin-hydrochlorure-hydrate, streptomycine, oxytétracycline, quinoxyfen, proquinazid, fludioxonil, fenpiclonil, fluoroimide, procymidone, iprodione, vinclozolin, édifenphos, iprobenfos, pyrazophos, isoprothiolane, propamocarb, hydrochlorure de propamocarb, extrait de théier, triforine, pyriténox, pyrisoxazole, fénarimol, nuarimol, azaconazole, bromuconazole, diniconazole, diniconazole-M, époxiconazole, fluquinconazole, oxpoconazole, pefurazoate, difénoconazole, fenbuconazole, imibenconazole, ipconazole, metconazole, tétraconazole, triadimefon, triadiménol, triticonazole, uniconazole, imazalil, bitertanol, triflumizole, étaconazole, propiconazole, penconazole, flusilazole, flutriafol, myclobutanil, paclobutrazol, prothioconazole, cyproconazole, tébuconazole, hexaconazole, prochloraz, simeconazole, aldimorphe, dodémorph, acétate de dodémorpe, tridémorphe, fenpropimorphe, diméthomorphe, flumorphe, pyrimorphe, pipéraline, fenpropidine, spiroxamine, fenhexamide, fenpyrazamine, ferbam, métame, méthasulfocarb, métirame, thirame, manzeb, maneb, zineb, zirame, polycarbamate, propinèbe, thiuram, pyributicarb, validamycine, mildiomycinr, polyoxine, benthiavalicarb, benthiavalicarb-isopropyle, valifénalate, iprovalicarb, mandipropamide, fthalide, pyroquilon, tricyclazole, carpropamide, diclocymet, fénoxanil, acibenzolar-S-méthyle, probénazole, tiadinil, isotianil, cymoxanil, fosétyl, técloftalarn, triazoxide, flusulfamide, diclomézine, cyflufenamide, métrafénone, pyriofénone, flutianil, tébufloquine, fosétyl-aluminum, tolclofos-méthyle, échlomézol, tolprocarb, mélange de Bordeaux, acétate de cuivre, sulfate de cuivre basique, oxychlorure de cuivre, hydroxide cuprique, oxyquinoléine de cuivre, cuivre, soufre, captane, captafol, folpet, anilazine, chlorothalonil, dichlorophène, pentachlorophénol et des sels de ceux-ci, hexachlorobenzène, quintozène, acétate d'iminoctadine, albésilate d'iminoctadine, guanidine, dodine, base sans dodine, guazatine, acétate de guazatine, albésilate, dithianon, quinométhionate, fluoroimide, tolylfluanide, dichlofluanide, dinobuton, dazomet, dipymétitrone, pyraziflumide, picarbutrazox, tecnazène, nitrothal-isopropyle, dicyclomet, acibenzolar, prohexadione-calcium, bronopol, diphénylamine, flumetover, bentoxazine, biphényle, chloroneb, CNA, iodocarb, prothiocarb, N-(1-(2,4-dichlorophényl)-1-méthoxypropan-2-yl)-2-2-iodobenzamide, bromure, 3-(5-fluoro-3,3,4,4-tétraméthyl-3,4-dihydroisoquinoléine-1-yl)quinoléine, 3-(4,4-difluoro-3,3-diméthyl-3,4-dihydroisoquinoléine-1-yl)quinoléine, 3-(4,4,5-trifluoro-3,3-diméthyl-3,4-dihydroisoquinoléine-1-yl)quinoléine, des espèces de Bacillus et protéines insecticides et protéines bactéricides produites à partir de ceux-ci, et des protéines insecticides, des protéines bactéricides, des bactéries entomopathogènes, des virus entomopathogènes et des champignons entomopathogènes produits à partir de cultures de Bt.

**9.** Composition pour protéger des animaux contre des organismes invertébrés parasites nuisibles, comprenant le composé ou le sel de celui-ci selon la revendication 1 en une quantité efficace sur le plan parasiticide et au moins un support.

**10.** Procédé de lutte contre les organismes invertébrés nuisibles, comprenant la mise en contact des organismes invertébrés nuisibles ou de l'environnement de ceux-ci avec le composé ou le sel de celui-ci selon la revendication 1 en une quantité biologiquement efficace, dans lequel l'environnement de ceux-ci n'englobe pas le corps humain ou animal.

**11.** Procédé pour améliorer la vitalité des cultures, comprenant la mise en contact des cultures, des semences à partir desquelles les cultures vont pousser, ou de la base des cultures avec le composé ou le sel de celui-ci selon la revendication 1 en une quantité biologiquement efficace.

**12.** Semence traitée comprenant le composé ou le sel de celui-ci selon la revendication 1 en une quantité d'environ 0,0001 à environ 1 % en masse par rapport à la semence après traitement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011017342 A
- WO 2012092115 A
- WO 2009099929 A


### Non-patent literature cited in the description


- Pesticide Manual. The British Crop Protection Council
- SHIBUYA INDEX. SHIBUYA INDEX RESEARCH GROUP, 2011
- SHIBUYA INDEX. SHIBUYA INDEX RESEARCH GROUP, 2012
- Mode of Action Classification. IRAC
- Mode of Action of Fungicide. FRAC, 2015